# EUROPEAN PATENT APPLICATION

(11) **EP 4 759 363 A1**
(43) Date of publication of application: **17.06.2026**
(21) Application number: 24383373.8
(22) Date of filing: 16.12.2024
(51) Int. Cl.: A61P 25/28, C07D 417/04, A61K 31/4415

(54) **AZOBENZENES FOR PHOTOREVERSIBLE MODULATION OF NEUROLOGICAL DISORDERS**

(71) Applicant: Fundació Institut de Bioenginyeria de Catalunya (IBEC), 08028 Barcelona (ES); Institució Catalana de Recerca i Estudis Avançats, 08010 Barcelona (ES); Julius-Maximilians-Universität Würzburg, in Vertretung des Freistaates Bayern, 97070 Würzburg (DE)
(72) Inventor: GOROSTIZA LANGA, Pau, 08028 Barcelona (ES); MALIEIEVA, Galyna, 08028 Barcelona (ES); SORTINO, Rosalba, 08028 Barcelona (ES); MATERA, Carlo, 08028 Barcelona (ES); RIEFOLO, Fabio, 08028 Barcelona (ES); GERWE, Hubert, 97070 Würzburg (DE); DECKER, Michael, 97070 Würzburg (DE)
(74) Representative: ABG Intellectual Property Law, S.L.

(57) **Abstract**

The present invention relates to a compound of formula (I) or a pharmaceutically acceptable salt or stereoisomer thereof, to pharmaceutical compositions comprising said compound, and to their use in the treatment and/or prevention of neurological disorders.

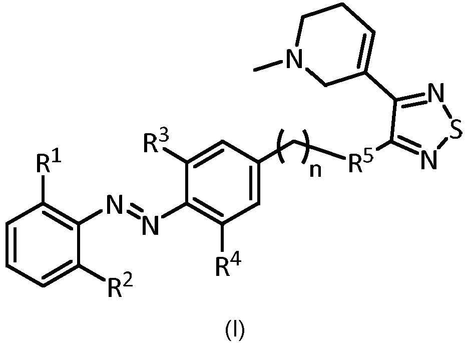

## Description

### FIELD OF THE INVENTION

The present invention relates to compounds useful for the treatment of neurological disorders, to the use of said compounds and pharmaceutical composition comprising them as a medicament, in particular for the treatment and/or prevention of neurological disorders.

### BACKGROUND OF THE INVENTION

Muscarinic acetylcholine receptors (mAChRs) are members of the class A G protein-coupled receptor (GPCR) family and have a crucial role in both the central and peripheral nervous systems (CNS, PNS) [Eglen, R. M., Overview of Muscarinic Receptor Subtypes. In Muscarinic Receptors, 2012; pp 3-28]. The group comprises five receptor subtypes denoted M₁-M₅, of which the odd numbered receptors are excitatory by coupling to G_{q/11} while the even numbered receptors are inhibitory by predominantly coupling to G_{i/o}. Altogether they are involved in a widespread range of functions, regulated by their endogenous ligand acetylcholine. In the CNS vital functions like learning, memory and cognitive function are controlled by mAChRs, while in the peripheral nervous system mAChRs mediate negative chronotropic and ionotropic effects, smooth muscle contraction and glandular secretions, to name only the most prominent functions [Eglen, R. M., Muscarinic Receptor Subtype Pharmacology and Physiology. 2005; pp 105-136].

Unsurprisingly, given the broad range of tasks mediated by mAChRs, impairment of their function is associated with various chronic neurological and peripheral disorders. In the CNS disturbed function is associated with Schizophrenia (SZ), Alzheimer's and Parkinson's disease (AD, PD) [Winek, K. et al., J. Neurochem., 2021, 158 (6), 1425-1438] while in the periphery drugs targeting mAChRs are already used in urology, pulmonology, ophthalmology and gastroenterology [Kruse, A. C. et al., Nature Reviews Drug Discovery, 2014, 13 (7), 549-560]. More recently, muscarinic receptors were also linked to and postulated as potential therapeutic strategies in acute brain diseases such as traumatic brain injury [Shin, S. S. et al., J. Neurotrauma, 2015, 32 (19), 1429-1440] and stroke [Martin, A. et al., Ther. Adv. Neurol. Disord., 2018, 11, 1756286418774267].

Various therapeutic approaches targeting muscarinic receptors have been developed to treat the above-mentioned neurological disorders, however, with limited success [Felder, C. C. et al., J. Med. Chem., 2000, 43 (23), 4333-4353; Erskine, D. et al., Drug Discov. Today, 2019, 24 (12), 2307-2314; Subramaniam, S. et al., J. Alzheimers Dis., 2021,83 (2), 491-503; Brown, A. J. H. et al., Cell, 2021, 184 (24), 5886-5901.e22]. The abundant expression in almost all tissues combined with the highly conserved orthosteric binding site of the mAChR subtypes, has made selectively targeting the right receptor subtype in the desired tissue a historically complicated challenge for medicinal chemists. Some experimental therapeutics, as for example the functional M₁/M₄ selective orthosteric agonist xanomeline, have shown promising efficacy in clinical studies targeting AD and SZ, but were ultimately stopped due to intolerable adverse effects [Bodick, N. C. et al. Arch. Neurol., 1997, 54 (4), 465-473; Shekhar, A. et al., Am. J. Psychiatry, 2008, 165 (8), 1033-1039]. However, the initial efficacy was promising enough that interest continued, leading to KarTX, a mixture of xanomeline and the peripherally restricted muscarinic antagonist tiotropium bromide. Up to now, KarTX has advanced to a phase III clinical study for the treatment of SZ (clinicaltrials.gov). Even though the KarTX approach seems to successfully limit the agonists actions to the CNS, such conventional agonists are not limited to a specific region of the brain, thus potentially activating their target in multiple CNS regions simultaneously, unintentionally affecting other physiological functions.

To engage this lack of spatial selectivity of conventional pharmacological agents, methods that allow the activation of ligands with high spatiotemporal resolution are required.

Since 2014 various ligands were developed whose biological activity can be modulated using light as an external stimulus. These photopharmacological agents have been used to photo-modulate a variety of G protein-coupled receptors (GPCRs) with unprecedented spatiotemporal resolution *in vitro* and even *in vivo* [Morstein, J. et al., Angew Chem Int Ed Engl, 2022, 61 (18), e202117094; Gerwe, H. et al., Angew. Chem. Int. Ed. Engl., 2022, 61 (26), e202282661; Donthamsetti, P. et al., Nature Communications, 2021, 12 (1), 4775; Donthamsetti, P. et al., J. Am. Chem. Soc., 2021, 143 (24), 8951-8956; Bosma, R. et al., iScience, 2022, 25 (9), 104882; Hauwert, N. et al., Angew Chem Int Ed Engl, 2019, 58 (14), 4531-4535; Gomez-Santacana, X. et al. Angew Chem Int Ed Engl, 2018, 57 (36), 11608-11612; Lahmy, R. et al., Chemistry - A European Journal, 2022, 28 (63), e202201515; Rustler, K. et al., ChemMedChem, 2019, 14 (6), 636-644; Lachmann, D. et al., Org. Biomol. Chem., 2019, 17 (9), 2467-2478; Matera, C. et al., Int. J. Mol. Sci., 2022, 23 (17), 10114; Garrido-Charles, A. et al., J. Am. Chem. Soc., 2022, 144 (21), 9229-9239; Barbero-Castillo, A. et al., Adv Sci (Weinh), 2021, 8 (14), e2005027; Riefolo, F. et al., J. Med. Chem., 2021, 64 (13), 9259-9270; Bossi, S. et al., Front. Cell. Neurosci., 2018, 12, 449; Morstein, J. et al., J. Am. Chem. Soc., 2020, 142 (24), 10612-10616; Acosta-Ruiz, A. et al., Neuron, 2020, 105 (3), 446-463.e13; Gutzeit, V. A. et al., Cell Chemical Biology, 2021, 28 (11), 1648-1663.e16; Ricart-Ortega, M. et al., Mol Cell Endocrinol, 2019, 488, 36-51; Wijtmans, M. et al., Curr. Opin. Pharmacol., 2022, 63, 102192]. Some reported muscarinic photoswitchable ligands like PAI [Barbero-Castillo, A. et al., Adv Sci (Weinh), 2021, 8 (14), e2005027] enable reversible modulation of slow brain-waves *in vivo,* allowing the reactivation of brain regions displaying reduced activity as a result of stroke [Cassidy, J. M. et al. Stroke, 2020, 51, 1442-1450] or traumatic brain injury; [Hogan, S. E. et al., J. Clin. Sleep Med., 2020, 16, 1445-1454; Kaada, B. R. et al., Electroencephalogr. Clin. Neurophysiol., 1961, 13, 785-789] the regulation of brain activity associated with sleep disorders [Kaltiainen, H. et al. Brain Topogr., 2018, 31, 1037-1046]; the induction of faster recovery from anesthesia [Modarres, M. H. et al. Neurobiol. Sleep Circadian Rhythms, 2017, 2, 59-70] or the awakening from coma state [Torao-Angosto, M. et al. Front. Syst. Neurosci., 2021, 15, DOI: 10.3389/fnsys.2021.609645].

Among the so far developed photoswitchable GPCR ligands, a considerable amount has targeted the muscarinic receptors, including the first ever reported photoswitchable ligand in 1982 [Lester, H. A. et al., Annu. Rev. Biophys. Bioeng., 1982, 11 (1), 151-175; Nargeot, J. et al., J. Gen. Physiol., 1982, 79 (4), 657-678]. Even though a great deal of work has been done on this target, including photoswitchable dualsteric ligands [Agnetta, L. et al., Angew. Chem. Int. Ed., 2017, 56 (25), 7282-7287; Riefolo, F. et al., J. Am. Chem. Soc., 2019, 141 (18), 7628-7636], tuning of the operational wavelengths [Agnetta, L. et al., J. Med. Chem., 2019, 62 (6), 3009-3020] and *in vivo* photomodulation of cardiac activity in rats [Riefolo, F. et al., J. Am. Chem. Soc., 2019, 141 (18), 7628-7636], all these photoswitchable ligands share the same shortcomings. They are more active in their thermodynamically stable *trans*-form (*trans*-on) and operational with only poorly tissue penetrating wavelengths. The inherent disadvantage of *trans*-on compounds arise out of the thermodynamically driven relaxation from the *cis-* to the trans-isomer. This non-radiative process allows an inactive *cis*-isomer to spontaneously convert to its active *trans*-form, thereby uncontrollably activating itself, reducing its spatiotemporal resolution in the process.

Thus, there is a need for new photoswitchable mAChRs ligands that overcome the shortcomings of the compounds disclosed in the prior art.

### SUMMARY OF THE INVENTION

The inventors have surprisingly found new compounds that show reversible muscarinic modulation of brain activity, which do not produce any effect in the absence of illumination, whereas upon illumination at single-photon excitation (1 PE) and two-photon excitation (2PE) they show a muscarinic acetylcholine receptor agonistic activity. Thus, the effects are only localized at the region of illumination and on demand, decreasing the incidence of severe adverse effects and paving the way to a new strategy for personalized precision pharmacotherapy. Moreover, the pharmacologically inactive form of the compounds of the invention is the thermodynamically stable *trans* isomer, a particularly advantageous and uncommon behavior in photopharmacology. This feature allows the administration of inactive compounds *in vivo* and their spatiotemporal precise activation only under illumination with a specific wavelength range (*trans*-to-*cis* isomerization) in the body. In the absence of illumination, these compounds spontaneously revert to the inactive form (*cis*-to-*trans* isomerization) in the body. This action has been demonstrated *in vivo* in zebrafish larvae. Few photopharmacological compounds have been tested *in vivo* and demonstrated light-activated effects. As also shown in the examples, in zebrafish larvae, the compounds of the invention are not toxic, do not alter zebrafish larvae behaviour in the dark, and produce behavioural responses upon illumination.

Thus, in the first aspect, the present invention relates to a compound of formula (I): 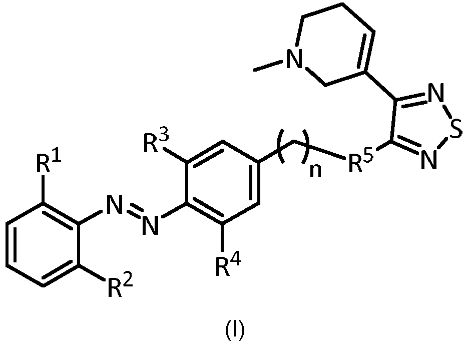 wherein
R¹, R², R³ and R⁴ are independently selected from the group consisting of H, halogen atom and C₁-C₃ alkoxy,
R⁵ is selected from O and S, and
n is an integer from 4 to 6,
with the proviso that R¹ and R² are not simultaneously F when n is 4,
or a pharmaceutically acceptable salt or stereoisomer thereof.

As shown in the examples, the value of n is highly relevant since compounds having n being 2, 3 or 7 (not encompassed by the invention) did not show a strong potency- or efficacy-shift upon irradiation, whereas compounds wherein n is 4-6 showed useful functional photoswitching.

In a second aspect, the present invention relates to a pharmaceutical composition comprising a compound according to the first aspect and a pharmaceutically acceptable excipient.

In a third aspect, the present invention relates to a compound according to the first aspect or pharmaceutical composition according to the second aspect, for use in medicine.

In a fourth aspect, the present invention relates to a compound according to the first aspect or pharmaceutical composition according to the second aspect, for use in the treatment and/or prevention of neurological disorders.

### DESCRIPTION OF THE FIGURES

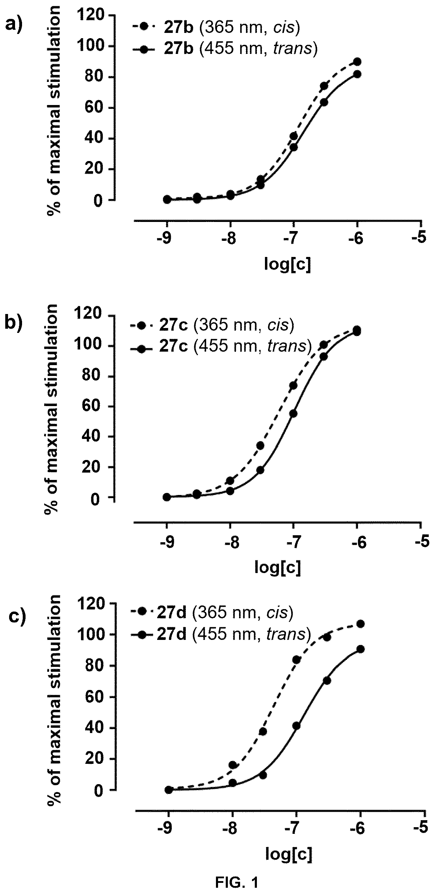

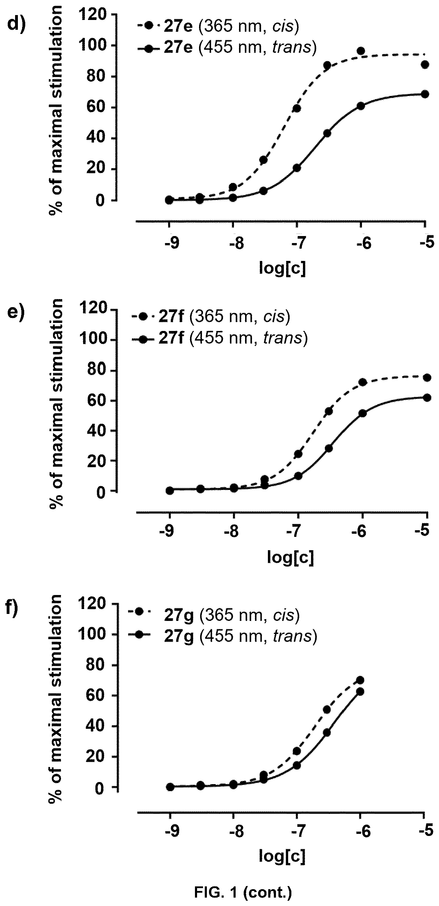

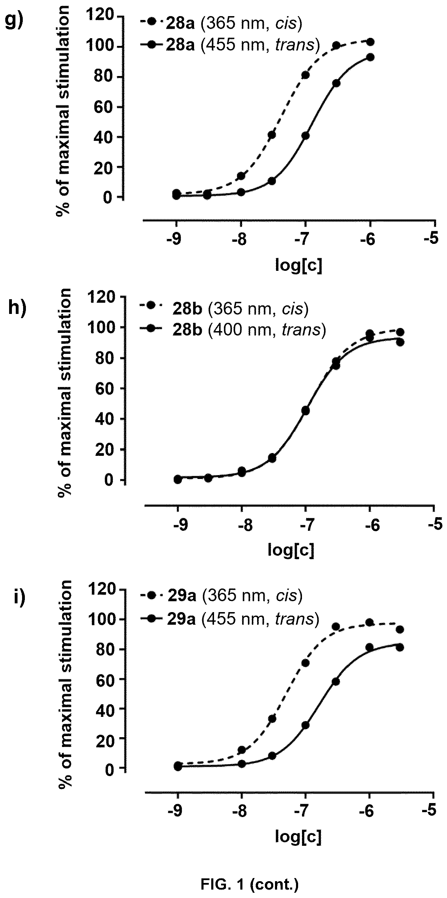

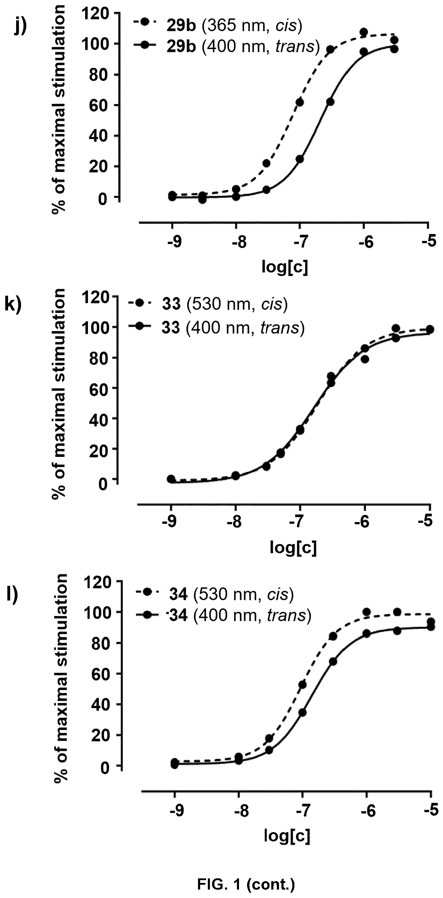

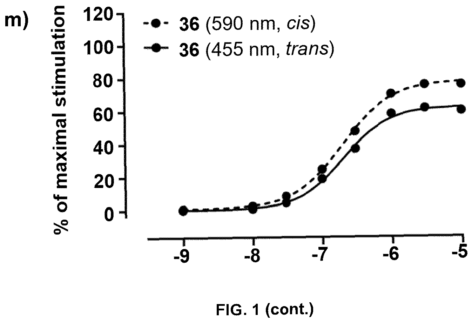

Figure 1 shows the *in vitro* characterization using a split luciferase complementation assay detecting the interaction between Gα_{q} and its effector phospholipase C-β3 (PLC-β3) as a quantifiable parameter of receptor activation. Concentration-response-curves of pre-illuminated compounds in either *cis-* or *trans*-form. Data is given as the mean AUC ± standard error of mean (SEM) of 3 independent experiments and normalized over the maximum response obtained with 10 µM carbachol.

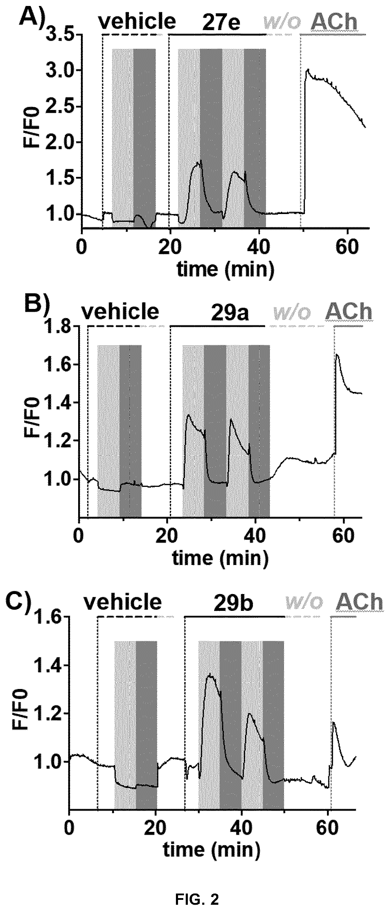

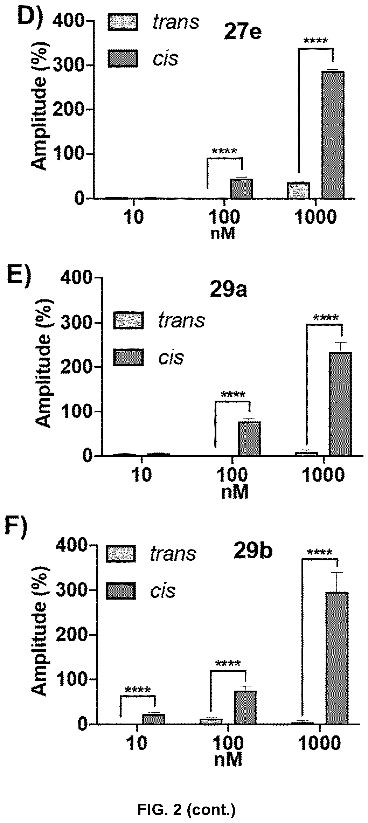

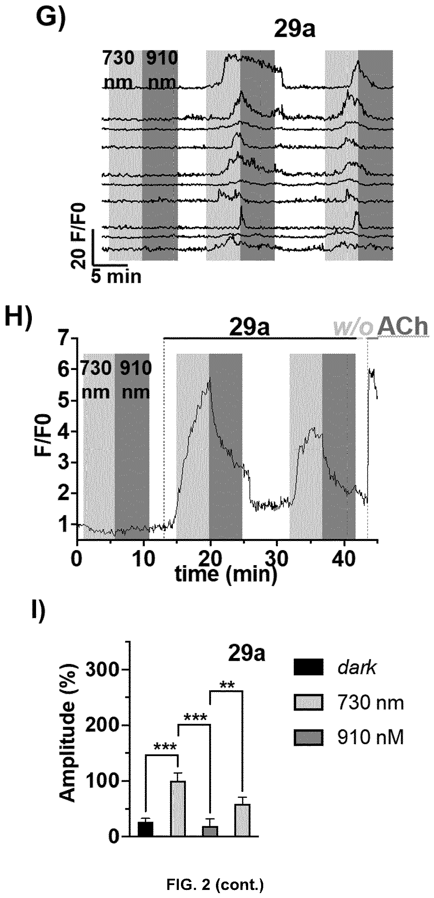

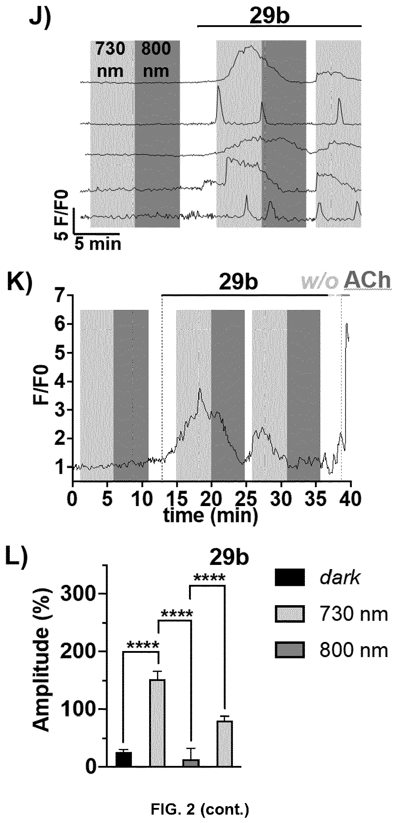

Figure 2 shows the *in vitro* characterization of photoswitchable M₁R ligands using a calcium fluorescence imaging under 1PE and 2PE conditions. (A,B,C) Time course of M₁R F/F0 responses in the presence of (A) 100 nM Example 27e (black bar, n = 37 cells), (B) 100 nM Example 29a (black bar, n = 62 cells) and (C) 100 nM Example 29b (black bar, n = 76 cells); 500 nM ACh (grey bar) served as positive control. Dashed light grey bar indicates wash-out (w/o) periods. Reversible photocontrol of the receptor under 1PE conditions was demonstrated by dynamic photomodulation of Ca²⁺ mobilization by irradiation with alternating wavelengths (Examples 27e and 29a 365/455 nm represented with light and dark grey area respectively; Example 29b 365/400 nm represented with light and dark grey area respectively). (D,E,F) Quantification of M₁R responses to (D) Example 27e, (E) Example 29a and (F) Example 29b showed significant differences between cis- (dark grey column) and trans-enriched (light grey column) PSSs at various concentrations 10 nM (Cell quantity: Example 27e: n = 51; Example 29a: n = 65; Example 29b: n = 43); 100 nM (Cell quantity: Example 27e: n = 37; Example 29a: n = 94; Example 29b: n = 48); 1000 nM (Cell quantity: Example 27e: n = 71; Example 29a: n = 48; Example 29b: n = 76). (G,H,J,K) Two-photon switching experiments of Example 29a (300 nM) and Example 29b (200 nM). Representative traces of calcium activity (F/F0) (G; Example 29a: n = 10 cells, J; Example 29b: n = 5 cells) and average (H; Example 29a: black bar n = 70 cells, K; Example 29b: black bar, n = 15 cells). Responses are triggered upon irradiation with 730 nm (light grey area) and are terminated under 910 nm (dark grey area), 800 nm illumination (dark grey area) using Examples 29a, 29b, respectively. Subsequent *trans* to *cis* isomerization under 730 nm light rapidly reactivates M₁R activity in both cases. Irradiation with indicated wavelengths in the absence of Examples 29a,b does not trigger any responses. (I,L) Quantification of M₁R responses to (I) Example 29a (300 nM; n = 71 cells) and (L) Example 29b (200 nM; n = 16 cells) showed reversible and significant bidirectional photoswitching under 2PE conditions. Statistical analysis of panel D, E, F, I and L were performed by the paired sample Wilcoxon signed rank test (**** < 0.0001; *** < 0.001; ** < 0.01) using GraphPad Prism 9. Error bars indicate ± SEM.

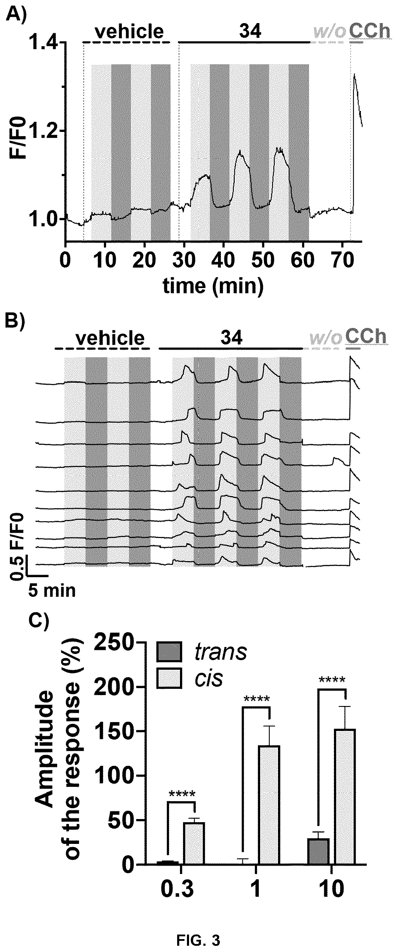

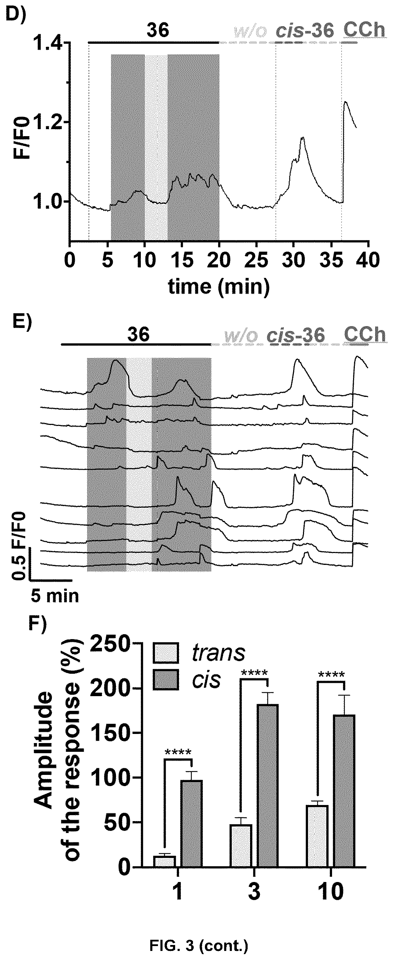

Figure 3 shows the *in vitro* characterization of compounds from Examples 34 and 36 using calcium fluorescence imaging. (A and D) Time course of M₁ mAChR FIFO responses in the presence of vehicle (black dashed bar) and (A) 0.3 µM Example 34 (black bar, n = 33 cells), (D) 1 µM Example 36 (black bar, n = 37 cells). Carbachol 10 µM (grey bar) was used as positive control. Dashed light and dark grey bars indicate wash-out (w/o) periods and application of the preilluminated *cis*-36, respectively. Example traces (B, n = 10 cells) and (E, n = 11 cells) of calcium activity (FIFO) for compounds of Examples 34 and 36, respectively. Photomodulation of the response by isomerization through 1PE was demonstrated by alternating 530 nm (light grey area) and 400 nm (dark grey area) for Example 34 (A and B) and 590 nm (dark grey area) and 455 nm (light grey area) for Example 36 (D and E). (C and F) Quantification of M₁ mAChR photoresponses by (C) Example 34, and (F) Example 36. (C) Example 34 showed significant differences between 530 nm and 400 nm at different concentrations 0.3 µM (n = 63 cells); 1 µM (n = 31 cells) and 10 µM (n = 65 cells). (F) Example 36 showed significant differences between 590 nm and 455 nm at different concentrations 1 µM (n = 102 cells); 3 µM (n = 102 cells) and 10 µM (n = 63 cells). Statistical analysis of panel C and F were performed by the paired sample Wilcoxon signed rank test (p-value (****) < 0.0001).

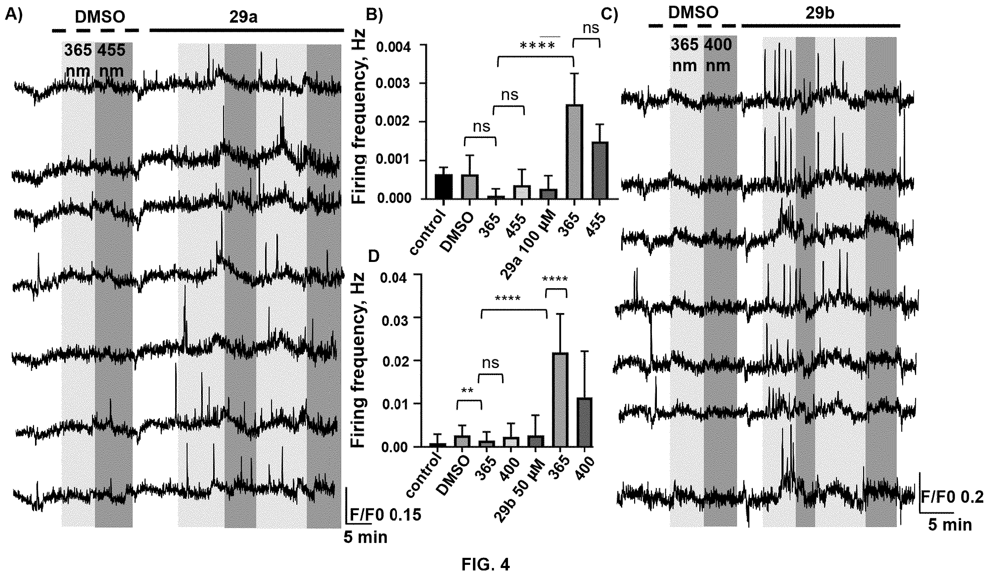

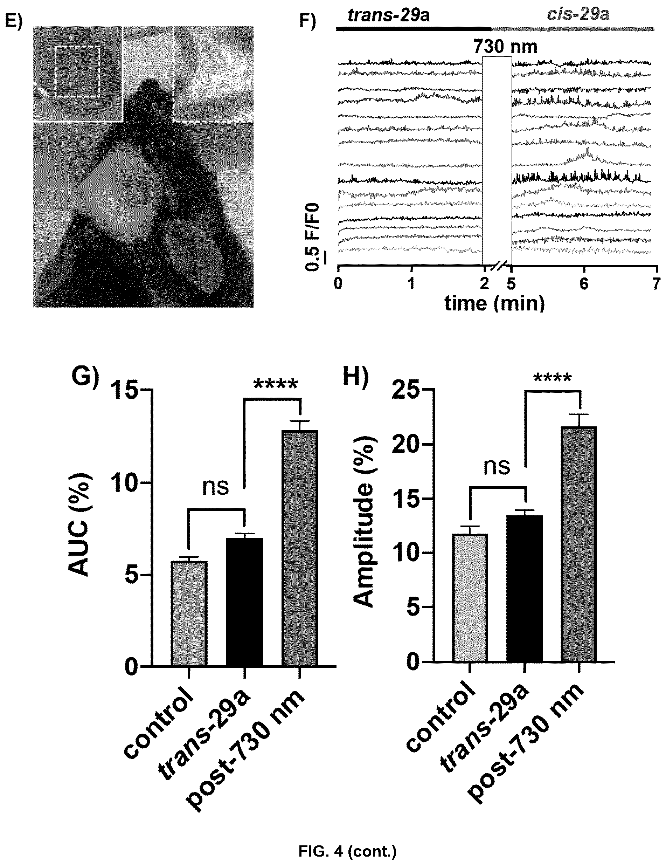

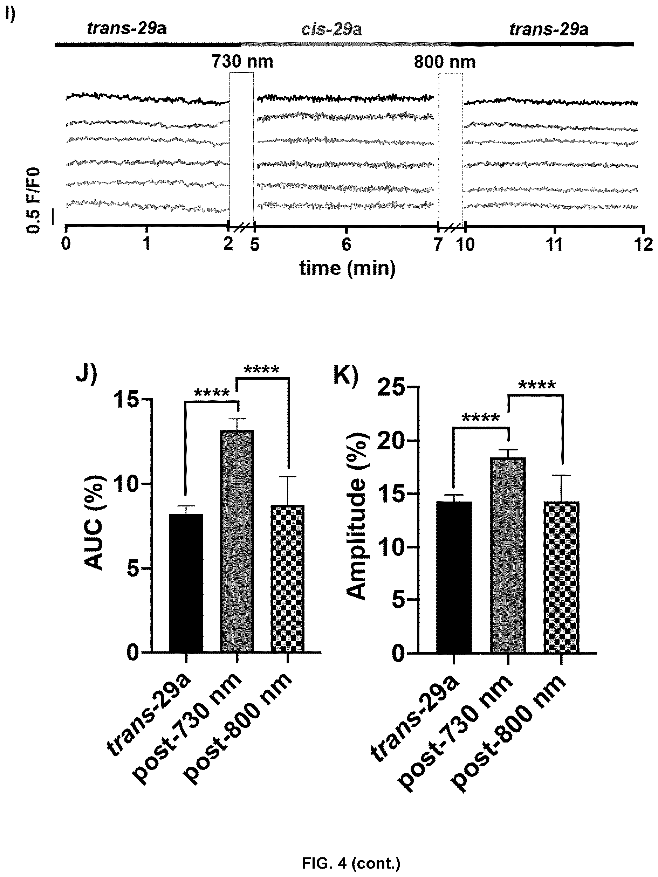

Figure 4 shows the light-dependently modulation of firing activity of hippocampal neurons *in vitro* (A-D) and *in vivo* (E-K) of compounds of Examples 29a,b. Bars above traces indicate application of DMSO (dashed black bar) or of the respective compound (black bar) in the given concentration. (A,C) Live-cell imaging recordings of OGB-1 fluorescence. Calcium transients were induced by UV light (365 nm) during application of Example 29a (100 µM) (A) or Example 29b (50 µM) (C). (B,D) Cumulative graph showing the effect of light on the frequency of generation of calcium transients in the presence of DMSO or test compound (B, Example 29a, 100 µM, 365 nm and 455 nm, indicated with light and dark grey areas; D, Example 29b, 50 µM, 365 nm and 400 nm, indicated with light and dark grey areas). Statistical analysis of panels B (32 cells, 4 independent experiments) and D (28 cells, 4 independent experiments) was performed with paired t-test (**** < 0.0001; ** < 0.01; ns - not significant). (E) Image of the cranial window and the Ca²⁺ activity in this region. (F) Representative traces (15 cells) of 2P-induced photoactivation of endogenous muscarinic responses in animals treated with Example 29a (100 µM). Photoconversion from the *trans* (black bar) to the *cis* form (grey bar) of 29a occurred upon 730 nm (white area outlined with continuous black line) (G,H) Quantification of photoresponses as AUC and amplitude (n = 68 cells, 4 animals). (I) Representative traces (6 cells) showing the bidirectional photocontrol of the endogenous muscarinic receptors, in animals injected with Example 29a. *Trans*-to-*cis* isomerization occurred upon 730 nm (white area outlined with continuous black line) and *cis*-to-*trans* isomerization upon 800 nm (white area outlined with dashed black line). Illumination at 730 nm was conducted at maximal laser power, illumination with 800 nm light at 50%, while 25% was used for imaging). (J,K) Quantification of photoresponses as AUC and amplitude (n = 16 cells, 1 animal). Statistical analysis of panel G, H, J and K were performed by the paired sample Wilcoxon signed rank test (**** < 0.0001) using GraphPad Prism 9. Error bars indicate ± SEM.

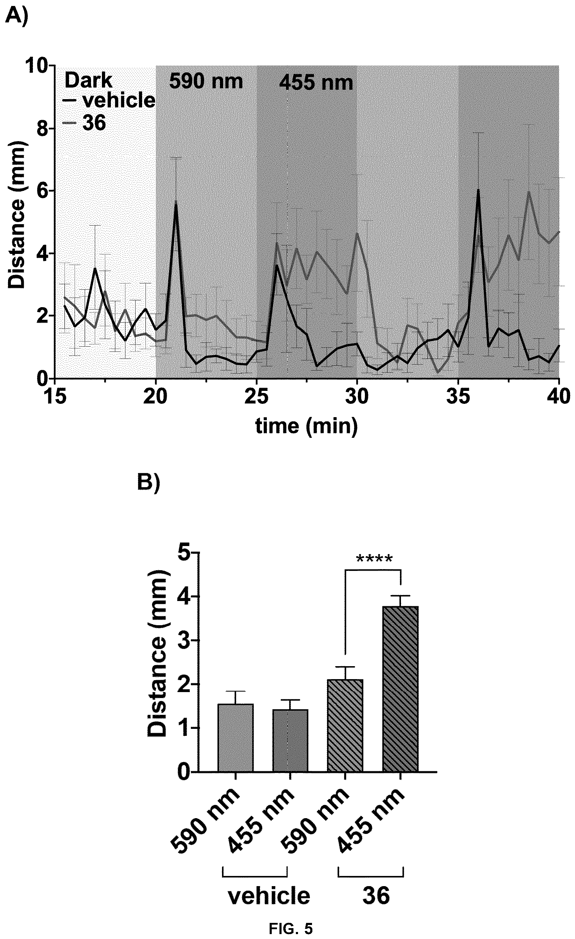

Figure 5 shows the behavioral effect of Example 36 in blinded zebrafish larvae. (A) One-minute trajectories of average swimming distances (n = 6) are shown for vehicle (0.3% DMSO, black line) and 36 (30 µM, grey line). In the first 20 min, larvae were undisturbed in complete darkness (relaxation period, RP). Following RP, larvae were illuminated with two consecutive cycles of amber-light (590 nm indicated with a light grey area; 2 mW) and bluelight (455 nm indicated with a dark grey area; 7 mW) light for 5 min each cycle. Error bars represent standard error of the mean (SEM). (B) Quantification of the total distances swum by the control group (vehicle, n = 6) and the treatment group (36, n = 6) during two consecutive cycles of 590 nm and 455 nm light. Data are mean ± SEM and were analyzed by two-way ANOVA followed by Tukey's post hoc test (p-value (****) < 0.0001 for vehicle 590 nm vs. *trans*-Example 36; p-value (****) < 0.0001 for vehicle 455 nm vs. *trans*-Example 36; p-value (****) < 0.0001 for cis-Example 36 vs. *trans*-Example 36) using GraphPad Prism 9. Error bars indicate ± SEM.

### DETAILED DESCRIPTION OF THE INVENTION

### Compounds of formula (I)

In the first aspect, the present invention relates to a compound of formula (I): 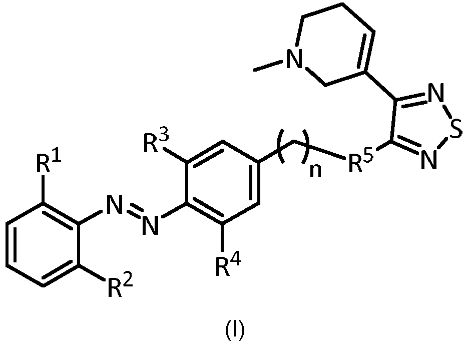 wherein
R¹, R², R³ and R⁴ are independently selected from the group consisting of H, halogen atom and C₁-C₃ alkoxy,
R⁵ is selected from O and S, and
n is an integer from 4 to 6,
with the proviso that R¹ and R² are not simultaneously F when n is 4,
or a pharmaceutically acceptable salt or stereoisomer thereof.

The term "halogen atom" as used herein alone or as part of another group refers to fluorine, chlorine, bromine and iodine atoms, preferably to fluorine and chlorine atoms, more preferably to fluorine atoms.

As used herein, the term "pharmaceutically acceptable salt" embraces salts with a pharmaceutically acceptable acid which are synthesized from the parent compound which contains a basic moiety by addition of a pharmaceutically acceptable acid. Pharmaceutically acceptable acids include both inorganic acids, for example, hydrochloric (HCl), sulfuric (H₂SO₄), phosphoric (H₃PO₄), diphosphoric (H₄P₂O₇), hydrobromic (HBr), hydroiodic (HI), and nitric acid (HNO₃), and organic acids, for example, citric, fumaric, maleic, malic, mandelic, ascorbic, oxalic, succinic, tartaric, benzoic, acetic (AcOH), methanesulfonic, ethanesulfonic, benzenesulfonic, or p-toluenesulfonic acid.

All stereoisomers of the compounds of this invention are contemplated either alone or as mixtures thereof. Stereoisomers refer to compounds having stereogenic centres, e.g., enantiomers, diastereomers, meso and racemic forms, and to compounds having different substituents on the atoms linked to form a multiple bond, such as a double or triple bond (e.g., -HC=CH-, -C≡C-, -N=N-, etc.), i.e. cis (*Z*) and trans (*E*) isomers. In particular, the compounds of the invention have *E-Z* stereoisomerism around the N=N double bond. When diastereomeric or enantiomeric products are prepared, they can be separated by conventional methods, for example, chromatographic or functional crystallization. When *Z* and *E* isomers of the compounds of formula (I) according to the present invention are prepared they can be reversibly interconverted from one isomer to the other isomer by irradiation at the appropriate wavelength, i.e., to obtain the *E* isomer from the *Z* isomer, a single-photon excitation by irradiation at a wavelength between 400 nm and 500 nm may be used, or a two-photon excitation by irradiation at a wavelength between 800 to 1000 nm may be used; and to obtain the *Z* isomer from the *E* isomer a single-photon excitation by irradiation at a wavelength between 350 nm and 650 nm may be used, or a two-photon excitation by irradiation at a wavelength between 700 to 1300 nm may be used. The specific wavelengths depend on the particular compound, but in any case, they fall under the ranges previously defined. For a particular compound, the wavelength to obtain the *E* isomer from the *Z* isomer does not overlap with that to obtain the *Z* isomer from the *E* isomer. The *Z* isomer of the compounds of formula (I) is the active form, whereas the *E*-isomer of the compounds of formula (I) is the inactive form. In one embodiment, the compounds of formula (I) are in the form of the *Z*-isomer. In another embodiment, the compounds of formula (I) are in the form of the *E*-isomer. The *Z*-isomer is then irradiated at the appropriate wavelength (as described above) to provide the active E-isomer.

In a preferred embodiment, in the compounds of formula (I), R⁵ is O.

In another preferred embodiment, in the compounds of formula (I) R¹, R², R³ and R⁴ are independently selected from the group consisting of H, halogen atom and methoxy; preferably R¹, R², R³ and R⁴ are independently selected from the group consisting of H, F, Cl and methoxy; more preferably R¹, R², R³ and R⁴ are independently selected from the group consisting of H and halogen atom; more preferably R¹, R², R³ and R⁴ are independently selected from the group consisting of H, F and Cl; still more preferably R¹, R², R³ and R⁴ are independently selected from the group consisting of H and F.

In a preferred embodiment, in the compounds of formula (I), the halogen atom is independently selected from F and Cl; preferably the halogen atom is F.

In another preferred embodiment, in the compounds of formula (I), R³ and R⁴ are both H.

In one embodiment, R¹ and R² are both H.

In another embodiment, R¹, R², R³ and R⁴ are H.

In another embodiment, one of R¹ and R² is H and the other one is halogen atom; preferably one of R¹ and R² is H and the other one is F or Cl; more preferably one of R¹ and R² is H and the other one is F.

In another embodiment, one of R¹ and R² is H and the other one is halogen atom, and R³ and R⁴ are both H; preferably one of R¹ and R² is H and the other one is F or C!, and R³ and R⁴ are both H; more preferably one of R¹ and R² is H and the other one is F, and R³ and R⁴ are both H.

In another preferred embodiment, in the compounds of formula (I):
- R¹ and R² are both H,
- one of R¹ and R² is H and the other one is F, or
- R¹ and R² are both F.

In another preferred embodiment, in the compounds of formula (I):
- R¹ and R² are both H, or
- one of R¹ and R² is H and the other one is F.

In a particular embodiment, in the compounds of formula (I), R³ and R⁴ are both H and:
- R¹ and R² are both H,
- one of R¹ and R² is H and the other one is F, or
- R¹ and R² are both F.

In another particular embodiment, in the compounds of formula (I), R³ and R⁴ are both H and:
- R¹ and R² are both H, or
- one of R¹ and R² is H and the other one is F.

In another embodiment, in the compounds of formula (I), R¹, R², R³ and R⁴ are halogen atom, preferably R¹, R², R³ and R⁴ are simultaneously F or R¹, R², R³ and R⁴ are simultaneously Cl. In a preferred embodiment, in the compounds of formula (I), R¹, R², R³ and R⁴ are simultaneously F. In another preferred embodiment, in the compounds of formula (I), R¹, R², R³ and R⁴ are simultaneously Cl.

In a particular embodiment, in the compounds of formula (I), R¹, R², R³ and R⁴ are simultaneously C₁-C₃ alkoxy, preferably methoxy.

In a preferred embodiment, in the compounds of formula (I), n is 4 or 5. In one embodiment n is 4. In another embodiment n is 5.

In a preferred embodiment, the compound of formula (I) is selected from the group consisting of:
3-(1-methyl-1,2,5,6-tetrahydropyridindin-3-yl)-4-(4-(4-(phenyldiazenyl)phenyl)butoxy)-1,2,5-thiadiazole,
3-(1-methyl-1,2,5,6-tetrahydropyridin-3-yl)-4-((5-(4-(phenyldiazenyl)phenyl)-pentyl)oxy)-1,2,5-thiadiazole,
3-(1-methyl-1,2,5,6-tetrahydropyridin-3-yl)-4-((6-(4-(phenyldiazenyl)phenyl)hexyl)-oxy)-1,2,5-thiadiazole,
3-(4-(4-((2-fluorophenyl)diazenyl)phenyl)butoxy)-4-(1-methyl-1,2,5,6-tetrahydropyridin-3-yl)-1,2,5-thiadiazole,
3-((5-(4-((2-fluorophenyl)diazenyl)phenyl)pentyl)oxy)-4-(1-methyl-1,2,5,6-tetrahydropyridin- 3-yl)-1,2,5-thiadiazole,
3-((5-(4-((2,6-difluorophenyl)diazenyl)phenyl)pentyl)oxy)-4-(1-methyl-1,2,5,6-tetrahydropyridin-3-yl)-1,2,5-thiadiazole,
3-((5-(4-((2,6-difluorophenyl)diazenyl)-3,5-difluorophenyl)pentyl)oxy)-4-(1-methyl-1,2,5,6-tetrahydropyridin-3-yl)-1,2,5-thiadiazole, and
3-((5-(3,5-dichloro-4-((2,6-dichlorophenyl)diazenyl)phenyl)pentyl)oxy)-4-(1-methyl-1,2,5,6-tetrahydropyridin-3-yl)-1,2,5-thiadiazole,
or a pharmaceutically acceptable salt or stereoisomer thereof.

As defined in the first aspect, the compounds of formula (I) of the invention do not include those wherein n is 4, and R¹ and R² are both F, i.e. compounds having formula (Ia) shown below: 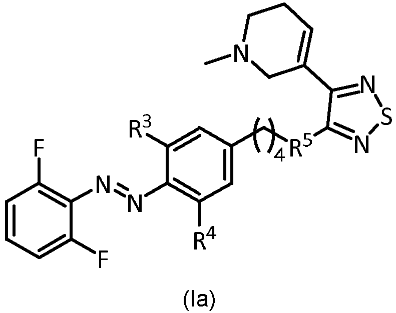 wherein R³, R⁴ and R⁵ are as previously defined.

### Synthesis of the compounds of formula (I)

The compounds of formula (I) may be synthesized by using some of the methods described below as well as other processes known in the field of organic chemistry.

In particular, compounds of formula (lb), i.e. compounds of formula (I) wherein R¹-R⁴ and n are as defined above and wherein R⁵ is O, may be synthesized following the route depicted in Scheme 1.

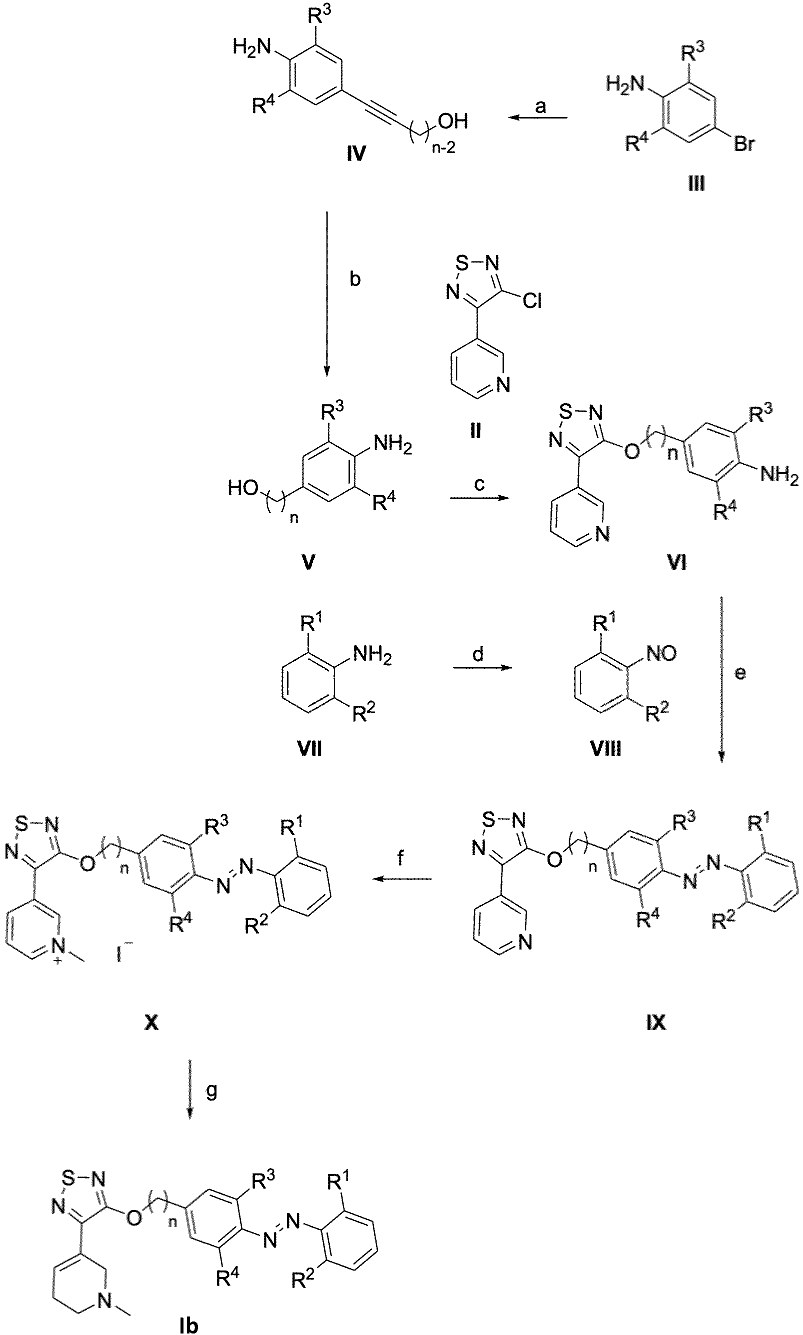

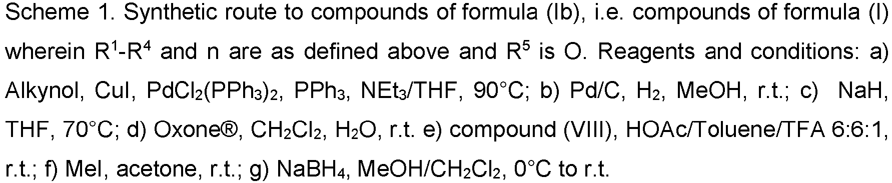

Starting from compound (III) an alkynol with the desired length may be introduced by Sonogashira cross coupling reaction yielding the corresponding aminoalkynol (IV). Alternatively, compound (IV) may be synthesized by reaction of the corresponding 1-bromo-4-nitrobenzene with the alkynol of the desired length also under the reaction conditions described for step a) above and subsequent reduction of the nitro group to an amino group and simultaneous reduction of the triple bond using reaction conditions described for step b) above. Subsequent palladium-catalyzed hydrogenation of the alkynol gives the saturated aminoalcohols (V). In a nucleophilic aromatic substitution, employing Williamson ether synthesis reaction conditions, these alcohols may be coupled to the thiadiazol core (II) resulting in thiadiazolephenyl aniline derivative (VI). The thiadiazole core fragment (II) may be synthesized according to literature [Sauerberg, P. et al., J. Med. Chem., 1992, 35 (12), 2274-83]. Starting from nicotinaldehyde a two-step Strecker-like synthesis may be employed to form the aminonitrile derivative. Subsequent cyclization of said aminonitrile derivative using sulfur monochloride in DMF gives the desired 3-chloro-4-(pyridin-3-yl)-1,2,5-thiadiazole (II). In a Baeyer-Mills reaction the azobenzene compounds (IX) may be formed, condensing the aniline (VI) with the respective nitrosobenzene (VIII) in a mixture of acetic acid, THF and trifluoro acetic acid. Nitrosobenzene (VIII) may be synthesized by partial oxidation of the corresponding aniline (VII) in a heterogeneous water/dichloromethane system using Oxone^{®} and may be directly used without further purification. Quaternization of compound (IX) may be performed by N-methylation of the pyridine with iodomethane in acetone and may be followed by reduction of the resulting pyridinium cation (X) with sodium tetra borohydride in methanol to regioselectively yield the desired compounds of formula (Ib), i.e. compounds of formula (I) wherein R¹-R⁴ and n are as defined above and R⁵ is O.

Tetra-ortho-chlorinated compounds of formula (Ic), i.e. compounds of formula (I) wherein R¹-R⁴ are Cl, comprising an alkylene linker of 4-6 methylene units, i.e. n=4, 5 or 6, and wherein R⁵ is O, may also be synthesized following the route depicted in Scheme 2.

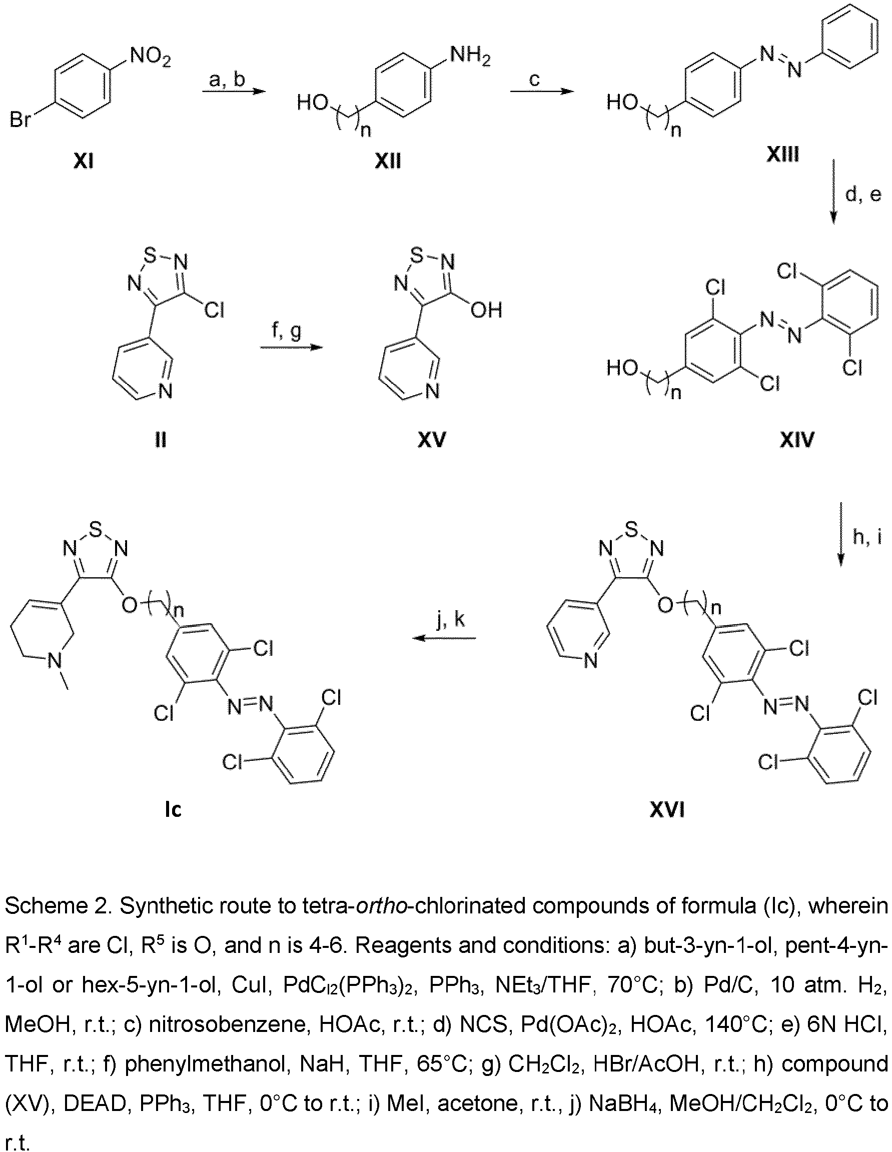

Using Sonogashira cross coupling reaction, followed by catalytic hydrogenation (4-aminophenyl)alkanC₄-C₆-1-ol compound (XII) may be synthesized. In a Baeyer-Mills reaction compound (XII) may be reacted with commercially available nitrosobenzene yielding (4-(phenyldiazenyl)phenyl) alkanC₄-C₆-1-ol compound (XIII). Functionalization by late stage chlorination [Konrad, D. B. et al., Chemistry - A European Journal, 2016, 22 (13), 4364-4368] followed by ester saponification of the resulting acetate may be conducted to obtain the desired tetra-*ortho*-chlorinated azobenzene (XIV). The necessary aromatic alcohol (XV) may be synthesized by nucleophilic aromatic substitution of compound (II) with benzyl alcohol followed by benzyl deprotection using HBr in AcOH. The resulting hydroxyarene may then be coupled to compound (XIV) using standard Mitsunobu conditions to yield compound (XVI). Subsequent quaternization and reduction may be carried out as described above to yield the tetra-*ortho*-chlorinated-compounds of formula (Ic), i.e. compounds of formula (I) wherein R¹-R⁴ are C!, R⁵ is O, and n is 4-6.

Compounds of formula (Id), i.e. compounds of formula (I) wherein R¹-R⁴ and n are as defined above and wherein R⁵ is S, may be synthesized following the route depicted in Scheme 3.

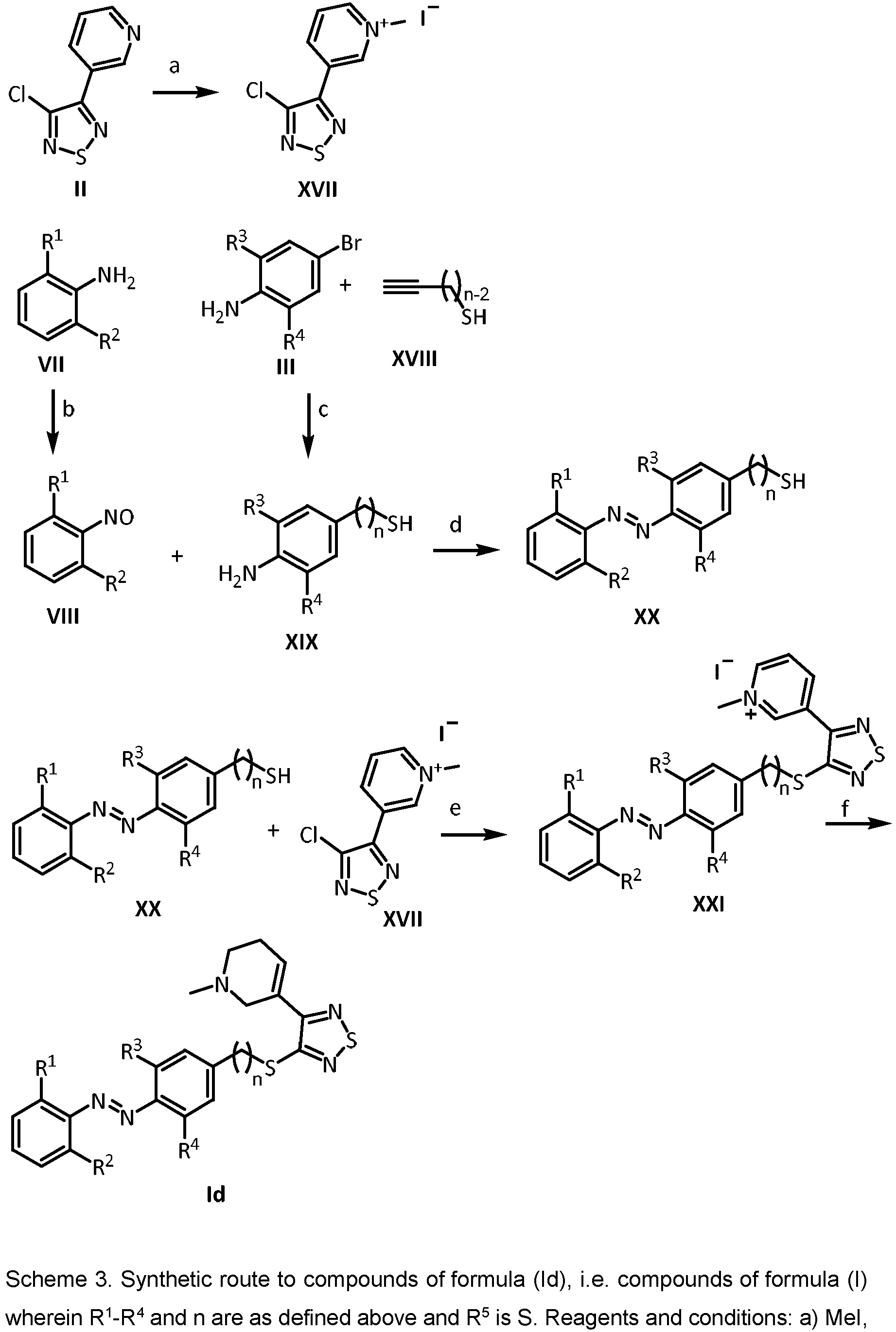

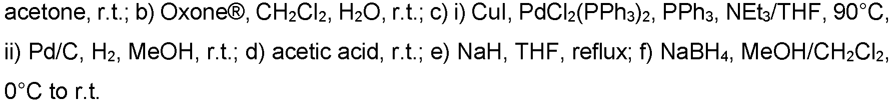

Compound (XIX) may be synthesized by reaction of the corresponding 1-bromo-3-R₃-5-R₄-4-aminobenzene derivative with the alkynethiol of the desired length under the reaction conditions described for step c) above. These thiols may be coupled to the nitrosobenzene derivative (VIII) resulting in azobenzene compounds (XX). Nitrosobenzene (VIII) may be synthesized by partial oxidation of the corresponding aniline (VII) in a heterogeneous water/dichloromethane system using Oxone^{®} and may be directly used without further purification. The thiadiazole core fragment (II) may be synthesized according to literature [Sauerberg, P. et al., J. Med. Chem., 1992, 35 (12), 2274-83]. Starting from nicotinaldehyde a two-step Strecker-like synthesis may be employed to form the aminonitrile derivative. Subsequent cyclization of said aminonitrile derivative using sulfur monochloride in DMF gives the desired 3-chloro-4-(pyridin-3-yl)-1,2,5-thiadiazole (II). Quaternization of said compound (II) may be performed by N-methylation of the pyridine with iodomethane in acetone. Compound (XX) is condensed with compound (XVII) using sodium hydride in THF and under reflux to five compound (XXI), followed by reduction of the resulting pyridinium cation in compound (XXI) with sodium tetra borohydride in methanol to yield the desired compounds of formula (Id), i.e. compound of formula (I) wherein R¹-R⁴ and n are as defined above and R⁵ is S.

Compounds of formula (le), i.e. compounds of formula (I) wherein R¹-R⁴ are C₁-C₃ alkoxy (Y in Scheme 4 below), such as methoxy, n is as defined above and R⁵ is O, may also be synthesized following the route depicted in Scheme 4. The reaction conditions are as previously described.

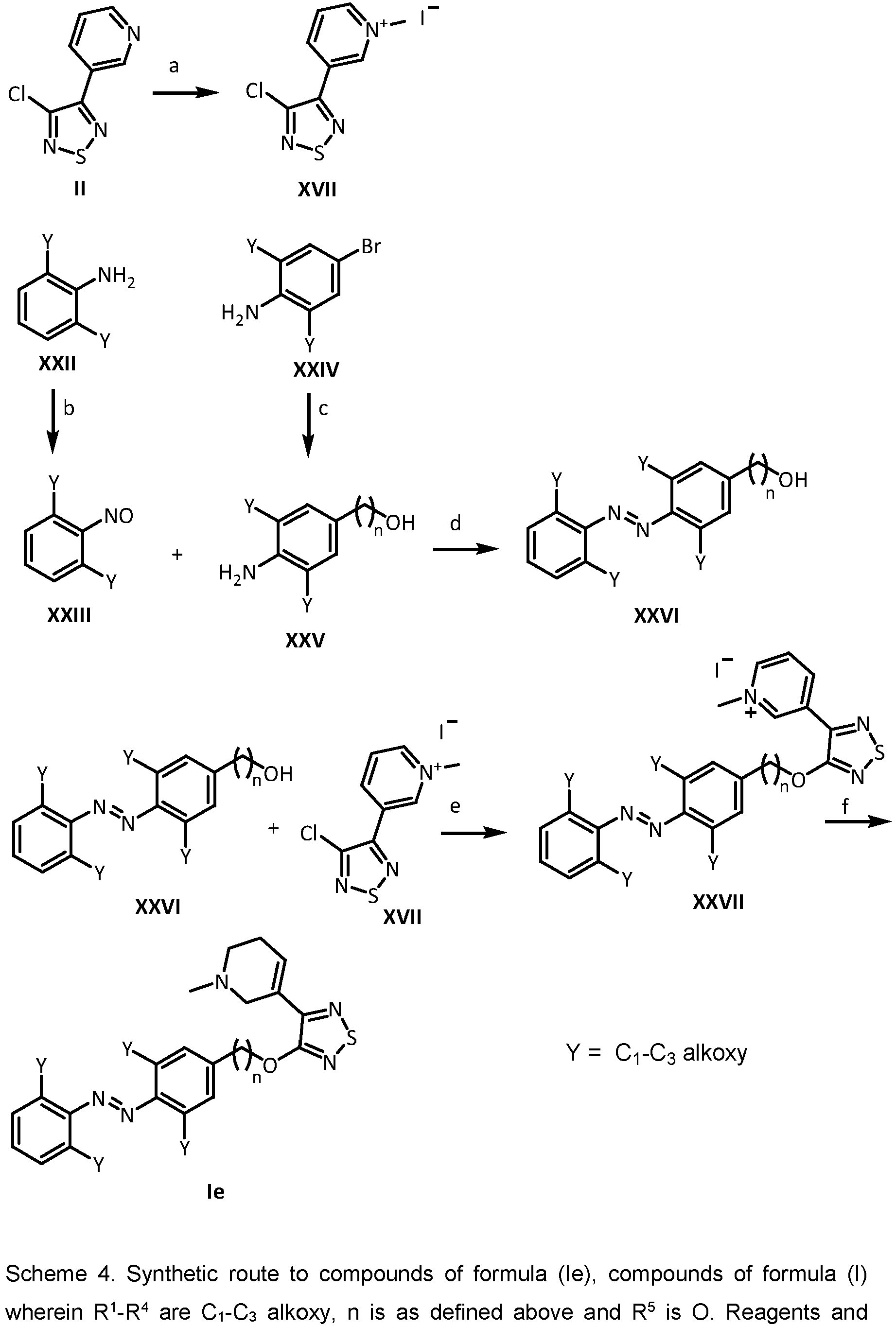

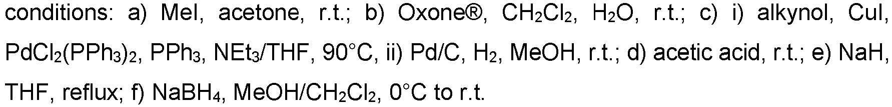

### Pharmaceutical compositions comprising the compound of formula (I)

In a second aspect, the invention is directed to a pharmaceutical composition comprising a compound of formula (I) as defined above, and a pharmaceutically acceptable excipient.

The term "pharmaceutically acceptable excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similar, surfactant, bile acid, chelating agents, preservatives, cyclodextrins, among others. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005. Other suitable excipients are cyclodextrins and polyethylene glycols (e.g., liquid PEGs such as PEG₄₀₀), glycine, histidine, lactic acid, tartaric acid, benzyl alcohol, glucose, sorbitol, sucrose, glycerol, oleic acid, and polyvinyl alcohol. Surfactants, bile acids, chelating agents, preservatives, and cyclodextrins increase chemical stability and bioavailability and decrease local irritation [Brian G. Short, Toxicologic Pathology, 2008, 36(1), 49-62].

Compounds formula (I) according to the present invention may be administered by the topical, oral, sublingual, parenteral, subcutaneous, intramuscular, intravenous, transdermal, intranasal, and/or rectal routes.

The compounds may be administered alone or in combination with one or more other compounds of the invention or one or more other drugs. In general, the compounds should be administered as a formulation in association with one or more pharmaceutically acceptable excipients. Any administration method commonly used for drugs, such as solutions (including eye drops), cream, ointment, powder, tablets, coated tablets, capsules, syrup, powder, and suppository, may be used. The pharmaceutical composition can be formulated employing conventional liquid or solid vehicles or diluents and pharmaceutical additives, according to the desired mode of administration. The mentioned formulations will be prepared using standard methods, such as those described or referred to in the Spanish, E.U., and U.S. Pharmacopoeias and similar reference texts.

The compounds of formula (I) may be administered in a therapeutically effective amount. "Effective" amount or a "therapeutically effective amount" of a compound is meant a nontoxic but sufficient amount of the drug or agent to provide the desired effect. The amount that is "effective" will vary from subject to subject, depending on the age and general condition of the individual, the particular active agent or agents, and the like. Thus, it is not always possible to specify an exact "effective amount". However, an appropriate "effective" amount in any individual case may be determined by one of ordinary skill in the art using routine experimentation. The compounds will typically be administered once or more times a day, for example 1, 2, 3 or 4 times daily.

### Medical uses of the compound of formula (I)

As shown in the examples, the compounds of formula (I) show reversible muscarinic modulation of brain activity, which do not produce any effect in the absence of illumination, whereas upon illumination at 1PE and 2PE they show a muscarinic acetylcholine receptor agonistic activity.

Thus, in a third aspect, the present invention provides a compound of formula (I) as defined above, or a pharmaceutical composition comprising a compound of formula (I) as defined above, for use as a medicament.

The invention also relates to the use of a compound of formula (I) as defined above, or a pharmaceutical composition comprising a compound of formula (I) as defined above, for the manufacture of a medicament.

In a fourth aspect, the present invention provides a compound of formula (I) as defined above, or a pharmaceutical composition comprising a compound of formula (I) as defined above, for use in the treatment and/or prevention of neurological disorders, in particular schizophrenia, Alzheimer's disease, Parkinson's disease, stroke, traumatic brain injury, sleep disorders, and coma state.

The present invention also relates to the use of a compound of formula (I) as defined above, or a pharmaceutical composition comprising a compound of formula (I) as defined above, for manufacturing a medicament for the treatment and/or prevention of neurological disorders, in particular schizophrenia, Alzheimer's disease, Parkinson's disease, stroke, traumatic brain injury, sleep disorders, and coma state.

The invention also relates to a method of treatment and/or prevention of a subject in need thereof of neurological disorders, in particular schizophrenia, Alzheimer's disease, Parkinson's disease, stroke, traumatic brain injury, sleep disorders, and coma state, which comprises the administration of a compound of formula (I) as defined above, or a pharmaceutical composition comprising a compound of formula (I) as defined above.

The terms "treat", "treatment", or "treatment of" as used herein refer to reducing the potential for a certain disease or disorder, reducing the occurrence of a certain disease or disorder, and/or a reduction in the severity of a certain disease or disorder, preferably, to an extent that the subject no longer suffers discomfort and/or altered function due to it. It also refers to mitigating or decreasing at least one clinical symptom and/or inhibition or delay in the progression of the condition and/or prevention or delay of the onset of a disease or illness.

The terms "prevention", "preventing" or "prevent" as used herein refer to avoiding the appearance of a certain disease or disorder. The prevention can be complete (e.g., the total absence of a disease). The prevention can also be partial, such that for example the occurrence of a disease in a subject is less than that which would have occurred without the administration of the combination or composition of the present invention. Prevention also refers to reduced susceptibility to a clinical condition. The prevention also includes reducing the risk of suffering the disease.

The subject to which the compounds and pharmaceutical compositions described herein are administered is a human or animal; preferably subjects are mammals, more preferably humans.

In all cases, the therapeutic effects of the compounds of formula (I) are released locally and on demand without concomitant adverse effects in non-illuminated regions of the body.

The following examples represent specific embodiments of the present invention. They do not intend to limit in any way the scope of the invention defined in the present description.

### EXAMPLES

### Methods and Materials

Common reagents and solvents were purchased from the following commercial suppliers: ABCR, Alfa Aesar, BLD-Pharm, Sigma-Aldrich, Merck, TCI Chemicals and used without further purification unless otherwise stated. Dry tetrahydrofuran (THF) was freshly distilled from sodium-benzophenone under an argon atmosphere. Reaction progress was monitored using analytical thin-layer chromatography (TLC) on precoated silica gel 60 GF254 plates (Macherey Nagel GmbH & Co. KG, Düren, Germany). Detection was carried out by irradiation and consequent fluorescence quenching at 254 nm or excitation at 365 nm. Compounds were purified by column chromatography using silica gel 60 (60 Å pore size, 40-63 µm; Macherey Nagel GmbH & Co. KG, Düren, Germany) as the stationary phase.

Reverse phase column chromatography was performed on an Interchim Puri Flash 430 (Ultra Performance Flash Purification) instrument connected to an Interchim Flash ELSD. FlashPure 4 g or 12 g C18 columns were used with gradients of H₂O/MeOH as eluent systems. Nuclear magnetic resonance spectra were measured on a Bruker AV-400 NMR instrument (Bruker, Karlsruhe, Germany) in deuterated solvents (DMSO-d₆, CDCl₃, MeOD-d₄, THF-ds). Chemical shifts are expressed in ppm relative to DMSO-d₆, CDCl₃, MeOD-d₄, THF-d₈ (2.50; 7.26; 3.31; 3.58/1.73 for ¹H; 39.52; 77.16; 49.00; 67.57/25.37 for ¹³C).

Measurements for verification and purity of the compounds were performed with a Shimadzu LC/MS system, comprising a DGU-20A3R controller, pump LC-20AB, degasser DGU-20A, and SPD-20A UV/Vis detector. Compounds were dissolved in MeOH and filtered through syringe filters prior to measurement. As a stationary phase, a Synergi 4U fusion-RP 80 Å (150 × 4.6 mm) column was used (Phenomenex). As a mobile phase, a gradient of H₂O/MeOH (both containing 0.1% formic acid) was used. Method: flow rate: 1.0 mL/min; detection: 254 nm; scan range: 60-1000 m/z; gradient: A: H₂O (0.1 % HCOOH); B: MeOH (0.1 % HCOOH) 0-8 min 5 %→90 % B, 8-13 min 90 % B, 13-14 min 90 %→5 % B, 14-18 min 5 % B. The purity of all target compounds was found to be ≥95%. Purities were not tested at a particular photostationary state and therefore show an arbitrary cis/trans ratio (cf. purity chromatograms). ESI ionization was accomplished by a downstream Shimadzu LCMS-2020 mass spectrometer. Data are reported as mass-to-charge ratio (m/z) of the corresponding positively charged molecular ions.

### Determination of Photophysical Properties

UV/Vis spectra were recorded on a Varian Cary 50 Bio UV/Vis Spectrophotometer connected to a water bath for temperature control. Hellma (Type 100-QS) cuvettes (10mm light path) were used. Characterization was performed with 25 µM/50 µM solutions in DMSO/H₂O 1:1 (V:V). Samples were irradiated using LEDs of Seoulviosys (UV Z5 series CUNx6A1B; 365 nm), Roschwege (RSW-P01-385-2; 385 nm), LedEngin (LZ4-00UB00-U7, 405 nm), Osram (LD CQ7P-2U3U-W5-1-K, 451 nm; LT CP7P-KZLX-26, 528 nm; LY CP7P-JUKQ-36, 590 nm; LA CP7P-KQKS-W3, 617 nm), Cree (XPeBBL-L1-0000-00205, 475 nm) and Lumileds (LXML-PE01, 505 nm). LED modules were combined to a rated power of 4W and used with a forward current of IF = 350 mA. Irradiation of the solutions with the respective wavelengths were performed until the corresponding photostationary state (PSS) had equilibrated (1-2 min). Thermal relaxation was measured for a period over 2 h to determine whether the relaxation was significant with regards to the duration of the biological assays. For this purpose, the photochromic compounds were switched into the maximally *trans* enriched photostationary state. The absorption at the wavelength were the spectrum of the *cis-* and *trans*-isomer showed maximal difference (λ_{Δmax}) was measured for 15 min. Subsequently, the solutions were switched to the thermodynamically less stable *cis* isomer using 365 nm light. The relaxation was recorded by monitoring the change of absorption at λ_{Δmax} for 2 hours at 37 °C. Afterwards, the solutions were switched to their maximally *trans* enriched PSS again. Percentage relaxation was calculated as Δ = (A_{*cis* enriched t = 2h} - A_{*cis* enriched t = 0}) * 100 / (A_{*trans* enriched} - A_{*cis* enriched t = 0}). Absence of photo-fatigue was checked using either the described above Varian Cary UV/Vis Spectrophotometer plus Hellma (Type 100-QS) cuvettes or in case of characterization of multiple compounds at the same time, a 96-well plate and a Spectramax 250 absorbance microplate reader (molecular devices) were used. The compounds were irradiated alternatingly with the indicated wavelengths and their absorbance at λ_{Δmax} was checked to be constant. The photostationary distributions (PSD) of the PSSs of interests were determined by HPLC (see above). The amount of *cis* and *trans* isomer was quantified by integration of the respective peak. Absorption was measured at the respective isosbestic point.

### Split Luciferase Complementation Assay

The activity of the compounds was determined by means of a HEK293T (Human Embryonic Kidney) cell line, stably expressing the human muscarinic acetylcholine receptor 1 (*h*M₁R) and a split luciferase complementation system: The G_{aq} subunit fused to a click-beetle luciferase fragment and the phospholipase C β3 fused to the corresponding fragment [Littmann, T. et al., Sci. Rep., 2018, 8 (1), 17179]. Cells were routinely monitored for mycoplasma contamination and were negative. The cells were cultivated in Dulbecco's modified Eagle's medium (DMEM) containing 10% FCS (full medium), 0.6 mg/mL G418 and 0.001 mg/mL Puromycin, at 37 °C in a water-saturated atmosphere containing 5% CO₂. On the evening before the assay, the cells were detached from a 75-cm² flask by trypsinization and centrifuged. The pellet was resuspended in assay medium consisting of Leibovitz' L-15 (w/o phenol red) with 5% FCS and 10 µM HEPES and the density of the suspension was adjusted to 1.25*10⁶ cells/mL. Then, 80 µl of the cells were seeded in Nunc^{™} MicroWell^{™} 96 well plates (Thermo Scientific: 136101) and incubated overnight in a humidified atmosphere at 37°C without additional CO₂. For the assay, 10 µl of 10 mM luciferin (Thermo Scientific: 88293) in L-15 were added to each well and the cells were allowed to equilibrate for 10 min at 37°C. Afterwards, 10 µl per concentration of a pre irradiated ligand dilution series was added (stock solution of the test compound was 10 mM in DMSO, which was diluted down to (100 µM) - (30 µM) - 10 µM - 3000 nM - 1000 nM - 300 nM - 100 nM - 10 nM) with Leibovitz' L-15 (w/o phenol red)), yielding final in-well concentrations of (10 µM) - (3 µM) - 1 µM - 300 nM - 100 nM - 30 nM - 10 nM - 1 nM). Afterwards the plate was read in a pre-warmed Berthold Mithras LB 940. Additionally, a duplicate of the reference substance Carbachol and a negative control (L-15) was included in each individual experiment. The luminescent signal was continuously monitored for 30 min after which a stable plateau had formed for every concentration. Each substance was measured in at least three independent experiments, in which each condition was tested in duplicate. Data was analyzed by taking the last 5 data points of each plateau and plotting them in GraphPad Prism 5. These values were normalized to the maximal response of reference agonist CCh (100%) and the negative control L-15 (0%). Afterwards, the values of the independent experiments were pooled to generate the overall concentration-response curves which were fitted using Prisms built-in log(agonist) vs. response equation.

### Cell Culture and Transient Transfection

Human Embryonic Kidney tsa201 (HEK tsa201, American Type Culture Collection; ATCC) cells were maintained in Dulbecco's Modified Eagle's Medium/Nutrient Mixture F-12 Ham (DMEM/F12 1:1, Life Technologies) supplemented with 10% fetal bovine serum (FBS, Life Technologies), penicillin and streptomycin (1%, Sigma-Aldrich) in a controlled environment (37 °C, 98% humidity and 5% CO₂). The cells were transiently co-transfected with the human M₁ receptor (Addgene) and the green or the red genetically encoded calcium indicators (GCaMP6s or R-GECO1; ratio 1:1) using X-tremeGENE 9 DNA Transfection Reagent (Roche Applied Science) following the manufacturer's instructions. As generally known, M₁ mAChRs prevalently activate Gq proteins, leading to the activation of the phospholipase C pathway, which results in the production of inositol 1,4,5-trisphosphate (IP3) and the subsequent release of intracellular calcium from the endoplasmic reticulum. The day after, cells were harvested with accutase (Sigma-Aldrich) and seeded onto 16 mm or 25 mm (1PE or 2PE experiments) glass coverslips (Fisher Scientific) coated with poly-L-lysine (Sigma-Aldrich) to allow cell adhesion. Finally, the seeded cells were used for the experiments after 24 h.

### In Vitro Single-Cell Calcium Imaging

The calcium imaging assay was performed in the HEK tsa201 cell line, using a calcium indicator to determine changes in intracellular calcium concentrations upon receptor activation. In the experiments involving compounds 27e, 29a and 29b HEK cells were cotransfected with M₁R and GCaMP6s. Whereas, in the studies using compounds 34 and 36 HEK cells were cotransfected with M₁R and R-GECO1. The bath solution used for single cell intracellular calcium recordings contained: 140 mM NaCl, 5.4 mM KCI, 1 mM MgCl₂, 10 mM HEPES, 10 mM glucose and 2 mM CaCl₂, with pH 7.40. Before each experiment, cells were mounted on the recording chamber and rinsed with fresh solution. Then, the recording chamber was filled with 1ml of the bath solution and placed on an IX71 inverted microscope (Olympus) with a XLUMPLFLN 20XWx20/1 water immersion objective (Olympus). GCaMP6s was excited during 50 ms at 488 nm by using a Polychrome V light source (Till Photonics) equipped with a Xenon Short Arc lamp (Ushio) and a 505 nm dichroic beam splitter (Chroma Technology). Emission at 510 nm was filtered by a D535/40 nm emission filter (Chroma Technology) and finally collected by a C9100-13 EM-CCD camera (HAMAMATSU). However, R-GECO1 was excited during 50 ms at 562 nm by using a Polychrome V light source (Till Photonics GmbH) equipped with a Xenon Short Arc lamp (Ushio Europe B.V) and a 585 nm dichroic beam splitter (Chroma Technology). The emission at 600 nm was filtered by ET630/75nm emission filter (Chroma Technology) and collected by a C9100-13 EM-CCD camera (Hamamatsu Photonics). Images were acquired at room temperature with an imaging interval of 4 s with the SmartLux software (HEKA), and the imaging analysis was done with FIJI (ImageJ, version 1.50i). Photostimulation under 1PE during recordings was done by illumination of the entire focused field using the Polychrome V connected to a personal computer, and shutter and wavelengths were controlled using Patchmaster software (HEKA). Light intervals lasted a total of 5 min for all the HEK tsA201 cell experiments, except for the illumination of compound 36 with 455 nm where it lasted 3 min. Examples 27e and 29a isomerizes with flashes of 455 nm (3.5 s duration) and 365 nm (3.5 s duration) light. Furthermore, for the 29b flashes of 400 nm (3.5 s duration) and 365 nm (3.5 s duration) light were used. Additionally, Example 34 isomerizes with flashes of 530 nm (3.5 s duration) and 400 nm (3.5 s duration) light. Furthermore, for the compound of Example 36 flashes of 590 nm (3.5 s duration) and 455 nm (3.5 s duration) light were used. An extra LED of continuous illumination with 460 nm light was used to guarantee an efficient photoswitching. The preilluminated cis-Example 36 was applied to validate our results. The preirradiation was made with a LED from OSRAM Oslon (SSL 80-green, part number LT-2014, 590 nm). LED modules were combined to a rated power of 4W and used with a forward current of IF = 350 mA.

2PE experiments with compounds 29a and 29b were performed in the Advanced Digital Microscopy Core Facility of the Institute for Research in Biomedicine (IRB, Barcelona, Spain) with a confocal multiphoton microscope equipped with a pulsed broadband Ti:Sapphire laser (Mai Tai, Spectra- Physics) which can be tuned between 710-990 nm (80 MHz repetition rate, 80 fs pulse, light power 2.8 mW·µm⁻² at 720-840 nm). Images under 2PE were acquired at room temperature with an inverted laser-scanning confocal microscope (TCS SP5, Leica Microsystems) equipped with a HCX PL APO 40×/1.25-0.75-NA oil objective for imaging cultured cells, and a HC PL APO 20×/.0.7-NA CS air objective (Leica Microsystems). The imaging interval was 4 s. Acquired image sequences were stored in the Leica image format and stacks for offline analysis with Fiji (NIH, imaged).

### Primary Hippocampal Neurons Culture

Procedures were performed in accordance with the European guidelines for animal care and use in research and were approved by the Animal Experimentation Ethics Committee at the University of Barcelona. Sprague-Dawley rat pups (P 1-5) were sacrificed by decapitation, hippocampi were isolated and treated with 0,1% trypsin in HBSS (10 min, 37 °C). Neurons were plated on Poly-D-lysine (PDL)-coated 16mm coverslips (0.5-1 × 10⁵ cells) and incubated at 37 °C, 5% CO₂ for 1.5 h (to allow cells adhesion) in MEM supplemented with heat-inactivated FBS (5%), heat-inactivated HS (5%), pen/strep (10 Ul/ml), L-glutamine (2mM) and glucose (20 mM). Neurons were cultured in Neurobasal A medium, supplemented with B-27 (5%), pen/strep (5 Ul/ml), glutaMAX (0,5x) and glucose (15 mM). On DIV 3 cultured cells were treated with 1 µM of Ara-C to prevent proliferation of non-neuronal cells. 50% of the maintenance medium was exchanged every 4 days.

### Calcium Imaging of Culture Hippocampal Neurons

Calcium imaging experiment were done using inverted IX71 Olympus microscope (Japan) with XLUMPLFLN 20X Olympus water immersion objective (Japan). Polychrome V (Till Photonics, USA) with Xenon Short-Arc lamp (Ushio, Japan) was used as a light source. Excitation light was directed to the specimen by 505 nm dichroic beam splitter, emitted light was filtered by D535/40 nm emission filter (Chroma Technology, USA) and collected by C9100-13 EM-CCD Hamamatsu camera (Japan). Before each experiment neurons were incubated in PBS solution containing 10 µM of Oregon Green BAPTA-1 AM (OGB-1 AM) chemical calcium indicator (Thermofisher, USA) for 30 min at 37 °C and 5% CO₂. During imaging experiment neurons were maintained in the solution containing: 150 mM NaCl, 3 mM KCl, 1 mM MgCl₂, 10 mM HEPES and 10 mM glucose with pH 7.40-7.42. OGB-1 was excited with 488 nm light during 100 ms every 2 s. Switching of the molecules with 365/400/455 nm light was done through the optic path of the microscope using Polychrome (Till Photonics, USA) during 1.5 s every 2 s.

### Compound Preparation and Application

All the calcium imaging experiments with HEK tsA201 and hippocampal neurons were performed under dark conditions to keep the compounds in their *trans*-configuration.

Addition of agonists and vehicle was carried out by carefully pipetting directly into the recording chamber for the final dilution of approximately 1:1000. Stock solutions of the agonists were stored at -20 °C in dimethyl sulfoxide (DMSO, Sigma-Aldrich) and diluted with the assay buffer to a 0.1% final concentration of DMSO. Acetylcholine (ACh, Sigma-Aldrich) was used as a positive control to stimulate M1 mAChRs in HEK tsA201 cells. In calcium imaging with tsA201 cells, under 1-photon excitation (1PE), compounds 29a and 29b were tested at concentrations of 10 nM, 100 nM, and 1000 nM. Under 2-photon excitation (2PE), compounds 29a and 29b were tested at 300 nM and 200 nM, respectively. Data were normalized over the maximum response obtained with ACh at 500 nM. In calcium imaging with hippocampal neurons compounds 29a and 29b were tested at 100 and 50 µM, respectively.

### Data Analysis

Imaging data were extracted with FIJI. All data were analyzed by using Excel (Microsoft, version 16.62) and GraphPad Prism (GraphPad Software, version 9). Statistical differences were analyzed by paired-sample Wilcoxon signed-rank test, whereas a value of p ≤ 0.05 was considered as significant. The data are expressed as the mean ± SEM.

### In Vivo Experiments

Animal work was conducted according to the Spanish law (Real Decreto 53/2013) in compliance with the EU Directive 2010/63/EU for animal experiments, and with approval of the ethics committees: Comite Etic d'Experimentacio Animal de la Universitat de Barcelona (CEEA) (10702) and the local competent organs of the Generalitat de Catalunya. The study followed the ARRIVE guidelines. Four mice (Male and female of approximately 3 months of age, C57BI6J strain) were used for 2P *in vivo* imaging experiments.

The animals were anesthetized with isofluorane, and were positioned in a stereotaxic apparatus for the insertion of a cranial window (de la Rosa, X. et al. European Journal of Nuclear Medicine and Molecular Imaging 40, 426-438, doi:10.1007/s00259-012-2277-7 (2013)).

Briefly, using a surgical microdrill, a skull puncture, approximately 4.5 mm in diameter, was made at the coordinates AP: -1 mm; L: 3 mm of the right hemisphere, and covered with a glass coverslip modified to add an injection port sealed with medical grade silicone (Sylgard-184, Sigma) (Roome, C. J. & Kuhn, B. Front Cell Neurosci 8, 379, doi:10.3389/fncel.2014.00379 (2014)). The window was held in place with cyanoacrylate and dental cement. In addition, a small metal bracket was attached for fixation to a stand adapted for the microscope. After 24 h, the 2P *in vivo* imaging experiment was performed. Stock solution of OGB-1 AM was prepared by dissolving it in PBS with 20% pluronic acid till the concentration of 10 mM. For tissue loading stock solution was diluted 20-fold into physiological saline containing 150 mM NaCl, 3 mM KCI, 1 mM MgCl₂, 2 mM CaCl₂, 10 mM HEPES, 10 mM D-glucose; pH 7.40-7.42. The loading solution was made immediately prior to the injection.

The animal was anesthetized by i.p. injection of ketamine/xylazine (100mg/kg and (10 mg/kg respectively), and an injection line was prepared to deliver additional doses as needed (approximately every 30 min). The mouse was positioned in the stereotaxic device. Glass capillaries with the tip size of 2-3 µM were used for injection of the OGB-1 AM solution into the cortex. The glass tip was submerged to a depth of around 100-200 µm from the surface of the brain. OGB-1 AM was pressure-injected from the pipette with the pressure of 500 hPa for 15-20 s.

Imaging was performed after 1 h post injection. The mouse was placed on the microscope stand, fixed by the metal insert to minimize motion artifacts during image acquisition. After acquiring the baseline, the coordinates were recorded for subsequent re-positioning, and the microinjection process was repeated. A micropipette was filled with 3-4 ml of the Example compound 29a at 100 µM in 1 % DMSO and 500 hPa pressure was applied for 15-20 s and 2-3 µl of 29a were injected. The mouse was repositioned at the same coordinates and images were acquired at the same position after 1 h post injection. At the end of the experiment the animal were euthanized.

*In vivo* calcium imaging was performed using an inverted laser-scanning confocal microscope (TCS SP5, Leica Microsystems), coupled with a mode-locked Ti:Sapphire laser (720-990 nm) and resonant scanner. Excitation light was focused onto tissue using 25X, NA 0.95 water immersion objective working distance 2.5 mm with a water dispenser (manufactured in CCiTUB workshop). All the data were collected on the Leica system. Excitation wavelength was 800 nm at 25% of the maximum laser power.

Photoisomerization of the compound 29a was achieved by illuminating the field with 730 nm (maximum laser power) at 1 × 64. line scan for 3 min and with 800 nm (50% of the maximum laser power) 1 × 64. line scan for 3 min.

Calcium transients were imaged using 512 × 512 pixels, 1 × 64. line scan and 8000 Hz frame rate. The imaging interval was of 0.5 s. Acquired image sequences were stored in the Leica image format and stacks for offline analysis with Fiji (NIH, imaged).

### Zebrafish Experiments

Wild-type zebrafish embryos (Tüpfel long-fin strain) were purchased from the animal facility of the Barcelona Biomedical Research Park (PRBB) and raised in darkness for 6 days at 28.5 °C in UV filtered tap water in petri dishes (daily cleaned and refilled). Animal development [Matera, C. et al. Int. J. Mol. Sci., 2022, 23, 10114] was checked every 24 h. Unhealthy or abnormal embryos and larvae were removed and euthanatized in tricaine methanosulfonate 0.02%. All experiments and procedures were conducted according to the European Directive 2010/63/EU. For the experiments blinded zebrafish were used. The animals underwent a noninvasive and highly specific blinding technique that induces photoreceptor apoptosis in the dorsal and central retina. [Matera, C. et al. Int. J. Mol. Sci., 2022, 23, 10114; Taylor, S. et al. Methods Mol. Biol. 2012, 884, 247-54]. The light-lesioning procedure was performed in a thermostatic and well-ventilated room equipped with small fans for air circulation and heat dissipation. Larvae at 7 days post-fertilization (dpf) were placed in a 25-mm petri dish containing 20 ml of UV filtered tap water, inserted in a closed mirrored chamber, and then exposed to 135.000 lux light emitted by a mercury lamp (model Olympus U-LH100HG) for 30 min. Temperature was monitored throughout the whole procedure. The experiment was carried at 12 h after the induction of blindness. Each fish was randomly placed between vehicle (n = 6) and treatment group (n = 6) in a separate well of a 96-well plate, each containing 100 µl of fresh sterilized water. The animals were left undisturbed in the dark for 20 min to get acquainted with the new setting (habituation time). The compound (i.e., 30 µM of Example 36 in 0.3% of DMSO) and vehicle (0.3% of DMSO) were prepared in fresh water. The application was made in darkness and with a multichannel pipette to exclude differences due to delays in the application of each solution. At this point, the plate was inserted into the ZebraBox and movements were recorded and analysed using the ZebraBox tracking system and the ZebraLab software (ViewPoint Life Science). After 20 min of dark adaptation, the animals were exposed to controlled cycles: 590 nm (ProLight 3W Power Led) at 2 mW for 5 min, 455 nm (ProLight 3W Power Led) at 7 mW for 5 min. The sources were placed at 8 cm of distance from the 96-well plate. The light intensity was measured with Optical Power Monitor, Power Meter Interface PM101, and the sensor S470C, Thermal Power Sensor Head (Thorlabs). Alterations of locomotor activity were determined by monitoring and measuring fast movements, swimming distances and duration of high-speed swimming. Data were analyzed by two-way ANOVA (GraphPad Prism 9).

### 1. Synthesis of example and comparative example compounds

### General synthetic methods

### General methods A-G for the synthesis of compounds of formula (I) wherein R¹ and R² are independently H or F, R³ and R⁴ are H, n is 2-7 and R⁵ is O

### • General Method A: Preparation of nitrophenyl alkyne alcohols

All steps were carried out under an argon atmosphere and only degassed liquids were used. 1-bromo-4-nitrobenzene (1.0 equiv.), PdCl₂(PPh₃)2 (0.25 equiv.), copper(I)iodide (0.015 equiv.) and triphenylphosphine (0.25 equiv.) were dissolved in a solution of 60 vol.-% triethylamine in tetrahydrofuran. The respective alkyne (1.25-3.0 equiv.) was added and the solution was stirred at 70 ∘C overnight. The reaction mixture was then quenched with water, diluted with ethyl acetate, and extracted with 1N HCl as well as with sat. aq. NaHCO₃. The organic phase was dried over Na₂SO₄. The solvent was removed under reduced pressure and the oily residue was purified by silica column chromatography, yielding the desired nitrophenyl alcohol (64-98%).

### • General Method B: Preparation of aminophenyl alkane alcohols

The respective nitrophenyl alcohol (1.0 equiv.) was dissolved in methanol. The mixture was flushed with argon and palladium on activated charcoal (20-30 wt%) was added. The mixture was then flushed with argon and stirred for 2-5 h at ambient temperature under a hydrogen atmosphere (10 atm.). The reaction was subsequently filtrated, and the filtrate concentrated. The desired aminophenyl alcohol was obtained as a brown oil and used without further purification (80%-quant.).

### • General Method C: Preparation of pyridinyl-thiadiazol-anilines

NaH (3.0 equiv.) was added to a solution of the respective alcohol (1.0-2.5 equiv.) in anhydrous THF and stirred at ambient temperature until gas development stopped. Subsequently, 3-chloro-4-(pyridin-3-yl)-1,2,5-thiadiazole (1.0-1.05 equiv.) dissolved in THF was added and the reaction was stirred overnight at 70 ∘C. The mixture was afterwards quenched with an excess of water and extracted with EtOAc. The combined organic layers were dried over Na₂SO₄ and concentrated under reduced pressure. Purification by silica column chromatography yielded the desired pyridinyl-thiadiazol-aniline (49-71%).

### • General Method D: Preparation of phenyldiazenyl-pyridinyl-thiadiazoles

The respective pyridinyl-thiadiazol-aniline (1.0 equiv.) and the respective nitrosobenzene (1.0-2.85 equiv.) were stirred in acetic acid overnight at ambient temperature. The reaction mixture was concentrated under reduced pressure, diluted in EtOAc and washed with sat. aq. NaHCO₃ and brine. The aqueous layers were extracted with ethyl acetate until no orange color could be detected in the aqueous. The combined organic layers were then dried over Na₂SO₄ and concentrated under reduced pressure. The crude was purified by silica column chromatography yielding the desired azobenzene (56-93%).

### • General Method E: Preparation of nitrosobenzenes

The respective aniline (1.0 equiv.) was dissolved in CH₂Cl₂. A solution of Oxone^{®} (2.0 equiv.) in water was added and the heterogeneous reaction mixture was stirred for 2 hour. Subsequently layers were separated, and the organic layer was washed with water and brine. The organic layer was dried over Na₂SO₄ and concentrated under reduced pressure. The crude product was filtrated over silica and used without further purification and characterization.

### • General Method F: Preparation of pyridinium

The respective phenyldiazenyl-pyridinyl-thiadiazole (1.0 equiv.) was dissolved in acetone. After the addition of iodomethane (5.0-10 equiv) the reaction mixture was stirred for 2 days at ambient temperature. The desired pyridinium iodide precipitated as an orange solid (77-99%), which was filtered off and washed with acetone. No further purification was performed.

### • General Method G: Preparation of 1,,2,5,6-tetrahydropyridinyl-phenyldiazenyl-1,2,5-thiadiazole

A solution of the respective pyridinium iodide (1.0 equiv.) in a 1:1 mixture of dichloromethane/methanol was cooled down to 0 ∘C, sodium tetrahydroborate (2.8-3.0 equiv.) was added portion wise and the reaction mixture was allowed to warm up and stirred overnight at ambient temperature. Afterwards, the remaining sodium tetrahydroborate was quenched with an excess of water. The mixture was then extracted with dichloromethane. The combined organic layers were subsequently dried over Na₂SO₄, concentrated under reduced pressure and purified by silica gel column chromatography and reverse phase (C18) flash chromatography, yielding the target compound of formula (I) (30-78%).

### General methods H-L for the synthesis of compounds of formula (I) wherein R¹-R⁴ are all F or all Cl, n is 4-5 and R⁵ is O:

### • General Method H: Sonogashira Cross-Coupling

All steps were carried out under an argon atmosphere and every liquid was degassed before use. The respective aryl bromide (1.0 equiv.), PdCl₂(PPh₃)₂ (0.25 equiv.), copper(I)iodide (0.015 equiv.) and triphenylphosphine (0.25 equiv.) were dissolved in a solution of 60 vol.-% triethylamine in tetrahydrofuran. The respective alkyne (1.25 - 3.0 equiv.) was added and the solution was stirred at 70°C overnight. The reaction mixture was then diluted with water and ethyl acetate and washed with 1 M HCl. Subsequently, the organic layer was washed with sat. aq. NaHCO₃ and brine. The organic phase was dried over Na₂SO₄. And the solvent was removed under reduced pressure. Purification of the oily residue by silica column chromatography yielded the desired products.

### • General Method I: Nucleophilic Substitution at 3-chloro-4-(pyridin-3-yl)-1,2,5-thiadiazole

A solution of the respective alcohol (1.0 - 2.5 equiv.) in anhydrous THF was prepared. NaH (60% dispersion in mineral oil, 3.0 - 9.0 equiv.) was added and the mixture was stirred at ambient temperature until gas development stopped. Subsequently, 3-chloro-4-(pyridin-3-yl)-1,2,5-thiadiazole (1.0 - 1.05 equiv.) dissolved in anhydrous THF was added and the reaction was stirred overnight at 70°C. The mixture was afterwards quenched with an excess of water and extracted with EtOAc. The combined organic layers were dried over Na₂SO₄ and concentrated under reduced pressure. Purification by silica column chromatography yielded the desired products.

### • General Method J: Baeyer-Mills-Reaction

The respective aniline (1.0 equiv.) and the respective nitrosobenzene (1.0 - 2.85 equiv.) were stirred in acetic acid or a mixture of acetic acid/toluene/TFA (6:6:1) overnight at ambient temperature. The reaction mixture was concentrated under reduced pressure, diluted in EtOAc and washed with sat. aq. NaHCO₃ and brine. The aqueous layers were extracted with ethyl acetate until no orange colour could be detected in the aqueous. The combined organic layers were then dried over Na₂SO₄ and concentrated under reduced pressure. The crude was purified by silica column chromatography yielding the desired azobenzene.

### • General Method K: Preparation of pyridinium iodides

The respective pyridinyl-thiadiazole (1.0 equiv.) was dissolved in acetone. After the addition of iodomethane (5.0 - 10 equiv.) the reaction mixture was stirred for 1 to 2 days at ambient temperature. Parts of the desired pyridinium iodide precipitated as an orange or yellow solid, which was filtered off and washed with acetone (no further purification was performed). The filtrate was concentrated, the residue purified by silica column chromatography and combined with the precipitate yielding the desired pyridinium iodides.

### • General Method L: Reduction of pyridinium iodides to tetrahydropyridines

A solution of the respective pyridinium iodide (1.0 equiv.) in a 1:1 mixture of dichloromethane/methanol was cooled down to 0°C, sodium tetrahydroborate (2.8 - 3.0 equiv.) was added portionwise and the reaction mixture was allowed to warm up and stirred overnight at ambient temperature. Afterwards, the remaining sodium tetrahydroborate was quenched with an excess of water. The mixture was then extracted with dichloromethane. The combined organic layers were subsequently dried over Na₂SO₄, concentrated under reduced pressure and purified by silica gel column chromatography and reverse phase (C18) flash chromatography, yielding the xanomeline derivative.

### 1.1. Preparation of compounds of formula (I), wherein R¹ and R² are H and/or F, R⁵ is O and n is 2-7.

### • 1.1.1. Preparation of Nitrophenyl Alkyne Alcohols:

### 3-(4-Nitrophenyl)prop-2-yn-1-ol

According to general method A, 1-bromo-4-nitrobenzene (1000 mg, 4.95 mmol, 1.0 equiv.) was coupled with prop-2-yn-1-ol (867 µL, 835 mg, 14.9 mmol, 3.0 equiv.). Purification by silica column chromatography (petroleum ether/ethyl acetate 5:2) afforded 3-(4-nitrophenyl)prop-2-yn-1-ol (800 mg, 4.52 mmol, 91%) as a slightly orange solid. ¹HNMR (400 MHz, CDCl₃): δ 8.27-8.10 (m, 2H), 7.61 - 7.49 (m, 2H), 4.63 - 4.46 (m, 2H), 2.06 - 1.92 (m, 1H). ¹³C-NMR (101 MHz, CDCl₃): δ 147.35, 132.52, 129.59, 123.70, 92.72, 83.88, 51.58.

### 4-(4-Nitrophenyl)but-3-yn-1-ol

According to general method A, 1-bromo-4-nitrobenzene (2000 mg, 9.90 mmol, 1.0 equiv.) was coupled with but-3-yn-1-ol (2.24 mL, 2082 mg, 29.70 mmol, 3.0 equiv.). Purification by silica column chromatography (petroleum ether/ethyl acetate 5:2) afforded 4-(4-nitrophenyl)but-3-yn-1-ol (1811mg, 9.47 mmol, 96%) as an orange solid. ¹H-NMR (400 MHz, CDCl₃): δ 8.19 - 8.13 (m, 2H), 7.57 - 7.51 (m, 2H), 3.86 (t, J = 6.3 Hz, 2H), 2.74 (t, J = 6.3 Hz, 2H), 1.74 (s, 1H). ¹³C-NMR (101 MHz, CDCl₃): δ 147.05,132.57, 130.56, 123.68, 92.73, 80.95, 61.04, 24.05.

### 5-(4-Nitrophenyl)pent-4-yn-1-ol

According to general method A, 1-bromo-4-nitrobenzene (2000 mg, 9.90 mmol, 1.0 equiv.) was coupled with pent-4-yn-1-ol (1.15 mL, 1041 mg, 12.40 mmol, 1.25 equiv.). Purification by silica column chromatography (petroleum ether/ethyl acetate 5:2) afforded 5-(4-nitrophenyl)pent-4-yn-1-ol (1.98 g, 9.66 mmol, 98%) as an slightly orange solid. ¹HNMR (400 MHz, CDCl₃): δ 8.22 - 8.01 (m, 2H), 7.58 - 7.41 (m, 2H), 3.79 (dd, J = 6.7, 5.6 Hz, 2H), 2.57 (t, J = 7.0 Hz, 2H), 1.92 - 1.80 (m, 3H). ¹³C-NMR (101 MHz, CDCl₃): δ 146.72, 132.34, 130.97, 123.56, 95.80, 79.71, 61.54, 31.20, 16.17.

### 6-(4-Nitrophenyl)hex-5-yn-1-ol

According to general method A, 1-bromo-4-nitrobenzene (1029 mg, 5.09 mmol, 1.0 equiv.) was coupled with hex-5-yn-1-ol (1.12 mL, 1000mg, 10.19 mmol, 2.0 equiv.).

Purification by silica column chromatography (petroleum ether/ethyl acetate 5:2) afforded 6-(4-nitrophenyl)hex-5-yn-1-ol (711 mg, 3.24 mmol, 64%) as a brown solid. ¹H-NMR (400 MHz, CDCl₃): δ 8.21 - 8.02 (m, 2H), 7.56 - 7.40 (m, 2H), 3.77 - 3.64 (m, 2H), 2.53 - 2.43 (m, 2H), 1.78 - 1.67 (m, J = 2.9 Hz, 4H), 1.60 (s, 1H). ¹³C-NMR (101 MHz, CDCl₃): δ 146.74, 132.36, 131.12, 123.59, 96.28, 79.72, 62.42, 31.94, 24.87, 19.48.

### 7-(4-Nitrophenyl)hept-6-yn-1-ol

According to general method A, 1-bromo-4-nitrobenzene (900 mg, 4.46 mmol, 1.0 equiv.) was coupled with hept-6-yn-1-ol (1.18 mL, 1000 mg, 8.92 mmol, 2.0 equiv.). Purification by silica column chromatography (petroleum ether/ethyl acetate 5:2) afforded 7-(4-nitrophenyl)hept-6-yn-1-ol (1017mg, 4.36 mmol, 97%) as a brown solid. ¹H-NMR (400 MHz, CDCl₃): δ 8.17 - 8.09 (m, 2H), 7.52 - 7.44 (m, 2H), 3.66 (t, J = 6.3 Hz, 2H), 2.45 (t, J = 7.0 Hz, 2H), 1.69 - 1.58 (m, 5H), 1.51 (s, 2H). ¹³C-NMR (101 MHz, CDCl₃): δ 146.62, 132.29, 131.16, 123.51, 96.50, 79.50, 62.69, 32.23, 28.25, 25.21, 19.59.

### • 1.1.2. Preparation of Aminophenyl Alkane Alcohols:

### 3-(4-Aminophenyl)propan-1-ol

According to general method B, 3-(4-nitrophenyl)prop-2-yn-1-ol (500 mg, 2.82 mmol, 1.0 equiv.) was hydrogenated to give 3-(4-aminophenyl)propan-1-ol (342mg, 2.26 mmol, 80%) as a slightly beige solid. ¹H-NMR (400 MHz, MeOD₄): δ 7.21 - 7.14 (m, 2H), 7.12 - 7.02 (m, 2H), 3.52 (t, J = 6.5 Hz, 2H), 2.62 (t, 2H), 1.83 - 1.68 (m, 2H). ¹³CNMR (101 MHz, MeOD4): δ 140.69, 135.27, 130.66, 121.55, 62.04, 35.32, 32.34. MS (ESI, positive): *m*/*z* calcd. for [C₉H₁₄NO]⁺: 152.11, found: 152.15 [M+H]⁺.

### 4-(4-Aminophenyl)butan-1-ol

According to general method B, 4-(4-nitrophenyl)but-3-yn-1-ol (851mg, 4.45 mmol, 1.0 equiv.) was hydrogenated to give 4-(4-aminophenyl)butan-1-ol (651mg, 3.94 mmol, 89%) as a beige solid. ¹H-NMR (400 MHz, CDCl₃): *δ* 7.01 - 6.92 (m, 2H), 6.66 - 6.57 (m, 2H), 3.63 (t, J = 6.3 Hz, 2H), 2.53 (t, J = 7.3 Hz, 2H), 1.68 - 1.49 (m, 4H). ¹³C-NMR (101 MHz, CDCl₃): *δ* 144.24, 132.60, 129.26, 115.39, 62.95, 34.84, 32.41, 27.90. MS (ESI, positive): *m*/*z* calcd. for [C₁₀H₁₆NO]⁺: 166.12, found: 166.15 [M+H]⁺.

### 5-(4-Aminophenyl)pentan-1-ol

According to general method B, 5-(4-nitrophenyl)pent-4-yn-1-ol (1.90 g, 9.26 mmol, 1.0 equiv.) was hydrogenated to give 5-(4-aminophenyl)pentan-1-ol (1.58 g, 8.80 mmol, 95%) as a brown solid. ¹H-NMR (400 MHz, CDCl₃): *δ* 7.02 - 6.92 (m, 2H), 6.67 - 6.59 (m, 2H), 3.63 (t, J = 6.6 Hz, 2H), 2.51 (t, J = 7.6 Hz, 2H), 1.69 - 1.48 (m, 6H). ¹³C-NMR (101 MHz, CDCl₃): *δ* 143.87, 133.44, 129.30, 115.46, 63.15, 35.15, 32.84, 31.68, 25.47. MS (ESI, positive): *m*/*z* calcd. for [C₁₁H₁₈NO]⁺: 180.14, found: 180.10 [M+H]⁺.

### 6-(4-Aminophenyl)hexan-1-ol

According to general method B, 6-(4-nitrophenyl)hex-5-yn-1-ol (600 mg, 2.74 mmol, 1.0 equiv.) was hydrogenated to give 6-(4-aminophenyl)hexan-1-ol (529mg, 2.74 mmol, quant.) as a brown solid. ¹H-NMR (400 MHz, CDCl₃): *δ* 7.00 - 6.90 (m, 2H), 6.74 - 6.58 (m, 2H), 3.61 (t, J = 6.6 Hz, 2H), 2.52 - 2.47 (m, 2H), 1.62 - 1.50 (m, 4H), 1.40 - 1.30 (m, 2H). ¹³C-NMR (101 MHz, CDCl₃): *δ* 143.97, 133.11, 129.23, 115.43, 63.06, 35.08, 32.83, 31.80, 29.10, 25.73. MS (ESI, positive): *m*/*z* calcd. for [C₁₂H₂₀NO]⁺: 194.15, found: 194.15 [M+H]⁺.

### 7-(4-Aminophenyl)heptan-1-ol

According to general method B, 7-(4-nitrophenyl)hept-6-yn-1-ol (400mg, 1.71 mmol, 1.0 equiv.) was hydrogenated to give 7-(4-aminophenyl)heptan-1-ol (332mg, 1.60 mmol, 94%.) as a brown solid. ¹H-NMR (400 MHz, CDCl₃): *δ* 7.02 - 6.90 (m, 2H), 6.67 - 6.58 (m, 2H), 3.62 (t, J = 6.6 Hz, 2H), 2.52 - 2.44 (m, 2H), 1.60 - 1.51 (m, 4H), 1.37 - 1.29 (m, 6H). ¹³C-NMR (101 MHz, CDCl₃): *δ* 143.83, 133.31, 129.25, 115.50, 63.14, 35.16, 32.89, 31.81, 29.41, 29.30, 25.80. MS (ESI, positive): *m*/*z* calcd. For [C₁₃H₂₂NO]⁺: 208.17, found: 208.20 [M+H]⁺.

### • 1.1.3. Preparation of Pyridinyl-thiadiazol-anilines:

### 4-(2-((4-(Pyridin-3-yl)-1,2,5-thiadiazol-3-yl)oxy)ethyl)aniline

According to general method C, 3-chloro-4-(pyridin-3-yl)-1,2,5-thiadiazole (300mg, 1.52 mmol, 1.0 equiv.) and 2-(4-aminophenyl)ethan-1-ol (520 mg, 3.79 mmol, 2.5 equiv.) were reacted. Purification by silica column chromatography (petroleum ether/ethyl acetate 1:2) afforded 4-(2-((4-(pyridin-3-yl)-1,2,5-thiadiazol-3-yl)oxy)ethyl)aniline (321 mg, 1.08 mmol, 71%) as a slightly yellow solid. ¹H-NMR (400 MHz, CDCl₃): *δ* 9.34 - 9.27 (m, 1H), 8.61 - 8.55 (m, 1H), 8.30 - 8.23 (m, 1H), 7.31 - 7.26 (m, 1H), 7.06 - 6.98 (m, 2H), 6.65 - 6.56 (m, 2H), 4.61 (t, J = 6.9 Hz, 2H), 3.68 (s, 2H), 3.01 (t, J = 6.9 Hz, 2H). ¹³C-NMR (101 MHz, CDCl₃): *δ* 162.45, 149.91, 148.54, 145.20, 144.85, 134.59, 129.63, 127.38, 126.92, 123.17, 115.14, 71.97, 34.41. MS (ESI, positive): *m*/*z* calcd. for [C₁₅H₁₅N₄OS]⁺: 299.10, found: 299.05 [M+H]⁺.

### 4-(3-((4-(Pyridin-3-yl)-1,2,5-thiadiazol-3-yl)oxy)propyl)aniline

According to general method C, 3-chloro-4-(pyridin-3-yl)-1,2,5-thiadiazole (300mg, 1.52 mmol, 1.0 equiv.) and 3-(4-aminophenyl)propan-1-ol (574 mg, 3.79 mmol, 2.5 equiv.) were reacted. Purification by silica column chromatography (petroleum ether/ethyl acetate 1:1) afforded 4-(3-((4-(pyridin-3-yl)-1,2,5-thiadiazol-3-yl)oxy)propyl)aniline (272 mg, 0.87 mmol, 57%) as a slightly yellow solid. ¹H-NMR (400 MHz, CDCl₃): *δ* 9.42 - 9.39 (m, 1H), 8.68 - 8.64 (m, 1H), 8.44 - 8.39 (m, 1H), 7.42 - 7.37 (m, 1H), 7.02 - 6.96 (m, 2H), 6.65 - 6.59 (m, 2H), 4.53 (t, J = 6.5 Hz, 2H), 3.28 (s, 2H), 2.72 (t, J = 7.5 Hz, 2H), 2.17 (p, J = 6.7 Hz, 2H). ¹³C-NMR (101 MHz, CDCl₃): *δ* 162.91, 150.27, 148.79, 145.11, 144.67, 134.85, 130.95, 129.34, 127.82, 123.56, 115.49, 70.78, 31.47, 30.83. MS (ESI, positive): *m*/*z* calcd. for [C₁₅H₁₇N₄OS]⁺: 313.11, found: 313.15 [M+H]⁺.

### 4-(4-((4-(Pyridin-3-yl)-1,2,5-thiadiazol-3-yl)oxy)butyl)aniline

According to general method C, 3-chloro-4-(pyridin-3-yl)-1,2,5-thiadiazole (818mg, 4.14 mmol, 1.05 equiv.) and 4-(4-aminophenyl)butan-1-ol (651mg, 3.94 mmol, 1.0 equiv.) were reacted. Purification by silica column chromatography (petroleum ether/ethyl acetate 1:1 + 0.1% NEt₃) afforded 4-(4-((4-(pyridin-3-yl)-1,2,5-thiadiazol-3-yl)oxy)butyl)-aniline (897 mg, 2.78 mmol, 70%) as a yellow solid. ¹H-NMR (400 MHz, CDCl₃): *δ* 9.43 - 9.34 (m, 1H), 8.70 - 8.61 (m, 1H), 8.49 - 8.38 (m, 1H), 7.44 - 7.37 (m, 1H), 7.02 - 6.95 (m, 2H), 6.71 - 6.63 (m, 2H), 4.53 (t, J = 6.5 Hz, 2H), 3.59 (s, 2H), 2.61 (t, J = 7.4 Hz, 2H), 1.99 - 1.71 (m, 4H). ¹³C-NMR (101 MHz, CDCl₃): *δ* 162.95, 150.02, 148.55, 144.98, 143.58, 135.06, 132.60, 129.35, 127.91, 123.65, 115.86, 71.42, 34.65, 28.49, 28.04. MS (ESI, positive): *m*/*z* calcd. for [C₁₇H₁₉N₄OS]⁺: 327.13, found: 327.15 [M+H]⁺.

### 4-(5-((4-(Pyridin-3-yl)-1,2,5-thiadiazol-3-yl)oxy)pentyl)aniline

According to general method C, 3-chloro-4-(pyridin-3-yl)-1,2,5-thiadiazole (176mg, 0.29 mmol, 1.0 equiv.) and 5-(4-aminophenyl)pentan-1-ol (192 mg, 1.07 mmol, 1.2 equiv.) were reacted. Purification by silica column chromatography (petroleum ether/ethyl acetate 5:3 + 1% NEt₃ to 1:1+ 1% NEt₃) afforded 4-(5-((4-(pyridin-3-yl)-1,2,5-thiadiazol-3- yl)oxy)pentyl)aniline (191 mg, 0.56 mmol, 63%) as a yellow solid. ¹H-NMR (400 MHz, CDCl₃): *δ* 9.39 - 9.38 (m, 1H), 8.71 - 8.59 (m, 1H), 8.46 - 8.31 (m, 1H), 7.44 - 7.33 (m, 1H), 7.04 - 6.87 (m, 2H), 6.65 - 6.57 (m, 2H), 4.51 (t, J = 6.6 Hz, 2H), 3.40 (s, 2H), 2.54 (t, J = 7.5 Hz, 2H), 1.95 - 1.86 (m, 2H), 1.71 - 1.62 (m, 2H), 1.55 - 1.47 (m, 2H). ¹³C-NMR (101 MHz, CDCl₃): *δ* 162.95, 150.19, 148.74, 145.08, 144.18, 134.85, 132.55, 129.25, 127.81, 123.55, 115.42, 71.45, 34.96, 31.40, 28.91, 25.63. MS (ESI, positive): *m*/*z* calcd. for [C₁₈H₂₁N₄OS]⁺: 341.14, found: 341.10 [M+H]⁺.

### 4-(6-((4-(Pyridin-3-yl)-1,2,5-thiadiazol-3-yl)oxy)hexyl)aniline

According to general method C, 3-chloro-4-(pyridin-3-yl)-1,2,5-thiadiazole (429mg, 2.17 mmol, 1.05 equiv.) and 6-(4-aminophenyl)hexan-1-ol (400 mg, 2.07 mmol, 1.0 equiv.) were reacted. Purification by silica column chromatography (petroleum ether/ethyl acetate 1:1) afforded 4-(6-((4-(pyridin-3-yl)-1,2,5-thiadiazol-3-yl)oxy)hexyl)aniline (455 mg, 1.28 mmol, 66%) as a slightly yellow solid. ¹H-NMR (400 MHz, CDCl₃): *δ* 9.43 - 9.37 (m, 1H), 8.68 - 8.62 (m, 1H), 8.44 - 8.38 (m, 1H), 7.41 - 7.35 (m, 1H), 6.98 - 6.92 (m, 2H), 6.63 - 6.58 (m, 2H), 4.50 (t, J = 6.6 Hz, 2H), 3.29 (s, 2H), 2.50 (t, J = 7.6 Hz, 2H), 1.88 (p, J = 6.8 Hz, 2H), 1.66 - 1.35 (m, 6H). ¹³C-NMR (101 MHz, CDCl₃): *δ* 162.94, 150.20, 148.73, 145.07, 144.19, 134.78, 132.75, 129.21, 127.81, 123.54, 115.33, 71.50, 35.02, 31.69, 28.95, 28.88, 26.00. MS (ESI, positive): *m*/*z* calcd. For [C₁₉H₂₃N₄OS]⁺: 355.16, found: 355.15 [M+H]⁺.

### 4-(7-((4-(Pyridin-3-yl)-1,2,5-thiadiazol-3-yl)oxy)heptyl)aniline

According to general method C, 3-chloro-4-(pyridin-3-yl)-1,2,5-thiadiazole (280mg, 1.42 mmol, 1.05 equiv.) and 7-(4-aminophenyl)heptan-1-ol (280 mg, 1.35 mmol, 1.0 equiv.) were reacted. Purification by silica column chromatography (petroleum ether/ethyl acetate 1:1) yielded 4-(7-((4-(pyridin-3-yl)-1,2,5-thiadiazol-3-yl)oxy)heptyl)aniline (245mg, 0.66 mmol, 49%) as a slightly yellow solid. ¹H-NMR (400 MHz, CDCl₃): *δ* 9.46 - 9.34 (m, 1H), 8.74 - 8.60 (m, 1H), 8.49 - 8.35 (m, 1H), 7.41 - 7.33 (m, 1H), 7.00 - 6.91 (m, 2H), 6.66 - 6.57 (m, 2H), 4.51 (t, J = 6.6 Hz, 2H), 3.33 (s, 2H), 2.49 (t, J = 7.7 Hz, 2H), 1.88 (p, J = 6.8 Hz, 2H), 1.59 - 1.33 (m, 8H). ¹³C-NMR (101 MHz, CDCl₃): *δ* 162.97, 150.23, 148.76, 145.09, 144.15, 134.81, 132.97, 129.23, 127.80, 123.53, 115.34, 71.53, 35.12, 31.77, 29.24, 29.16, 28.99, 26.05. MS (ESI, positive): *m*/*z* calcd. For [C₂₀H₂₅N₄OS]⁺: 369.17, found: 369.15 [M+H]⁺.

### • 1.1.4. Preparation of Phenyldiazenyl-pyridinyl-thiadiazoles:

### 3-(4-(Phenyldiazenyl)phenethoxy)-4-(pyridin-3-yl)-1,2,5-thiadiazole

According to general method D, 4-(2-((4-(pyridin-3-yl)-1,2,5-thiadiazol-3-yl)oxy)ethyl)-aniline (300mg, 1.01 mmol, 1.0 equiv.) and nitrosobenzene (215 mg, 2.02 mmol, 2.0 equiv.) were reacted in a Bayer-Mills reaction. Purification by silica column chromatography (petroleum ether/ethyl acetate 5:2) afforded 3-(4-(phenyldiazenyl)phenethoxy)- 4-(pyridin-3-yl)-1,2,5-thiadiazole (355mg, 0.92 mmol, 91%) as an orange solid. ¹HNMR (400 MHz, CDCl₃): *δ* 9.42 - 9.32 (m, 1H), 8.69 - 8.59 (m, 1H), 8.33 - 8.25 (m, 1H), 7.94 - 7.86 (m, 4H), 7.55 - 7.41 (m, 5H), 7.38 - 7.30 (m, 1H), 4.80 (t, J = 6.7 Hz, 2H), 3.28 (t, J = 6.7 Hz, 2H). ¹³C-NMR (101 MHz, CDCl₃): *δ* 162.53, 152.77, 151.76, 150.34, 148.85, 145.18, 140.94, 134.82, 131.09, 129.81, 129.20, 127.58, 123.46, 123.29, 122.95, 71.44, 35.42. MS (ESI, positive): *m*/*z* calcd. for [C₂₁H₁₈N₅OS]⁺: 388.12, found: 388.25 [M+H]⁺.

### 3-(3-(4-(Phenyldiazenyl)phenyl)propoxy)-4-(pyridin-3-yl)-1,2,5-thiadiazole

According to general method D, 4-(3-((4-(pyridin-3-yl)-1,2,5-thiadiazol-3-yl)oxy)propyl)-aniline (226mg, 0.72 mmol, 1.0 equiv.) and nitrosobenzene (220 mg, 2.06 mmol, 2.85 equiv.) were reacted in a Bayer-Mills reaction. Purification by silica column chromatography (petroleum ether/ethyl acetate 5:2) afforded 3-(3-(4-(phenyldiazenyl)phenyl) propoxy)-4-(pyridin-3-yl)-1,2,5-thiadiazole (111 mg, 0.28 mmol, 38%) as an orange solid. ¹H-NMR (400 MHz, CDCl₃): *δ* 9.46 - 9.36 (m, 1H), 8.71 - 8.60 (m, 1H), 8.45 - 8.34 (m, 1H), 7.93 - 7.81 (m, 4H), 7.54 - 7.41 (m, 3H), 7.41 - 7.30 (m, 3H), 4.55 (t, J = 6.4 Hz, 2H), 2.89 (t, J = 7.6 Hz, 2H), 2.36 - 2.19 (m, 2H). ¹³C-NMR (101 MHz, CDCl₃): *δ* 162.64, 152.73, 151.29, 150.24, 148.63, 144.99, 144.34, 134.75, 130.89, 129.16, 129.12, 127.66, 123.50, 123.16, 122.83, 70.48, 32.26, 30.26. MS (ESI, positive): *m*/*z* calcd. for [C₂₂H₂₀N₅OS]⁺: 402.14, found: 402.15 [M+H]⁺.

### 3-(4-(4-(Phenyldiazenyl)phenyl)butoxy)-4-(pyridin-3-yl)-1,2,5-thiadiazole

According to general method D, 4-(4-((4-(pyridin-3-yl)-1,2,5-thiadiazol-3-yl)oxy)butyl)-aniline (450 mg, 1.38 mmol, 1.0 equiv.) and nitrosobenzene (293 mg, 2.76 mmol, 2.0 equiv.) were reacted in a Bayer-Mills reaction. Purification by silica column chromatography (petroleum ether/ethyl acetate 5:2) afforded 3-(4-(4-(phenyldiazenyl)phenyl)-butoxy)-4-(pyridin-3-yl)-1,2,5-thiadiazole (393 mg, 0.95 mmol, 69%) as an orange solid. ¹H-NMR (400 MHz, CDCl₃): *δ* 9.46 - 9.34 (m, 1H), 8.69 - 8.61 (m, 1H), 8.45 - 8.36 (m, 1H), 7.93 - 7.88 (m, 2H), 7.88 - 7.82 (m, 2H), 7.57 - 7.30 (m, 6H), 4.57 (t, J = 6.2 Hz, 2H), 2.80 (t, J = 7.2 Hz, 2H), 2.01 - 1.85 (m, 4H). ¹³C-NMR (101 MHz, CDCl₃): *δ* 162.89, 152.89, 151.32, 150.36, 148.81, 145.43, 145.43, 134.86, 130.93, 129.25, 129.21, 127.80, 123.59, 123.16, 122.90, 71.20, 35.45, 28.56, 27.67. MS (ESI, positive): *m*/*z* calcd. for [C₂₃H₂₂N₅OS]⁺: 416.15, found: 416.15 [M+H]⁺.

### 3-((5-(4-(Phenyldiazenyl)phenyl)pentyl)oxy)-4-(pyridin-3-yl)-1,2,5-thiadiazole

According to general method D, 4-(5-((4-(pyridin-3-yl)-1,2,5-thiadiazol-3-yl)oxy)pentyl)-aniline (163 mg, 0.48 mmol, 1.0 equiv.) and nitrosobenzene (52mg, 0.48 mmol, 1.0 equiv.) were reacted in a Bayer-Mills reaction. Purification by silica column chromatography (petroleum ether/ethyl acetate 5:1) afforded to give 3-((5-(4-(phenyldiazenyl)phenyl)- pentyl)oxy)-4-(pyridin-3-yl)-1,2,5-thiadiazole (192 mg, 0.45 mmol, 93%) as an orange solid. ¹H-NMR (400 MHz, CDCl₃): *δ* 9.39 (m, 1H), 8.67 - 8.61 (m, 1H), 8.46 - 8.33 (m, 1H), 7.97 - 7.86 (m, 2H), 7.87 - 7.80 (m, 2H), 7.58 - 7.44 (m, 3H), 7.41 - 7.35 (m, 1H), 7.34 - 7.29 (m, 2H), 4.54 (t, J = 6.5 Hz, 2H), 2.74 (t, J = 7.6 Hz, 2H), 1.95 (p, J = 6.8 Hz, 2H), 1.78 (p, J = 7.6 Hz, 2H), 1.63 - 1.51 (m, 2H). ¹³C-NMR (101 MHz, CDCl₃): *δ* 162.92, 152.89, 151.19, 150.10, 148.59, 145.93, 145.03, 134.97, 130.88, 129.22, 129.20, 123.63, 123.08, 122.87, 71.36, 35.80, 32.08, 29.85, 29.51, 25.75 MS (ESI, positive): *m*/*z* calcd. for [C₂₄H₂₄N₅OS]⁺: 430.17, found: 430.10 [M+H]⁺.

### 3-((6-(4-(Phenyldiazenyl)phenyl)hexyl)oxy)-4-(pyridin-3-yl)-1,2,5-thiadiazole

According to general method D, 4-(6-((4-(pyridin-3-yl)-1,2,5-thiadiazol-3-yl)oxy)hexyl)-aniline (300 mg, 0.85 mmol, 1.0 equiv.) and nitrosobenzene (181 mg, 1.69 mmol, 2.0 equiv.) were reacted in a Bayer-Mills reaction. Purification by silica column chromatography (petroleum ether/ethyl acetate 5:2) afforded 3-((6-(4-(phenyldiazenyl)phenyl) hexyl)oxy)-4-(pyridin-3-yl)-1,2,5-thiadiazole (305 mg, 0.69 mmol, 81%) as an orange solid. ¹H-NMR (400 MHz, CDCl₃): *δ* 9.44 - 9.37 (m, 1H), 8.67 - 8.60 (m, 1H), 8.46 - 8.35 (m, 1H), 7.94 - 7.86 (m, 2H), 7.88 - 7.81 (m, 2H), 7.54 - 7.39 (m, 3H), 7.40 - 7.32 (m, 1H), 7.33 - 7.26 (m, 2H), 4.50 (t, J = 6.6 Hz, 2H), 2.68 (t, J = 7.7 Hz, 2H), 1.87 (p, 2H), 1.69 (p, J = 7.4 Hz, 2H), 1.60 - 1.35 (m, 4H). ¹³C-NMR (101 MHz, CDCl₃): *δ* 162.82, 152.76, 151.02, 150.14, 148.61, 146.14, 144.95, 134.74, 130.74, 129.11, 129.07, 127.70, 123.45, 122.95, 122.77, 71.35, 35.78, 31.12, 28.89, 28.87, 25.93. MS (ESI, positive): *m*/*z* calcd. for [C₂₅H₂₆N₅OS]⁺: 444.19, found: 444.15 [M+H]⁺.

### 3-((7-(4-(Phenyldiazenyl)phenyl)heptyl)oxy)-4-(pyridin-3-yl)-1,2,5-thiadiazole

According to general method D, 4-(7-((4-(pyridin-3-yl)-1,2,5-thiadiazol-3-yl)oxy)heptyl)-aniline (300 mg, 0.81 mmol, 1.0 equiv.) and nitrosobenzene (175 mg, 1.63 mmol, 2.0 equiv.) were reacted in a Bayer-Mills reaction. Purification by silica column chromatography (petroleum ether/ethyl acetate 5:1) afforded to give 3-((7-(4-(phenyldiazenyl)-phenyl)heptyl)oxy)-4-(pyridin-3-yl)-1,2,5-thiadiazole (321 mg, 0.70 mmol, 86%) as an orange solid. ¹H-NMR (400 MHz, CDCl₃): *δ* 9.46 - 9.36 (m, 1H), 8.72 - 8.59 (m, 1H), 8.48 - 8.36 (m, 1H), 7.93 - 7.86 (m, 2H), 7.87 - 7.80 (m, 2H), 7.55 - 7.40 (m, 3H), 7.39 - 7.35 (m, 1H), 7.33 - 7.28 (m, 2H), 4.52 (t, J = 6.6 Hz, 2H), 2.69 (t, J = 7.7 Hz, 2H), 1.89 (p, J = 6.8 Hz, 2H), 1.68 (p, J = 7.5 Hz, 2H), 1.54 - 1.35 (m, 6H). ¹³C-NMR (101 MHz, CDCl₃): *δ* 162.93, 152.85, 151.08, 150.15, 148.64, 146.40, 145.02, 134.87, 130.79, 129.18, 129.14, 127.81, 123.55, 122.99, 122.82, 71.48, 35.93, 31.26, 29.21, 29.19, 28.98, 26.04. MS (ESI, positive): *m*/*z* calcd. for [C₂₆H₂₈N₅OS]⁺: 458.20, found: 458.15 [M+H]⁺.

### 3-(4-(4-((2-Fluorophenyl)diazenyl)phenyl)butoxy)-4-(pyridin-3-yl)-1,2,5-thiadiazole

According to general method D, 4-(4-((4-(pyridin-3-yl)-1,2,5-thiadiazol-3-yl)oxy)butyl)-aniline (175 mg, 0.54 mmol, 1.0 equiv.) and 1-fluoro-2-nitrosobenzene (84 mg, 0.67 mmol, 1.25 equiv.) were reacted in a Bayer-Mills reaction. Purification by silica column chromatography (petroleum ether/ethyl acetate 5:2) afforded 3-(4-(4-((2-fluorophenyl)diazenyl) phenyl)butoxy)-4-(pyridin-3-yl)-1,2,5-thiadiazole (205 mg, 0.47 mmol, 88%) as an orange solid. ¹H-NMR (400 MHz, CDCl₃): δ 9.39 (s, 1H), 8.69 - 8.60 (m, 1H), 8.41 - 8.31 (m, 1H), 7.91 - 7.83 (m, 2H), 7.75 - 7.68 (m, 1H), 7.43 - 7.34 (m, 2H), 7.33 - 7.28 (m, 2H), 7.25 - 7.15 (m, 2H), 4.52 (t, J = 6.2 Hz, 2H), 2.76 (t, J = 7.2 Hz, 2H), 1.95- 1.81 (m, 4H). ¹³C-NMR (101 MHz, CDCl₃): *δ* 162.71, 160.03 (d, J = 257.3 Hz), 151.33, 150.13, 148.58, 145.88, 144.92, 140.73 (d, J = 6.8 Hz), 134.71, 132.25 (d, J = 8.2 Hz), 129.14, 124.28 (d, J = 3.9 Hz), 123.46, 123.34, 117.77, 117.01 (d, J = 19.8 Hz), 71.04, 35.34, 28.42, 27.49. MS (ESI, positive): *m*/*z* calcd. for [C₂₃H₂₁FN₅OS]⁺: 434.14, found: 434.10 [M+H]⁺.

### 3-(4-(4-((2,6-Difluorophenyl)diazenyl)phenyl)butoxy)-4-(pyridin-3-yl)-1,2,5-thiadiazole

According to general method D, 4-(4-((4-(pyridin-3-yl)-1,2,5-thiadiazol-3-yl)oxy)butyl)-aniline (175mg, 0.54 mmol, 1.0 equiv.) and 1,3-difluoro-2-nitrosobenzene (192 mg, 1.34 mmol, 2.5 equiv.) were reacted in a Bayer-Mills reaction. Purification by silica column chromatography (petroleum ether/ethyl acetate 5:2) afforded 3-(4-(4-((2-fluorophenyl)diazenyl)phenyl)butoxy)-4-(pyridin-3-yl)-1,2,5-thiadiazole (172 mg, 0.38 mmol, 71%). ¹H-NMR (400 MHz, CDCl₃): *δ* 9.49 - 9.35 (m, 1H), 8.74 - 8.60 (m, 1H), 8.46 - 8.34 (m, 1H), 7.92 - 7.83 (m, 2H), 7.47 - 7.22 (m, 4H), 7.15 - 6.97 (m, 2H), 4.56 (t, J = 6.1 Hz, 2H), 2.80 (t, J = 7.2 Hz, 2H), 2.02 - 1.82 (m, 4H). ¹³C-NMR (101 MHz, CDCl₃): *δ* 162.73, 155.74 (dd, J = 258.6, 4.5 Hz), 151.77, 150.11, 148.56, 146.52, 144.93, 134.76, 131.54 (t, J = 10.3 Hz), 130.00 (t, J = 10.2 Hz), 129.19, 128.62, 127.68, 123.50, 123.20, 119.37, 112.87 - 112.26 (m), 71.05, 35.38, 28.43, 27.50. MS (ESI, positive): *m*/*z* calcd. for [C₂₃H₂₀N₅F₂OS]⁺: 452.14, found: 452.10[M+H]⁺.

### 3-((5-(4-((2-Fluorophenyl)diazenyl)phenyl)pentyl)oxy)-4-(pyridin-3-yl)-1,2,5-thiadiazole

According to general method D, 4-(5-((4-(pyridin-3-yl)-1,2,5-thiadiazol-3-yl)oxy)pentyl)-aniline (191 mg, 0.56 mmol, 1.0 equiv.) and 1-fluoro-2-nitrosobenzene (141mg, 1.13 mmol, 2.0 equiv.) were reacted in a Bayer-Mills reaction. Purification by silica column chromatography (petroleum ether/ethyl acetate 5:1) afforded 3-((5-(4-((2-fluorophenyl)diazenyl) phenyl)pentyl)oxy)-4-(pyridin-3-yl)-1,2,5-thiadiazole (208mg, 0.47 mmol, 83%). ¹H-NMR (400 MHz, CDCl₃): *δ* 9.46 - 9.32 (m, 1H), 8.72 - 8.56 (m, 1H), 8.44 - 8.33 (m, 1H), 7.91 - 7.82 (m, 2H), 7.79 - 7.70 (m, 1H), 7.48 - 7.16 (m, 6H), 4.52 (t, J = 6.5 Hz, 2H), 2.73 (t, J = 7.6 Hz, 2H), 1.93 (p, J = 6.8 Hz, 2H), 1.76 (p, J = 7.6 Hz, 2H), 1.60 - 1.51 (m, 2H). ¹³C-NMR (101 MHz, CDCl₃): *δ* 162.86, 160.10 (d, J = 257.4 Hz), 151.34, 150.13, 148.60, 146.46, 145.02, 140.86 (d, J = 6.9 Hz), 134.85, 132.28 (d, J = 8.2 Hz), 129.22, 127.79, 124.37 (d, J = 3.8 Hz), 123.58, 123.35, 117.87, 117.10 (d, J = 19.9 Hz), 71.30, 35.79, 30.88, 28.87, 25.70. MS (ESI, positive): *m*/*z* calcd. For [C₂₄H₂₃FN₅OS]⁺: 448.16, found: 448.15 [M+H]⁺.

### 3-((5-(4-((2,6-Difluorophenyl)diazenyl)phenyl)pentyl)oxy)-4-(pyridin-3-yl)-1,2,5-thiadiazole

According to general method D, 4-(5-((4-(pyridin-3-yl)-1,2,5-thiadiazol-3-yl)oxy)pentyl)-aniline (191 mg, 0.56 mmol, 1.0 equiv.) and 1,3-difluoro-2-nitrosobenzene (161 mg, 1.13 mmol, 2.0 equiv.) were reacted in a Bayer-Mills reaction. Purification by silica column chromatography (petroleum ether/ethyl acetate 5:1) afforded 3-((5-(4-((2,6-difluorophenyl) diazenyl)phenyl)pentyl)oxy)-4-(pyridin-3-yl)-1,2,5-thiadiazole (146 mg, 0.27 mmol, 56%). ¹H-NMR (400 MHz, CDCl₃): *δ* 9.48 - 9.33 (m, 1H), 8.73 - 8.59 (m, 1H), 8.50 - 8.40 (m, 1H), 7.90 - 7.79 (m, 2H), 7.47 - 7.38 (m, 1H), 7.36 - 7.27 (m, 3H), 7.08 - 6.98 (m, 2H), 4.54 (t, J = 6.5 Hz, 2H), 2.74 (t, J = 7.6 Hz, 2H), 1.95 (p, J = 6.7 Hz, 2H), 1.78 (p, J = 7.6 Hz, 2H), 1.62 - 1.51 (m, 2H). ¹³C-NMR (101 MHz, CDCl₃): *δ* 162.88, 155.82 (dd, J = 258.4, 4.4 Hz), 151.77, 150.20, 148.66, 147.09, 145.06, 134.84, 131.67 (t, J = 10.6 Hz), 130.00 (t, J = 10.2 Hz), 129.26, 128.66, 127.77, 123.56, 123.22, 112.82 - 112.31 (m), 71.31, 35.83, 30.89, 28.89, 25.73. MS (ESI, positive): *m*/*z* calcd. for [C₂₄H₂₂F₂N₅OS]⁺: 466.15, found: 466.15 [M+H]⁺.

### • 1.1.5. Preparation of Pyridinium Cations:

### 1-Methyl-3-(4-((4-(phenyldiazenyl)benzyl)oxy)-1,2,5-thiadiazol-3-yl)pyridin-1-ium iodide

According to general method F, 3-((4-(phenyldiazenyl)benzyl)oxy)-4-(pyridin-3-yl)-1,2,5-thiadiazole (29 mg, 0.078 mmol, 1.0 equiv.) was methylated with iodomethane (4.8 µL, 110 mg, 0.78 mmol, 10 equiv.) to give 1-methyl-3-(4-((4-(phenyldiazenyl)benzyl)oxy)-1,2,5-thiadiazol-3-yl)pyridin-1-ium iodide (31mg, 0.060 mmol, 78%) as an orange solid. ¹H-NMR (400 MHz, DMSO-d₆): *δ* 9.59 - 9.52 (m, 1H), 9.16 - 9.03 (m, 2H), 8.34 - 8.23 (m, 1H), 8.00 - 7.85 (m, 4H), 7.84 - 7.77 (m, 2H), 7.67 - 7.55 (m, 3H), 5.74 (s, 2H), 4.46 (s, 3H). ¹³C-NMR (101 MHz, DMSO-d₆): *δ* 162.29, 151.88, 151.75, 145.68, 144.21, 141.99, 141.40, 138.87, 131.72, 130.16, 129.51, 129.03, 127.99, 122.71, 122.57, 72.25, 48.54. MS (ESI, positive): *m*/*z* calcd. for [C₂₁H₁₈N₅OS]⁺: 388.12, found: 388.10 [M]⁺.

### 1-Methyl-3-(4-(4-(phenyldiazenyl)phenethoxy)-1,2,5-thiadiazol-3-yl)pyridin-1-ium iodide

According to general method F, 3-(4-(phenyldiazenyl)phenethoxy)-4-(pyridin-3-yl)-1,2,5-thiadiazole (350 mg, 0.90 mmol, 1.0 equiv.) was methylated with iodomethane (281 µL, 641 mg, 4.52 mmol, 5.0 equiv.) to give 1-methyl-3-(4-(4-(phenyldiazenyl)phenethoxy)-1,2,5-thiadiazol-3-yl)pyridin-1-ium iodide (409 mg, 0.78 mmol, 80%) as an orange solid. ¹H-NMR (400 MHz, DMSO-d₆): *δ* 9.44 - 9.38 (m, 1H), 9.09 - 9.02 (m, 1H), 9.02 - 8.95 (m, 1H), 8.30 - 8.23 (m, 1H), 7.92 - 7.84 (m, 4H), 7.67 - 7.54 (m, 5H), 4.84 (t, J = 6.3 Hz, 2H), 4.43 (s, 3H), 3.33 (t, J = 6.4 Hz, 2H). ¹³C-NMR (101 MHz, DMSO-d₆): *δ* 162.41, 151.88, 150.71, 145.67, 143.90, 142.01, 141.74, 141.02, 131.42, 130.05, 130.00, 129.44, 127.82, 122.70, 122.43, 71.85, 48.52, 34.20. MS (ESI, positive): *m*/*z* calcd. for [C₂₂H₂₀N₅OS]⁺: 402.14, found: 402.15 [M]⁺.

### 1-Methyl-3-(4-(3-(4-(phenyldiazenyl)phenyl)propoxy)-1,2,5-thiadiazol-3-yl)pyridin-1-ium iodide

According to general method F, 3-(3-(4-(phenyldiazenyl)phenyl)propoxy)-4-(pyridin-3-yl)-1,2,5-thiadiazole (90mg, 0.22 mmol, 1.0 equiv.) was methylated with iodomethane (139 µL, 318mg, 2.24 mmol, 10 equiv.) to give 1-methyl-3-(4-(3-(4-phenyldiazenyl)phenyl) propoxy)-1,2,5-thiadiazol-3-yl)pyridin-1-ium iodide (117 mg, 0.21 mmol, 96%) as an orange solid. ¹H-NMR (400 MHz, CDCl₃): *δ* 9.48 - 9.41 (m, 1H), 9.38 - 9.31 (m, 1H), 9.00 - 8.90 (m, 1H), 8.22 - 8.11 (m, 1H), 7.88 - 7.81 (m, 2H), 7.77 - 7.70 (m, 2H), 7.54 - 7.39 (m, 3H), 7.36 - 7.29 (m, 2H), 4.70 (s, 3H), 4.64 (t, J = 6.5 Hz, 2H), 2.88 (t, J = 7.3 Hz, 2H), 2.34 (p, J = 6.8 Hz, 2H). ¹³C-NMR (101 MHz, CDCl₃): *δ* 162.91, 152.55, 151.11, 145.49, 144.60, 142.92, 142.08, 139.62, 131.38, 131.12, 129.27, 129.25, 128.63, 123.03, 122.83, 71.96, 50.70, 32.50, 29.92. MS (ESI, positive): *m*/*z* calcd. For [C₂₃H₂₂N₅OS]⁺: 416.15, found: 416.10 [M]⁺.

### 1-Methyl-3-(4-(4-(4-(phenyldiazenyl)phenyl)butoxy)-1,2,5-thiadiazol-3-yl)pyridin-1-ium iodide

According to general method F, 3-(4-(4-(phenyldiazenyl)phenyl)butoxy)-4-(pyridin-3-yl)-1,2,5-thiadiazole (393 mg, 0.95 mmol, 1.0 equiv.) was methylated with iodomethane (884 µL, 2.02 g, 14.2 mmol, 10 equiv.) to give 1-methyl-3-(4-(4-(4-(phenyldiazenyl)phenyl) butoxy)-1,2,5-thiadiazol-3-yl)pyridin-1-ium iodide (399mg, 0.72 mmol, 76%) as an orange solid. ¹H-NMR (400 MHz, DMSO-d₆): *δ* 9.53 - 9.45 (m, 1H), 9.11 - 9.03 (m, 1H), 9.06 - 9.05 (m, 1H), 8.35 - 8.25 (m, 1H), 7.91 - 7.79 (m, 4H), 7.63 - 7.52 (m, 3H), 7.50 - 7.42 (m, 2H), 4.58 (t, J = 6.3 Hz, 2H), 4.46 (s, 3H), 2.79 (t, J = 7.4 Hz, 2H), 2.00 - 1.77 (m, 4H). ¹³C-NMR (101 MHz, DMSO-d₆): *δ* 162.66, 151.91, 150.26, 146.06, 145.59, 144.05, 141.81, 141.14, 131.28, 130.16, 129.42, 129.35, 127.94, 122.60, 122.37, 71.42, 48.52, 34.45, 27.73, 26.92. MS (ESI, positive): *m*/*z* calcd. for [C₂₄H₂₄N₅OS]⁺: 430.17, found: 430.15 [M]⁺.

### 1-Methyl-3-(4-((5-(4-(phenyldiazenyl)phenyl)pentyl)oxy)-1,2,5-thiadiazol-3-yl)pyridin-1-ium iodide

According to general method F, 3-((5-(4-(phenyldiazenyl)phenyl)pentyl)oxy)-4-(pyridin-3-yl)-1,2,5-thiadiazole (149 mg, 0.35 mmol, 1.0 equiv.) was methylated with iodomethane (110 µL, 251 mg, 1.73 mmol, 10 equiv.) to give 1-methyl-3-(4-((5-(4-(phenyldiazenyl)-phenyl)pentyl)oxy)-1,2,5-thiadiazol-3-yl)pyridin-1-ium iodide (197 mg, 0.34 mmol, 99%) as an orange solid. ¹H-NMR (400 MHz, DMSO-d₆): *δ* 9.51 - 9.45 (m, 1H), 9.08 - 9.01 (m, 2H), 8.31 - 8.23 (m, 1H), 7.91 - 7.83 (m, 2H), 7.84 - 7.75 (m, 2H), 7.65 - 7.51 (m, 3H), 7.44 - 7.39 (m, 2H), 4.56 (t, J = 6.6 Hz, 2H), 4.44 (s, 3H), 2.72 (t, J = 7.6 Hz, 2H), 1.93 (p, J = 6.8 Hz, 2H), 1.73 (p, J = 7.6 Hz, 2H), 1.56 - 1.46 (m, 2H). ¹³C-NMR (101 MHz, DMSO-d₆): *δ* 162.68, 151.92, 150.18, 146.28, 145.55, 144.07, 141.75, 141.10, 131.29, 130.17, 129.43, 129.29, 127.93, 122.56, 122.38, 71.55, 48.49, 34.77, 30.14, 27.87, 24.88. MS (ESI, positive): *m*/*z* calcd. for [C₂₅H₂₆N₅OS]⁺: 444.19, found: 444.15 [M]⁺.

### 1-Methyl-3-(4-((6-(4-(phenyldiazenyl)phenyl)hexyl)oxy)-1,2,5-thiadiazol-3-yl)pyridin- 1-ium iodide

According to general method F, 3-((6-(4-(phenyldiazenyl)phenyl)hexyl)oxy)-4-(pyridin- 3-yl)-1,2,5-thiadiazole (150 mg, 0.34 mmol, 1.0 equiv.) was methylated with iodomethane (106 µL, 241 mg, 1.70 mmol, 5.0 equiv.) to give 1-methyl-3-(4-((6-(4-(phenyldiazenyl)-phenyl)hexyl)oxy)-1,2,5-thiadiazol-3-yl)pyridin-1-ium iodide (196 mg, 0.33 mmol, 99%) as an orange solid. ¹H-NMR (400 MHz, DMSO-d₆): *δ* 9.53 - 9.45 (m, 1H), 9.10 - 9.01 (m, 2H), 8.32 - 8.25 (m, 1H), 7.90 - 7.83 (m, 2H), 7.85 - 7.77 (m, 2H), 7.64 - 7.52 (m, 3H), 7.45 - 7.38 (m, 2H), 4.54 (t, J = 6.6 Hz, 2H), 4.45 (s, 3H), 2.69 (t, J = 7.6 Hz, 2H), 1.88 (p, J = 6.8 Hz, 2H), 1.67 (p, J = 7.6 Hz, 2H), 1.57 - 1.35 (m, 4H). ¹³C-NMR (101 MHz, DMSO-d₆): *δ* 162.71, 151.93, 150.19, 146.44, 145.58, 144.06, 141.80, 141.11, 131.28, 130.18, 129.43, 129.30, 127.96, 122.57, 122.38, 71.67, 48.52, 34.85, 30.51, 28.21, 28.03, 25.10. MS (ESI, positive): *m*/*z* calcd. for [C₂₆H₂₈N₅OS]⁺: 458.20, found: 458.15 [M]⁺.

### 1-Methyl-3-(4-((7-(4-(phenyldiazenyl)phenyl)heptyl)oxy)-1,2,5-thiadiazol-3-yl)pyridin- 1-ium iodide

According to general method F, 3-((7-(4-(phenyldiazenyl)phenyl)heptyl)oxy)-4-(pyridin-3-yl)-1,2,5-thiadiazole (220 mg, 0.48 mmol, 1.0 equiv.) was methylated with iodomethane (150 µL, 340 mg, 2.40 mmol, 5.0 equiv.) to give 1-methyl-3-(4-((7-(4-(phenyldiazenyl)-phenyl)heptyl)oxy)-1,2,5-thiadiazol-3-yl)pyridin-1-ium iodide (226 mg, 0.38 mmol, 78%) as an orange solid. ¹H-NMR (400 MHz, DMSO-d₆): *δ* 9.51 - 9.46 (m, 1H), 9.10 - 9.01 (m, 2H), 8.32 - 8.25 (m, 1H), 7.89 - 7.84 (m, 2H), 7.84 - 7.78 (m, 2H), 7.63 - 7.52 (m, 3H), 7.44 - 7.38 (m, 2H), 4.53 (t, J = 6.6 Hz, 2H), 4.45 (s, 3H), 2.68 (t, J = 7.6 Hz, 2H), 1.87 (p, J = 6.9 Hz, 2H), 1.64 (p, J = 7.5 Hz, 2H), 1.48 - 1.31 (m, 6H). ¹³C-NMR (101 MHz, DMSO-d₆): *δ* 162.69, 151.91, 150.17, 146.47, 145.55, 144.06, 141.76, 141.10, 131.24, 130.16, 129.40, 129.26, 127.92, 122.56, 122.36, 71.65, 48.50, 34.91, 30.54, 28.45, 28.43, 28.07, 25.18. MS (ESI, positive): *m*/*z* calcd. for [C₂₇H₃₀N₅OS]⁺: 472.22, found: 472.20 [M]⁺.

### 3-(4-(4-(4-((2-Fluorophenyl)diazenyl)phenyl)butoxy)-1,2,5-thiadiazol-3-yl)-1-methylpyridin-1-ium iodide

According to general method F, 3-(4-(4-((2-fluorophenyl)diazenyl)phenyl)butoxy)-4-(pyridin-3-yl)-1,2,5-thiadiazole (123 mg, 0.28 mmol, 1.0 equiv.) was methylated with iodomethane (89 µL, 202mg, 1.42 mmol, 5.0 equiv.) to give 3-(4-(4-(4-((2-fluorophenyl)diazenyl)phenyl)butoxy)-1,2,5-thiadiazol-3-yl)-1-methylpyridin-1-ium iodide (124 mg, 0.22 mmol, 77%) as an orange solid. ¹H-NMR (400 MHz, DMSO-d₆): *δ* 9.54 - 9.43 (m, 1H), 9.13 - 8.99 (m, 2H), 8.35 - 8.22 (m, 1H), 7.92 - 7.77 (m, 2H), 7.74 - 7.67 (m, 1H), 7.66 - 7.58 (m, 1H), 7.55 - 7.41 (m, 3H), 7.40 - 7.29 (m, 1H), 4.59 (t, J = 6.3 Hz, 2H), 4.45 (s, 3H), 2.80 (t, J = 7.4 Hz, 2H), 2.02 - 1.77 (m, 4H). ¹³C-NMR (101 MHz, DMSO-d₆): *δ* 162.66, 159.14 (d, J = 255.3 Hz), 150.52, 146.67, 145.59, 144.07, 141.82, 141.16, 139.81 (d, J = 6.9 Hz), 133.26 (d, J = 7.8 Hz), 130.18, 129.44, 127.93, 125.00 (d, J = 3.5 Hz), 122.85, 117.46, 117.25 (d, J = 19.4 Hz), 71.41, 48.50, 34.49, 27.74, 26.90. MS (ESI, positive): *m*/*z* calcd. for [C₂₄H₂₃FN₅OS]⁺: 448.16, found: 448.15 [M]⁺.

### 3-(4-(4-(4-((2,6-Difluorophenyl)diazenyl)phenyl)butoxy)-1,2,5-thiadiazol-3-yl)-1-methylpyridin-1-ium iodide

According to general method F, 3-(4-(4-((2,6-difluorophenyl)diazenyl)phenyl)butoxy)-4-(pyridin-3-yl)-1,2,5-thiadiazole (107mg, 0.24 mmol, 1.0 equiv.) was methylated with iodomethane (74 µL, 168 mg, 1.19 mmol, 5.0 equiv.) to give 3-(4-(4-(4-((2,6-difluorophenyl)diazenyl)phenyl)butoxy)-1,2,5-thiadiazol-3-yl)-1-methylpyridin-1-ium iodide (124 mg, 0.21 mmol, 87%) as an orange solid. ¹H-NMR (400 MHz, DMSO-d₆): *δ* 9.52 - 9.45 (m, 1H), 9.11 - 9.02 (m, 2H), 8.34 - 8.25 (m, 1H), 7.84 - 7.78 (m, 1.4 H), 7.56 (tt, J = 8.4, 6.1 Hz, 1H), 7.50 - 7.45 (m, 1.4 H), 7.37 - 7.22 (m, 2.4 H), 7.14 - 7.06 (m, 0.6 H), 6.91 - 6.85 (m, 0.6 H), 4.63 - 4.50 (m, 2H), 4.48 - 4.42 (m, 3H), 2.87 - 2.60 (m, 2H), 1.98 - 1.68 (m, 4H). ¹³C-NMR (101 MHz, DMSO-d₆): *δ* 162.65, 154.70 (dd, J = 256.4, 4.3 Hz), 152.23, 150.91, 147.34, 145.59, 144.06, 141.92 - 141.68 (m), 141.15, 131.39 (d, J = 10.9 Hz), 130.17, 129.49, 128.87, 127.93, 122.65, 118.83, 113.52 - 112.49 (m), 71.40, 48.51, 34.49, 27.70, 26.86. MS (ESI, positive): *m*/*z* calcd. for [C₂₄H₂₂F₂N₅OS]⁺: 466.15, found: 466.15 [M]⁺.

### 3-(4-((5-(4-((2-Fluorophenyl)diazenyl)phenyl)pentyl)oxy)-1,2,5-thiadiazol-3-yl)-1-methylpyridin-1-ium iodide

According to general method F, 3-((5-(4-((2-fluorophenyl)diazenyl)phenyl)pentyl)oxy)-4-(pyridin-3-yl)-1,2,5-thiadiazole (201mg, 0.47 mmol, 1.0 equiv.) was methylated with iodomethane (289 µL, 660 mg, 4.65 mmol, 10 equiv.) to give 3-(4-((5-(4-((2-fluorophenyl)-diazenyl)phenyl)pentyl)oxy)-1,2,5-thiadiazol-3-yl)-1-methylpyridin-1-ium iodide (248 mg, 0.42 mmol, 90%) as an orange solid. ¹H-NMR (400 MHz, CDCl₃): *δ* 9.51 - 9.46 (m, 1H), 9.09 - 9.01 (m, 2H), 8.32 - 8.24 (m, 1H), 7.85 - 7.78 (m, 2H), 7.75 - 7.67 (m, 1H), 7.65 - 7.58 (m, 1H), 7.53 - 7.47 (m, 1H), 7.46 - 7.41 (m, 2H), 7.39 - 7.32 (m, 1H), 4.56 (t, J = 6.5 Hz, 2H), 4.44 (s, 3H), 2.73 (t, J = 7.6 Hz, 2H), 1.93 (p, J = 6.8 Hz, 2H), 1.73 (p, J = 7.6 Hz, 2H), 1.57 - 1.44 (m, 2H). ¹³C-NMR (101 MHz, CDCl₃): *δ* 162.68, 159.13 (d, J = 255.6 Hz), 150.43, 146.90, 144.04, 141.76, 141.10, 139.80 (d, J = 7.1 Hz), 133.25 (d, J = 8.2 Hz), 130.17, 129.38, 127.93, 124.99 (d, J = 3.7 Hz), 122.80, 117.46, 117.25 (d, J = 19.6 Hz), 71.56, 48.50, 34.80, 30.13, 27.87, 24.89. MS (ESI, positive): *m*/*z* calcd. for [C₂₅H₂₅FN₅OS]⁺: 462.18, found: 462.20 [M]⁺.

### 3-(4-((5-(4-((2,6-Difluorophenyl)diazenyl)phenyl)pentyl)oxy)-1,2,5-thiadiazol-3-yl)-1-methylpyridin-1-ium iodide

According to general method F, 3-((5-(4-((2,6-difluorophenyl)diazenyl)phenyl)pentyl)-oxy)-4-(pyridin-3-yl)-1,2,5-thiadiazole (123 mg, 0.26 mmol, 1.0 equiv.) was methylated with iodomethane (164 µL, 375 mg, 2.64 mmol, 10 equiv.) to give 3-(4-((5-(4-((2,6-difluorophenyl)diazenyl)phenyl)pentyl)oxy)-1,2,5-thiadiazol-3-yl)-1-methylpyridin-1-ium iodide (125 mg, 0.21 mmol, 79%) as an orange solid. ¹H-NMR (400 MHz, DMSO-d₆): *δ* 9.52 - 9.45 (m, 1H), 9.10 - 9.01 (m, 2H), 8.31 - 8.23 (m, 1H), 7.83 - 7.76 (m, 1H), 7.59 - 7.51 (m, 0.5H), 7.49 - 7.41 (m, 1H), 7.40 - 7.25 (m, 1.5H), 7.25 - 7.18 (m, 1H), 7.15 - 7.05 (m, 1H), 6.93 - 6.80 (m, 1H), 4.60 - 4.48 (m, 2H), 4.45 (s, 3H), 2.74 (t, 1H), 2.06 - 1.78 (m, 2H), 1.78 - 1.57 (m, 2H), 1.55 - 1.37 (m, 2H). ¹³C-NMR (101 MHz, DMSO-d₆): *δ* 162.70, 152.17, 150.87, 147.59, 145.60, 144.09, 143.68, 141.88 - 141.65 (m), 141.14, 130.29 - 130.08 (m), 129.46, 128.83, 127.93, 122.63, 118.80, 113.20 - 112.75 (m), 71.57, 48.48, 34.83, 30.13, 27.89, 24.89. MS (ESI, positive): *m*/*z* calcd. For [C₂₅H₂₄F₂N₅OS]⁺: 480.17 found: 480.15 [M]⁺.

### • 1.1.6. Preparation of Examples and Comparative Examples of formula (I):

### 3-(1-Methyl-1,2,5,6-tetrahydropyridin-3-yl)-4-(4-(phenyldiazenyl)phenethoxy)-1,2,5-thiadiazole (Comparative Example 27b)

According to general method G, 1-methyl-3-(4-(4-(phenyldiazenyl)phenethoxy)-1,2,5-thiadiazol-3-yl)pyridin-1-ium iodide (356mg, 0.67 mmol, 1.0 equiv.) was reduced with sodium tetrahydroborate (71 mg, 1.88 mmol, 2.8 equiv.) in a mixture of CH₂Cl₂/MeOH (~5 mL). Purification by silica column chromatography (CH₂Cl₂/MeOH 30:1) afforded 3-(1-methyl-1,2,5,6-tetrahydropyridin-3-yl)-4-(4-(phenyldiazenyl)phenethoxy)-1,2,5-thiadiazole (134 mg, 0.33 mmol, 49%) as an orange film. ¹H-NMR (400 MHz, CDCl₃): *δ* 7.94 - 7.85 (m, 4H), 7.56 - 7.46 (m, 3H), 7.43 (d, J = 8.1 Hz, 2H), 6.96 - 6.90 (m, 1H), 4.73 (t, J = 6.7 Hz, 2H), 3.47 - 3.39 (m, 2H), 3.24 (t, J = 6.7 Hz, 2H), 2.57 (t, J = 5.7 Hz, 2H), 2.49 - 2.36 (m, 5H). ¹³C-NMR (101 MHz, CDCl₃): *δ* 162.21, 152.81, 151.71, 146.94, 141.27, 131.06, 129.82, 129.21, 129.04, 128.76, 123.19, 122.94, 71.02, 54.96, 51.27, 45.92, 35.44, 26.50. MS (ESI, positive): *m*/*z* calcd. for [C₂₂H₂₄N₅OS]⁺: 406.17, found: 406.20 [M+H]⁺. Purity (HPLC_{254 nm}) = 98.7%.

### 3-(1-Methyl-1,2,5,6-tetrahydropyridin-3-yl)-4-(3-(4-(phenyldiazenyl)phenyl) propoxy)-1,2,5-thiadiazole (Comparative Example 27c)

According to general method G, 1-methyl-3-(4-(3-(4-(phenyldiazenyl)phenyl)propoxy)-1,2,5-thiadiazol-3-yl)pyridin-1-ium iodide (250 mg, 0.46 mmol, 1.0 equiv.) was reduced with sodium tetrahydroborate (52 mg, 1.38 mmol, 3.0 equiv.) in CH₂Cl₂/MeOH (~3 mL). Purification by silica column chromatography (CH₂Cl₂/MeOH 20:1) and subsequent purification by reverse phase flash chromatography (H₂O/MeOH) afforded 3-(1-methyl-1,2,5,6-tetrahydropyridindin-3-yl)-4-(3-(4-(phenyldiazenyl)phenyl)propoxy)-1,2,5-thiadiazole (65mg, 0.16 mmol, 34%) as an orange film. ¹H-NMR (400 MHz, CDCl₃): *δ* 7.95 - 7.81 (m, 4H), 7.54 - 7.41 (m, 3H), 7.38 - 7.30 (m, 2H), 7.08 - 7.04 (m, 1H), 4.49 (t, J = 6.4 Hz, 2H), 3.52 - 3.42 (m, 2H), 2.87 (t, J = 7.6 Hz, 2H), 2.57 (t, J = 5.7 Hz, 2H), 2.45 2.49 - 2.42 (m, 5H), 2.22 (p, J = 6.6 Hz, 2H). ¹³C-NMR (101 MHz, CDCl₃): *δ* 162.39, 152.77, 151.31, 146.85, 144.56, 130.92, 129.40, 129.20, 129.14, 128.44, 123.17, 122.85, 70.02, 55.06, 51.27, 45.95, 32.30, 30.34, 26.66. MS (ESI, positive): *m*/*z* calcd. for [C₂₃H₂₆N₅OS]⁺: 420.29, found: 420.20 [M+H]⁺. Purity (HPLC_{254 nm}) = 99.8%.

### 3-(1-Methyl-1,2,5,6-tetrahydropyridindin-3-yl)-4-(4-(4-(phenyldiazenyl)phenyl) butoxy)-1,2,5-thiadiazole (Example 27d)

According to general method G, 1-methyl-3-(4-(4-(4-(phenyldiazenyl)phenyl)butoxy)-1,2,5-thiadiazol-3-yl)pyridin-1-ium iodide (365 mg, 0.66 mmol, 1.0 equiv.) was reduced with sodium tetrahydroborate (74 mg, 1.97 mmol, 3.0 equiv.) in CH₂Cl₂/MeOH (~4 mL). Purification by silica column chromatography (CH₂Cl₂/MeOH 20:1) and subsequent purification by reverse phase flash chromatography (H₂O/MeOH) afforded 3-(1-methyl-1,2,5,6-tetrahydropyridin-3-yl)-4-(4-(4-(phenyldiazenyl)phenyl)butoxy)-1,2,5-thiadiazole (84 mg, 0.19 mmol, 30%) as an orange film. ¹H-NMR (400 MHz, CDCl₃): *δ* 7.98 - 7.81 (m, 4H), 7.56 - 7.41 (m, 3H), 7.37 - 7.28 (m, 2H), 7.10 - 6.99 (m, 1H), 4.47 (t, J = 6.0 Hz, 2H), 3.49 - 3.42 (m, 2H), 2.77 (t, J = 7.1 Hz, 2H), 2.56 (t, J = 5.7 Hz, 2H), 2.50 - 2.37 (m, 5H), 1.96 - 1.77 (m, 4H). ¹³C-NMR (101 MHz, CDCl₃): *δ* 162.49, 152.79, 151.19, 146.89, 145.50, 130.84, 129.45, 129.14, 129.12, 128.42, 123.06, 122.82, 70.59, 55.11, 51.30, 45.99, 35.34, 28.43, 27.62, 26.70. MS (ESI, positive): *m*/*z* calcd. for [C₂₄H₂₈N₅OS]⁺: 434.20, found: 434.20 [M+H]⁺. Purity (HPLC_{254 nm}) = 99.8%.

### 3-(1-Methyl-1,2,5,6-tetrahydropyridin-3-yl)-4-((5-(4-(phenyldiazenyl)phenyl)-pentyl)oxy)-1,2,5-thiadiazole (Example 27e)

According to general method G, 1-methyl-3-(4-((5-(4-(phenyldiazenyl)phenyl)pentyl)-oxy)-1,2,5-thiadiazol-3-yl)pyridin-1-ium iodide (170mg, 0.30 mmol, 1.0 equiv.) was reduced with sodium tetrahydroborate (32 mg, 0.83 mmol, 2.8 equiv.) in CH₂Cl₂/MeOH (~2 mL). Purification by silica column chromatography (CH₂Cl₂/MeOH 20:1) and subsequent purification by reverse phase flash chromatography (H₂O/MeOH) afforded the desired 3-(1-methyl-1,2,5,6-tetrahydropyridin-3-yl)-4-((5-(4-(phenyldiazenyl)phenyl)-pentyl)oxy)-1,2,5-thiadiazole (56mg, 0.13 mmol, 42%) as an orange film. ¹H-NMR (400 MHz, CDCl₃): *δ* 7.94 - 7.81 (m, 4H), 7.56 - 7.40 (m, 3H), 7.38 - 7.28 (m, 2H), 7.05 - 6.98 (m, 1H), 4.45 (t, J = 6.5 Hz, 2H), 3.63 - 3.37 (m, 2H), 2.72 (t, J = 7.6 Hz, 2H), 2.56 (t, J = 5.7 Hz, 2H), 2.45 (s, 3H), 2.45 - 2.38 (m, 2H), 1.89 (p, J = 6.8 Hz, 2H), 1.82 - 1.69 (m, 2H), 1.53 (qd, J = 9.6, 8.8, 5.7 Hz, 2H). ¹³C-NMR (101 MHz, CDCl₃): *δ* 162.58, 152.85, 151.15, 146.84, 145.96, 130.86, 129.22, 129.19, 129.17, 128.41, 123.05, 122.84, 70.86, 54.99, 51.26, 45.89, 35.80, 30.92, 28.86, 26.57, 25.74. MS (ESI, positive): *m*/*z* calcd. for [C₂₅H₃₀N₅OS]⁺: 448.22, found: 448.15 [M+H]⁺. Purity (HPLC_{254 nm}) = 96.5%.

### 3-(1-Methyl-1,2,5,6-tetrahydropyridin-3-yl)-4-((6-(4-(phenyldiazenyl)phenyl)hexyl)-oxy)-1,2,5-thiadiazole (Example 27f)

According to general method G, 1-methyl-3-(4-((6-(4-(phenyldiazenyl)phenyl)hexyl)oxy)-1,2,5-thiadiazol-3-yl)pyridin-1-ium iodide (157mg, 0.27 mmol, 1.0 equiv.) was reduced with sodium tetrahydroborate (30 mg, 0.81 mmol, 3.0 equiv.) in CH₂Cl₂/MeOH (~1 mL). Purification by silica column chromatography (CH₂Cl₂/MeOH 20:1) and subsequent purification by reverse phase flash chromatography (H₂O/MeOH) afforded 3-(1-methyl-1,2,5,6-tetrahydropyridin-3-yl)-4-((6-(4-(phenyldiazenyl)phenyl)hexyl)oxy)-1,2,5-thiadiazole (66mg, 0.14 mmol, 53%) as an orange film. ¹H-NMR (400 MHz, CDCl₃): *δ* 7.92 - 7.88 (m, 2H), 7.88 - 7.82 (m, 2H), 7.55 - 7.41 (m, 3H), 7.35 - 7.28 (m, 2H), 7.08 - 7.03 (m, 1H), 4.44 (t, J = 6.6 Hz, 2H), 3.48 - 3.43 (m, 2H), 2.70 (t, J = 7.7 Hz, 2H), 2.57 (t, J = 5.7 Hz, 2H), 2.48 - 2.41 (m, 5H), 1.85 (p, J = 14.5, 6.8 Hz, 2H), 1.70 (p, J = 7.5 Hz, 2H), 1.57 - 1.37 (m, 4H). ¹³C-NMR (101 MHz, CDCl₃): *δ* 162.61, 152.85, 151.12, 146.91, 146.24, 130.81, 129.45, 129.18, 129.14, 128.41, 123.01, 122.83, 70.94, 55.14, 51.35, 46.02, 35.84, 31.23, 28.91, 28.91, 26.72, 25.96. MS (ESI, positive): *m*/*z* calcd. for [C₂₆H₃₂N₅OS]⁺: 462.23, found: 462.20 [M+H]⁺. Purity (HPLC_{254 nm}) = 98.3%.

### 3-(1-Methyl-1,2,5,6-tetrahydropyridin-3-yl)-4-((7-(4-(phenyldiazenyl)phenyl)heptyl) oxy)-1,2,5-thiadiazole (Comparative Example 27g)

According to general method G, 1-methyl-3-(4-((7-(4-(phenyldiazenyl)phenyl)heptyl)-oxy)-1,2,5-thiadiazol-3-yl)pyridin-1-ium iodide (200 mg, 0.33 mmol, 1.0 equiv.) was reduced with sodium tetrahydroborate (38 mg, 1.00 mmol, 3.0 equiv.) in CH₂Cl₂/MeOH (~2 mL). Purification by silica column chromatography (CH₂Cl₂/MeOH 20:1) and subsequent purification by reverse phase flash chromatography (H₂O/MeOH) yielded 3-(1-methyl-1,2,5,6-tetrahydropyridin-3-yl)-4-((7-(4-(phenyldiazenyl)phenyl)heptyl)oxy)-1,2,5-thiadiazole (100 mg, 0.21 mmol, 63%) as an orange film. ¹H-NMR (400 MHz, CDCl₃): *δ* 7.93 - 7.88 (m, 2H), 7.88 - 7.82 (m, 2H), 7.57 - 7.41 (m, 3H), 7.37 - 7.27 (m, 2H), 7.09 - 7.02 (m, 1H), 4.44 (t, J = 6.6 Hz, 2H), 3.51 - 3.45 (m, 2H), 2.69 (t, J = 7.7 Hz, 2H), 2.61 - 2.56 (m, 2H), 2.52 - 2.40 (m, 5H), 1.84 (p, J = 6.7 Hz, 2H), 1.68 (p, J = 7.5 Hz, 2H), 1.52 - 1.32 (m, 6H). ¹³C-NMR (101 MHz, CDCl₃): *δ* 162.65, 152.88, 151.11, 146.81, 146.43, 130.82, 129.20, 129.17, 128.37, 123.01, 122.84, 71.02, 54.96, 51.25, 45.86, 35.96, 31.29, 29.22, 29.20, 28.96, 26.51, 26.03. MS (ESI, positive): *m*/*z* calcd. for [C₂₇H₃₄N₅OS]⁺: 476.25, found: 476.20 [M+H]⁺. Purity (HPLC_{254 nm}) = 99.6%.

### 3-(4-(4-((2-Fluorophenyl)diazenyl)phenyl)butoxy)-4-(1-methyl-1,2,5,6-tetrahydropyridin-3-yl)-1,2,5-thiadiazole (Example 28a)

According to general method G, 3-(4-(4-(4-((2-fluorophenyl)diazenyl)phenyl)butoxy)-1,2,5-thiadiazol-3-yl)-1-methylpyridin-1-ium iodide (191 mg, 0.33 mmol, 1.0 equiv.) was reduced with sodium tetrahydroborate (38mg, 1.00 mmol, 3.0 equiv.) in a mixture of CH₂Cl₂/MeOH (1.3 mL). Purification by silica column chromatography (CH₂Cl₂/MeOH 20:1) and subsequent purification by reverse phase flash chromatography (H₂O/MeOH) yielded the desired 3-(4-(4-((2-fluorophenyl)diazenyl)phenyl)butoxy)-4-(1-methyl-1,2,5,6-tetrahydropyridin-3-yl)-1,2,5-thiadiazole (69 mg, 0.15 mmol, 46%) as an orange film. ¹H-NMR (400 MHz, CDCl₃): *δ* 7.94 - 7.87 (m, 2H), 7.80 - 7.73 (m, 1H), 7.50 - 7.40 (m, 1H), 7.38 - 7.32 (m, 2H), 7.32 - 7.18 (m, 2H), 7.10 - 7.02 (m, 1H), 4.50 (t, J = 6.0 Hz, 2H), 3.51 - 3.43 (m, 2H), 2.80 (t, J = 7.0 Hz, 2H), 2.59 (t, J = 5.7 Hz, 2H), 2.48 - 2.46 (m, 6H), 1.99 - 1.81 (m, 4H). ¹³C-NMR (101 MHz, CDCl₃): *δ* 162.53, 160.12 (d, J = 257.3 Hz), 151.44, 146.92, 146.11, 140.86 (d, J = 6.8 Hz), 132.32 (d, J = 8.3 Hz), 129.45, 129.23, 128.47, 124.38 (d, J = 3.8 Hz), 123.41, 117.89, 117.12 (d, J = 19.9 Hz), 70.63, 55.14, 51.35, 46.02, 35.44, 28.49, 27.66, 26.73. MS (ESI, positive): *m*/*z* calcd. for [C₂₄H₂₇FN₅OS]⁺: 452.19, found: 452.15 [M+H]⁺. Purity (HPLC_{254 nm}) = 98.8%.

### 3-(4-(4-((2,6-Difluorophenyl)diazenyl)phenyl)butoxy)-4-(1-methyl-1,2,5,6-tetrahydropyridin-3-yl)-1,2,5-thiadiazole (Comparative Example 28b)

According to general method G, 3-(4-(4-(4-((2,6-difluorophenyl)diazenyl)phenyl)butoxy)-1,2,5-thiadiazol-3-yl)-1-methylpyridin-1-ium iodide (180 mg, 0.30 mmol, 1.0 equiv.) was reduced with sodium tetrahydroborate (34mg, 0.91 mmol, 3.0 equiv.) in a mixture of CH₂Cl₂/MeOH (1.2 mL). Purification by silica column chromatography (CH₂Cl₂/MeOH 20:1) and subsequent purification by reverse phase flash chromatography (H₂O/MeOH) yielded the desired 3-(4-(4-((2-fluorophenyl)diazenyl)phenyl)butoxy)-4-(1-methyl-1,2,5,6- tetrahydropyridin-3-yl)-1,2,5-thiadiazole (71 mg, 0.15 mmol, 50%) as an orange film. ¹H-NMR (400 MHz, CDCl₃): *δ* 7.89 - 7.85 (m, 2H), 7.36 - 7.32 (m, 2H), 7.32 - 7.26 (m, 1H), 7.13 - 6.97 (m, 3H), 4.48 (t, J = 6.0 Hz, 2H), 3.49 - 3.42 (m, 2H), 2.78 (t, J = 7.1 Hz, 2H), 2.59 - 2.55 (m, 2H), 2.48 - 2.41 (m, 5H), 1.95 - 1.76 (m, 4H). ¹³C-NMR (101 MHz, CDCl₃): *δ* 162.51, 155.81 (dd, J = 258.7, 4.4 Hz), 151.84, 146.91, 146.72, 131.63 (t, J = 10.3 Hz), 130.03 (t, J = 10.3 Hz), 129.24, 128.46, 123.24, 119.41, 113.00 - 112.09 (m), 70.60, 55.13, 51.34, 46.01, 35.44, 28.46, 27.64, 26.72. MS (ESI, positive): *m*/*z* calcd. for [C₂₄H₂₆F₂N₅OS]⁺: 470.18, found: 470.15 [M+H]⁺. Purity (HPLC_{254 nm}) = 98.2%.

### 3-((5-(4-((2-Fluorophenyl)diazenyl)phenyl)pentyl)oxy)-4-(1-methyl-1,2,5,6-tetrahydropyridin- 3-yl)-1,2,5-thiadiazole (Example 29a)

According to general method G, 3-(4-((5-(4-((2-fluorophenyl)diazenyl)phenyl)pentyl)-oxy)-1,2,5-thiadiazol-3-yl)-1-methylpyridin-1-ium iodide (190 mg, 0.32 mmol, 1.0 equiv.) was reduced with sodium tetrahydroborate (37 mg, 0.97 mmol, 3.0 equiv.) in a mixture of CH₂Cl₂/MeOH (2 mL). Purification by silica column chromatography (CH₂Cl₂/MeOH 20:1) and subsequent purification by reverse phase flash chromatography (H₂O/MeOH) yielded the desired 3-(4-(4-((2-fluorophenyl)diazenyl)phenyl)butoxy)-4-(1-methyl-1,2,5,6-tetrahydropyridin-3-yl)-1,2,5-thiadiazole (70 mg, 0.15 mmol, 47%) as an orange film. ¹H-NMR (400 MHz, CDCl₃): *δ* 7.92 - 7.86 (m, 2H), 7.80 - 7.73 (m, 1H), 7.48 - 7.39 (m, 1H), 7.36 - 7.30 (m, 2H), 7.30 - 7.19 (m, 2H), 7.05 - 7.00 (m, 1H), 4.46 (t, J = 6.6 Hz, 2H), 3.51 - 3.42 (m, 2H), 2.73 (t, J = 7.6 Hz, 2H), 2.56 (t, J = 5.8 Hz, 2H), 2.46 (s, 3H), 2.44 - 2.38 (m, 2H), 1.89 (p, J = 7.1 Hz, 2H), 1.76 (p, J = 7.7 Hz, 2H), 1.59 - 1.48 (m, 2H). ¹³C-NMR (101 MHz, CDCl₃): *δ* 162.53, 160.07 (d, J = 257.3 Hz), 151.31, 146.91, 146.48, 140.83 (d, J = 6.9 Hz), 132.23 (d, J = 8.2 Hz), 129.40, 129.18, 128.43, 124.33 (d, J = 3.7 Hz), 123.32, 117.84, 117.06 (d, J = 19.8 Hz), 70.80, 55.11, 51.30, 45.98, 35.79, 30.84, 28.82, 26.70, 25.71. MS (ESI, positive): *m*/*z* calcd. for [C₂₅H₂₉FN₅OS]⁺: 466.21, found: 466.20 [M+H]⁺. Purity (HPLC_{254 nm}) = 99.2%.

### 3-((5-(4-((2,6-Difluorophenyl)diazenyl)phenyl)pentyl)oxy)-4-(1-methyl-1,2,5,6-tetrahydropyridin-3-yl)-1,2,5-thiadiazole (Example 29b)

According to general method G, 3-(4-((5-(4-((2,6-difluorophenyl)diazenyl)phenyl)pentyl)-oxy)-1,2,5-thiadiazol-3-yl)-1-methylpyridin-1-ium iodide (93 mg, 0.153 mmol, 1.0 equiv.) was reduced with sodium tetrahydroborate (18 mg, 0.459 mmol, 3.0 equiv.) in a mixture of CH₂Cl₂/MeOH (2 mL). Purification by silica column chromatography (CH₂Cl₂/MeOH 20:1) and subsequent purification by reverse phase flash chromatography (H₂O/MeOH) afforded 3-((5-(4-((2,6-difluorophenyl)diazenyl)phenyl)pentyl)oxy)-4-(1-methyl-1,2,5,6-tetrahydropyridin-3-yl)-1,2,5-thiadiazole as an orange film (38 mg, 0.120 mmol, 78%). ¹H-NMR (400 MHz, CDCl₃): *δ* 7.81 - 7.75 (m, 2H), 7.27 - 7.16 (m, 2H), 7.06 - 6.89 (m, 3H), 4.37 (t, J = 6.5 Hz, 2H), 3.40 - 3.33 (m, 2H), 2.65 (t, J = 7.6 Hz, 2H), 2.47 (t, J = 5.7 Hz, 2H), 2.37 (s, 3H), 2.35 - 2.29 (m, 2H), 1.87 - 1.73 (m, 2H), 1.74 - 1.52 (m, 2H), 1.49 - 1.32 (m, 2H). ¹³C-NMR (101 MHz, CDCl₃): *δ* 162.56, 155.80 (dd, J = 258.6, 4.5 Hz), 151.75, 147.14, 146.93, 131.66 (t, J = 10.4 Hz), 129.98 (t, J = 10.3 Hz), 129.23, 128.47, 123.19, 119.38, 112.88 - 112.27 (m), 70.83, 55.12, 51.31, 45.99, 35.84, 30.87, 28.85, 26.71, 25.74. MS (ESI, positive): *m*/*z* calcd. for [C₂₅H₂₈F₂N₅OS]⁺:484.20, found: 484.20 [M+H]⁺. Purity (HPLC_{254 nm}) = 98.0%.

### 1.2. Preparation of a tetra-ortho-fluoro-substituted compound of formula (I), wherein R¹-R⁴ are F. R⁵ is O and n is 5.

### 5-(4-amino-3,5-difluorophenyl)pent-4-yn-1-ol

According to general method H, 4-bromo-2,6-difluoroaniline (500 mg, 2.40 mmol, 1.0 equiv.) was coupled with pent-4-yn-1-ol (447 µL, 404 mg, 4.80 mmol, 2.0 equiv.) using triphenylphosphine, copper(I) iodide and bis(triphenylphosphine)-palladium(II) chloride, stirring the mixture in a solution of NEt₃/THF (10 mL) at 90°C for 15 hours. Purification by silica column chromatography (petroleum ether/ethyl acetate 5:2) afforded 5-(4-amino-3,5-difluorophenyl)pent-4-yn-1-ol 365 mg, 1.73 mmol, 72%) as brown solid. ¹H NMR (400 MHz, Methanol-*d*₄) δ 6.88 - 6.76 (m, 2H), 3.67 (t, *J* = 6.3 Hz, 2H), 2.45 (t, *J* = 7.1 Hz, 2H), 1.77 (p, *J* = 6.7 Hz, 2H). ¹³C NMR (101 MHz, Methanol-*d*₄) δ 152.52 (dd, *J* = 239.3, 9.7 Hz), 126.87 (t, *J =* 16.6 Hz), 115.54 -114.40 (m), 112.52 - 111.86 (m), 88.94, 80.35 (t), 61.60, 32.70, 16.41. MS (ESI, positive): m/z calcd. for [C₁₁H₁₂F₂NO]⁺: 212.09, found: 212.10 [M+H]⁺.

### 5-(4-amino-3,5-difluorophenyl)pentan-1-ol

To a solution of 5-(4-amino-3,5-difluorophenyl)pent-4-yn-1-ol (330 mg, 1.56 mmol) in MeOH (5 mL) was given palladium on activated charcoal (66 mg, 20 wt%). The reaction was flushed with H₂ and stirred under an atmosphere of H₂ at ambient temperature for 3 hours. Afterwards, the mixture was ordinarily filtrated over a paper filter and concentrated to give 5-(4-amino-3,5-difluorophenyl)pentan-1-ol (284 mg, 1.32 mmol, 85%) as a brown oil. ¹H NMR (400 MHz, Methanol-*d*₄) δ 6.73 - 6.60 (m, 2H), 3.53 (t, *J* = 6.6 Hz, 2H), 2.50 (t, *J* = 7.6 Hz, 2H), 1.62 - 1.51 (m, 4H), 1.41 - 1.32 (m, 2H). ¹³C NMR (101 MHz, Methanol-*d*₄) δ 153.54 (dd, *J* = 239.1, 8.6 Hz), 133.15 (t), 123.20 (m), 111.81 - 111.27 (m), 62.88, 35.79 (t), 33.43, 32.29, 26.38. MS (ESI, positive): m/z calcd. for [C₁₁H₁₆F₂NO]⁺: 216.12, found: 216.10 [M+H]⁺.

### 2,6-difluoro-4-(5-((4-(pyridin-3-yl)-1,2,5-thiadiazol-3-yl)oxy)pentyl)aniline

According to general procedure I, 3-chloro-4-(pyridin-3-yl)-1,2,5-thiadiazole (202 mg, 1.02 mmol, 1.05 equiv.) was reacted with 5-(4-amino-3,5-difluorophenyl)pentan-1-ol (209mg, 0.97 mmol, 1.0 equiv.) using NaH (117 mg, 2.91 mmol, 3.0 equiv.) in dry THF (4 mL) stirring the reaction for one overnight at 70°C. Purification by column chromatography (petroleum ether/ethyl acetate 5:1 à 4:1) yielded 2,6-difluoro-4-(5-((4-(pyridin-3-yl)-1,2,5-thiadiazol-3-yl)oxy)pentyl)aniline (223 mg, 0.59 mmol, 47%) as a beige solid. ¹H NMR (400 MHz, Chloroform-d) δ 9.47 - 9.32 (m, 1H), 8.75 - 8.58 (m, 1H), 8.53 - 8.37 (m, 1H), 7.51 - 7.37 (m, 1H), 6.70 - 6.56 (m, 2H), 4.52 (t, *J* = 6.6 Hz, 2H), 3.65 (s, 2H), 2.52 (t, *J* = 7.5 Hz, 2H), 2.01 - 1.85 (m, 2H), 1.73 - 1.59 (m, 2H), 1.57 - 1.44 (m, 2H). ¹³C NMR (101 MHz, Chloroform-d) δ 162.92, 152.13 (dd, *J* = 240.0, 8.3 Hz), 149.51, 148.02, 144.72, 135.45, 131.86 (t, *J* = 7.8 Hz), 128.06, 123.81, 121.50 (t, *J* = 16.4 Hz), 110.82 (dd, *J* = 14.5, 6.9 Hz), 71.41, 34.90 (t), 30.91, 28.86, 25.53. MS (ESI, positive): m/z calcd. for [C₁₈H₁₉F₂N₄OS]⁺: 377.12, found: 377.10 [M+H]⁺.

### 3-((5-(4-((2,6-difluorophenyl)diazenyl)-3,5-difluorophenyl)pentyl)oxy)-4-(pyridin-3-yl)-1,2,5-thiadiazole

A solution of 1,3-difluoro-2-nitrosobenzene (119 mg, 0.85 mmol, 2.0 equiv.) and 2,6-difluoro-4-(5-((4-(pyridin-3-yl)-1,2,5-thiadiazol-3-yl)oxy)pentyl)aniline (160 mg, 0.425 mmol, 1 equiv.) in acetic acid/toluene/TFA (6:6:1; 9 mL) was stirred overnight at ambient temperature. The reaction mixture was concentrated under reduced pressure. The residue was dissolved in EtOAc and washed with sat. aq. NaHCO₃ and brine. After drying the organic layer over Na₂SO₄, it was concentrated and purified by silica column chromatography (petroleum ether/ethyl acetate 5:1) yielding 3-((5-(4-((2,6-difluorophenyl)diazenyl)-3,5-difluorophenyl)pentyl)oxy)-4-(pyridin-3-yl)-1,2,5-thiadiazole (167 mg, 0.333 mmol, 79%) as a red oil. ¹H NMR (400 MHz, Chloroform-d) δ 9.41 - 9.33 (m, 1H), 8.67 - 8.59 (m, 1H), 8.44 - 8.32 (m, 1H), 7.46 - 7.27 (m, 2H), 7.10 - 6.96 (m, 2H), 6.90 - 6.79 (m, 2H), 4.52 (t, *J* = 6.5 Hz, 2H), 2.67 (t, *J* = 7.7 Hz, 2H), 1.99 - 1.85 (m, 2H), 1.78 - 1.63 (m, 2H), 1.60 - 1.45 (m, 2H). ¹³C NMR (101 MHz, Chloroform-d) δ 162.77, 155.72 (dd, *J* = 261.6, 4.9 Hz), 155.58 (dd, *J* = 260.4, 4.1 Hz), 150.14, 148.51, 148.12 (t, *J* = 9.5 Hz), 144.97, 134.84, 131.99 (t, *J* = 10.0 Hz), 131.14 (t, *J* = 10.4 Hz), 129.70, 127.72, 123.55, 112.84 - 112.30 (m), 71.15, 35.77 - 35.77 (m), 30.24, 28.77, 25.64. MS (ESI, positive): m/z calcd. for [C₂₄H₂₀F₄N₅OS]⁺:502.13, found: 502.10 [M+H]⁺.

### 3-(4-((5-(4-((2,6-difluorophenyl)diazenyl)-3,5-difluorophenyl)pentyl)oxy)-1,2,5-thiadiazol-3-yl)-1-methylpyridin-1-ium iodide

According to general method K, 3-((5-(4-((2,6-difluorophenyl)diazenyl)-3,5-difluorophenyl)pentyl)oxy)-4-(pyridin-3-yl)-1,2,5-thiadiazole (160 mg, 0.32 mmol, 1.0 equiv.) was methylated using iodomethane (100 µL, 1.6 mmol, 5.0 equiv.) in acetone, stirring the reaction for one overnight at ambient temperature. Purification by silica column chromatography (dichloromethane/methanol 20:1) yielded 3-(4-((5-(4-((2,6-difluorophenyl)diazenyl)-3,5-difluorophenyl)pentyl)oxy)-1,2,5-thiadiazol-3-yl)-1-methylpyridin-1-ium iodide (180 mg, 0.28 mmol, 87%) as a red solid. ¹H NMR (400 MHz, Chloroform-d) δ 9.47 (s, 2H), 9.11 - 9.08 (m, 1H), 8.32 - 8.22 (m, 1H), 7.40 - 7.12 (m, 1H), 7.08 - 6.97 (m, 1H), 6.93 - 6.77 (m, 2H), 6.71 -6.62 (m, 1H), 4.77 (s, 3H), 4.63 - 4.51 (m, 2H), 2.70 (t, *J* = 7.6 Hz, 1H), 2.58 (t, 1H), 2.04 - 1.90 (m, 2H), 1.69 (dp, *J =* 36.3, 7.5 Hz, 2H), 1.57 - 1.40 (m, 2H). ¹³C NMR (101 MHz, Chloroform-d) δ 163.11, 156.84 (t, *J=* 4.7 Hz), 154.25 (t, *J =* 4.3 Hz), 151.72 (dd, *J* = 253.8, 5.5 Hz), 151.52 (dd, *J* = 252.8, 6.1 Hz), 148.34 (t, *J* = 9.6 Hz), 146.23 (t, *J* = 8.4 Hz), 145.55, 143.15, 142.14, 139.67, 139.63, 131.64 - 131.17 (m), 130.13 - 129.47 (m), 128.81, 112.90-112.44 (m), 112.33 - 111.71 (m), 72.14, 50.71, 35.31 - 35.31 (m), 30.04, 28.55, 25.28. MS (ESI, positive): m/z calcd. for [C₂₅H₂₂F₄N₅OS]⁺:516.15, found: 516.15 [M]⁺.

### 3-((5-(4-((2,6-difluorophenyl)diazenyl)-3,5-difluorophenyl)pentyl)oxy)-4-(1-methyl-1,2,5,6-tetrahydropyridin-3-yl)-1,2,5-thiadiazole (Example 34)

According to general method L, 3-(4-((5-(4-((2,6-difluorophenyl)diazenyl)-3,5-difluorophenyl)pentyl)oxy)-1,2,5-thiadiazol-3-yl)-1-methylpyridin-1-ium iodide (160 mg, 0.25 mmol, 1.0 equiv.) was reduced with NaBH₄ (28 mg, 0.75 mmol, 3.0 equiv.) using methanol (1 mL) as solvent and stirring the reaction at ambient temperature for one overnight. After the first overnight, a second batch of NaBH₄ (28 mg, 0.75 mmol, 3.0 equiv.) had to be added to drive the reaction to completion. Purification by silica column chromatography (dichloromethane/ methanol 20:1) and subsequent reverse phase (C18) flash column chromatography (Water/MeOH) yielded 3-((5-(4-((2,6-difluorophenyl)diazenyl)-3,5-difluorophenyl)pentyl)oxy)-4-(1-methyl-1,2,5,6-tetrahydropyridin-3-yl)-1,2,5-thiadiazole **(34;** 55 mg, 0.107 mmol, 43%) as a red oil. ¹H NMR (400 MHz, Chloroform-d) δ 7.38 - 7.29 (m, 1H), 7.07 - 6.99 (m, 3H), 6.90 - 6.85 (m, 2H), 4.45 (t, *J* = 6.5 Hz, 2H), 3.46 - 3.43 (m, 2H), 2.68 (t, *J* = 7.6 Hz, 2H), 2.56 (t, *J* = 5.7 Hz, 2H), 2.47 - 2.39 (m, 5H), 1.88 (p, *J* = 6.7 Hz, 2H), 1.73 (p, *J* = 7.7 Hz, 2H), 1.57 - 1.47 (m, 2H),. ¹³C NMR (101 MHz, Chloroform-d) δ 162.52, 155.79 (dd, *J* = 261.6, 4.8 Hz), 155.64 (dd, *J* = 260.5, 4.1 Hz), 148.19 (t, *J =* 9.5 Hz), 146.91, 132.06 (t, *J* = 10.1 Hz), 131.16 (t, *J* = 10.3 Hz), 129.80 - 129.69 (m), 129.41, 128.44, 112.80, 112.78 - 112.32 (m), 70.71, 55.10, 51.31, 45.97, 35.85, 30.26, 28.79, 26.65, 25.66. MS (ESI, positive): m/z calcd. for [C₂₅H₂₆F₄N₅OS]⁺: 520.18, found: 520.15 [M+H]⁺. Purity (HPLC254nm) = 99%.

### 1.3. Preparation of tetra-ortho-chloro-substituted compound of formula (I), wherein R¹_R⁴ are Cl, R⁵ is O and n is 5.

### 3-(benzyloxy)-4-(pyridin-3-yl)-1,2,5-thiadiazole

According to general procedure I, 3-chloro-4-(pyridin-3-yl)-1,2,5-thiadiazole (309 mg, 1.73 mmol, 1.0 equiv.) was reacted with phenylmethanol (202 mg, 1.87 mmol, 1.1 equiv.) using NaH (207 mg, 5.18 mmol, 3.0 equiv.) in dry THF (7 mL) stirring the reaction for 16 h at 60°C. Purification by silica column chromatography (petroleum ether/ethyl acetate 10:1) yielded 3-(benzyloxy)-4-(pyridin-3-yl)-1,2,5-thiadiazole (378 mg, 1.26 mmol, 73%) as an off white solid. ¹H NMR (400 MHz, Chloroform-d) δ 9.44 - 9.38 (m, 1H), 8.65 - 8.62 (m, 1H), 8.44 - 8.40 (m, 1H), 7.54 - 7.46 (m, 2H), 7.46 - 7.33 (m, 4H), 5.57 (s, 2H). ¹³C NMR (101 MHz, Chloroform-d) δ 162.55, 150.24, 148.73, 145.17, 135.56, 134.95, 128.87, 128.77, 128.38, 127.72, 123.59, 73.05. MS (ESI, positive): m/z calcd. for [C₁₄H₁₂N₃OS]⁺: 270.07, found: 270.05 [M+H]⁺.

### 4-(pyridin-3-yl)-1,2,5-thiadiazol-3-ol

A solution of 3-(benzyloxy)-4-(pyridin-3-yl)-1,2,5-thiadiazole (316 mg, 1.17 mmol, 1.0 equiv.) in CH₂Cl₂ (6 mL) was mixed with HBr in AcOH (189 mg, 2.35 mmol, 2.0 equiv.) at ambient temperature and the reaction was stirred overnight at ambient temperature. Subsequently, the reaction mixture was quenched with sat. NaHCO₃ and extracted with CH₂Cl₂ at pH = 9 to remove the benzylic alcohol. After acidifying the mixture to pH = 5 the product could be extracted with EtOAc. The EtOAc was dried over Na₂SO₄ and concentrated under reduced pressure to yield the pure product as a white solid (193 mg, 1.08 mmol, 92%). ¹H NMR (400 MHz, DMSO-*d*₆) δ 13.38 (s, 1H), 9.31 - 9.22 (m, 1H), 8.71 - 8.60 (m, 1H), 8.50 - 8.40 (m, 1H), 7.59 - 7.50 (m, 1H). ¹³C NMR (101 MHz, DMSO-*d*₆) δ 162.56, 150.01, 147.82, 144.77, 134.30, 127.44, 123.78. MS (ESI, positive): m/z calcd. for [C₇H₆N₃OS]⁺: 180.02, found: 180.00 [M+H]⁺.

### 5-(4-nitrophenyl)pent-4-yn-1-ol

According to general method H, 1-bromo-4-nitrobenzene (2000 mg, 9.90 mmol, 1.0 equiv.) was coupled with pent-4-yn-1-ol (1.15 mL, 1041 mg, 12.40 mmol, 1.25 equiv.) using dry THF (40 mL) and stirring the reaction for 14 hours at 70°C. Purification by silica column chromatography (petroleum ether/ethyl acetate 7:2) afforded 5-(4-nitrophenyl)pent-4-yn-1-ol (1.98 g, 9.66 mmol, 98%) as an slightly orange solid. ¹H NMR (400 MHz, Chloroform-d) δ 8.22 - 8.01 (m, 2H), 7.58 - 7.41 (m, 2H), 3.79 (dd, *J* = 6.7, 5.6 Hz, 2H), 2.57 (t, *J* = 7.0 Hz, 2H), 1.92-1.80 (m, 3H). ¹³C NMR (101 MHz, Chloroform-d) δ 146.72, 132.34, 130.97, 123.56, 95.80, 79.71, 61.54, 31.20, 16.17.

### 5-(4-aminophenyl)pentan-1-ol

A solution of 5-(4-nitrophenyl)pent-4-yn-1-ol (1.90 g, 9.26 mmol, 1.0 equiv.) in methanol (30 mL) was flushed with argon and palladium on activated charcoal (150mg, 20 wt%) was added. The reaction was then stirred for four hours at room temperature under H₂ (10 atm.). Since the reaction was not complete, another 75 mg of palladium on activated charcoal was added and the reaction was stirred overnight at room temperature under 1 atm. of H₂. The reaction mixture was subsequently filtrated over a paper filter the filtrate was evaporated and subsequently filtered through a syringe filter yielding 5-(4-aminophenyl)pentan-1-ol (1.58 g, 8.80 mmol, 95%) as a brown solid. ¹H NMR (400 MHz, Chloroform-d) δ 7.02 - 6.92 (m, 2H), 6.67 - 6.59 (m, 2H), 3.63 (t, *J* = 6.6 Hz, 2H), 2.51 (t, *J* = 7.6 Hz, 2H), 1.69 - 1.48 (m, 6H). ¹³C NMR (101 MHz, Chloroform-d) δ 143.87, 133.44, 129.30, 115.46, 63.15, 35.15, 32.84, 31.68, 25.47. MS (ESI, positive): m/z calcd. for [C₁₁H₁₈NO]⁺: 180.14, found: 180.10 [M+H]⁺

### 5-(4-(phenyldiazenyl)phenyl)pentan-1-ol

According to general procedure J, nitrosobenzene (313 mg, 0.293 mmol 1.2 equiv.) and 5-(4-aminophenyl)pentan-1-ol (436 mg, 0.244 mmol, 1.0 equiv.) were reacted in acetic acid (2 mL). Purification by silica column chromatography (petroleum ether/ethyl acetate 5:2) yielded 5-(4-(phenyldiazenyl)phenyl)pentan-1-ol (550 mg, 2.05 mmol, 84%) as an orange solid. ¹H NMR (400 MHz, Chloroform-d) δ 7.96 - 7.82 (m, 4H), 7.55 - 7.43 (m, 3H), 7.35 - 7.29 (m, 2H), 3.64 (t, *J* = 6.6 Hz, 2H), 2.70 (t, *J* = 7.7 Hz, 2H), 2.47 (s, 1H), 1.75 - 1.56 (m, 4H), 1.48 - 1.38 (m, 2H). ¹³C NMR (101 MHz, Chloroform-d) δ 152.86, 151.11, 146.27, 130.83, 129.21, 129.16, 123.01, 122.83, 62.93, 35.93, 32.67, 31.16, 25.51. MS (ESI, positive): *m*/*z* calcd. for [C₁₇H₂₁N₂O]⁺: 269.16, found: 269.15 [M+H]⁺

### 5-(3,5-dichloro-4-((2,6-dichlorophenyl)diazenyl)phenyl)pentyl acetate

In a sealed reaction vessel, a solution of 5-(4-(phenyldiazenyl)phenyl)pentan-1-ol (250 mg, 0.93 mmol, 1.0 equiv) in degassed AcOH (9 mL) was mixed with Pd(OAc)₂ (21 mg, 93.2 µmol) and NCS (622 mg, 4.66 mmol, 5.0 equiv.). The reaction mixture was then heated to 140°C for 15 hours. After cooling down to ambient temperature, the reaction mixture was diluted with EtOAc, washed with sat. NaHCO₃, dried over sodium sulfate and concentrated under reduced pressure. Purification by silica column chromatography yielded 5-(3,5-dichloro-4-((2,6-dichlorophenyl)diazenyl)phenyl)pentyl acetate (163 mg, 0.364 mmol, 39%) as a red oil.¹H NMR (400 MHz, Chloroform-d) δ 7.43 - 7.02 (m, 5H), 4.05 (t, *J* = 6.6 Hz, 2H), 2.60 (t, *J* = 7.7 Hz, 2H), 2.03 (s, 3H), 1.71 - 1.59 (m, 4H), 1.44 - 1.34 (m, 2H). ¹³C NMR (101 MHz, Chloroform-d) δ 171.09, 147.88, 145.40, 129.40, 129.33, 127.53, 127.08, 64.23, 34.98, 30.35, 28.37, 25.43, 21.00. MS (ESI, positive): *m*/*z* calcd. for [C₁₉H₁₉Cl₄N₂O₂]⁺: 449.07, found: 449.00 [M+H]⁺

### 5-(3,5-dichloro-4-((2,6-dichlorophenyl)diazenyl)phenyl)pentan-1-ol

A solution of 5-(3,5-dichloro-4-((2,6-dichlorophenyl)diazenyl)phenyl)pentyl acetate (110 mg, 0.245 mmol) in THF (6 mL) was mixed with 6N HCl (1.5 mL) at ambient temperature. The mixture was heated to 70°C for 3 hours and the reaction was afterwards quenched with NaHCO₃ and extracted with EtOAc until the aqueous layer was colorless. The combined organic layers were dried over NaHCO₃ and concentrated. Purification by silica column chromatography (petroleum ether/ethyl acetate 5:1) yielded 5-(3,5-dichloro-4-((2,6-dichlorophenyl)diazenyl)phenyl)pentan-1-ol (100 mg, 0.245 mmol, quant.) as a dark red oil. ¹H NMR (400 MHz, Chloroform-d) δ 7.48 - 7.39 (m, 2H), 7.30 - 7.27 (m, 2H), 7.26 - 7.22 (m, 1H), 3.65 (t, J = 6.5 Hz, 2H), 2.64 (t, J = 7.7 Hz, 2H), 1.70 -1.58 (m, 4H), 1.46 - 1.41 (m, 2H). ¹³C NMR (101 MHz, Chloroform-d) δ 147.93, 145.64, 145.16, 129.45, 129.43, 129.36, 127.54, 127.10, 62.65, 35.18, 32.47, 30.64, 25.35·MS (ESI, positive): *m*/*z* calcd. for [C₁₇H₁₇Cl₄N₂O]⁺: 407.01, found: 406.95 [M+H]⁺

### 3-((5-(3,5-dichloro-4-((2,6-dichlorophenyl)diazenyl)phenyl)pentyl)oxy)-4-(pyridin-3-yl)-1,2,5-thiadiazole

A solution of PPh₃ (152 mg,0.579 mmol, 1.25 equiv.) was cooled down to 0°C before a solution of DEAD (91 µL, 0.579 mmol, 1.25 equiv.) in dry THF (1 mL) was added slowly. The mixture was stirred for 30 min before 4-(pyridin-3-yl)-1,2,5-thiadiazol-3-ol (104 mg, 0.579 mmol, 1.25 equiv.) and 5-(3,5-dichloro-4-((2,6-dichlorophenyl)diazenyl)phenyl)pentan-1-ol (188 mg, 0.463 mmol, 1.0 equiv.) dissolved in THF (1 mL) were added. The reaction was stirred overnight at room temperature. Afterwards the reaction was diluted with EtOAc and washed three times with sat. NaHCO₃ and brine. The organic layer was dried over Na₂SO₄ and concentrated under reduced pressure. Purification by silica column chromatography (petroleum ether/ethyl acetate 5:2) yielded 3-((5-(3,5-dichloro-4-((2,6-dichlorophenyl)diazenyl)phenyl)pentyl)oxy)-4-(pyridin-3-yl)-1,2,5-thiadiazole (262 mg, 0.462 mmol, 99%) as an dark red oil. ¹H NMR (400 MHz, Chloroform-d) δ 9.45 - 9.32 (m, 1H), 8.71 - 8.60 (m, 1H), 8.48 - 8.30 (m, 1H), 7.48 - 7.42 (m, 2H), 7.41 - 7.36 (m, 1H), 7.29 - 7.20 (m, 4H), 4.63 - 4.46 (m, 2H), 2.67 (t, J = 7.6 Hz, 2H), 2.03 - 1.87 (m, 2H), 1.85 - 1.66 (m, 3H), 1.64 - 1.50 (m, 2H). ¹³C NMR (101 MHz, Chloroform-d) δ 162.84, 150.35, 148.74, 148.05, 145.40, 145.30, 145.12, 134.81, 129.52, 129.50, 129.46, 127.74, 127.71, 127.26, 123.57, 71.21, 35.17, 30.60, 28.86, 25.74.MS (ESI, positive): *m*/*z* calcd. for [C₂₄H₂₀Cl₄N₅OS]⁺: 568.01, found: 567.95 [M+H]⁺

### 3-(4-((5-(3,5-dichloro-4-((2,6-dichlorophenyl)diazenyl)phenyl)pentyl)oxy)-1,2,5-thiadiazol-3-yl)-1-methylpyridin-1-ium iodide

A solution of 3-((5-(3,5-dichloro-4-((2,6-dichlorophenyl)diazenyl)phenyl)pentyl)oxy)-4-(pyridin-3-yl)-1,2,5-thiadiazole (263 mg, 0.463 mmol, 1.0 equiv.) in acetone was mixed with lodomethane (289 µL, 4.64 mmol, 10 equiv.) and the reaction was stirred for two overnights at room temperature. Subsequently the solvent was removed and the crude product was purified by column chromatography (CH₂Cl₂/MeOH 20:1) yielding the pyridinium salt as an red oil (184 mg, 0.270 mmol, 56%). ¹H NMR (400 MHz, Chloroform-d) δ 9.54 - 9.49 (m, 1H), 9.48 - 9.44 (m, 1H), 9.11 - 9.03 (m, 1H), 8.29 - 8.22 (m, 1H), 7.46 - 7.39 (m, 2H), 7.29 - 7.19 (m, 3H), 4.77 (s, 3H), 4.59 (t, J = 6.7 Hz, 2H), 2.67 (t, J = 7.6 Hz, 2H), 2.04 - 1.90 (m, 2H), 1.79 - 1.65 (m, 2H), 1.57 - 1.44 (m, 2H). ¹³C NMR (101 MHz, Chloroform-d) δ 163.12, 147.88, 145.61, 145.40, 145.26, 143.09, 142.09, 139.62, 131.51, 129.60, 129.53, 129.47, 128.76, 127.49, 127.14, 72.20, 50.75, 34.97, 30.38, 28.64, 25.34. MS (ESI, positive): *m*/*z* calcd. for [C₂₅H₂₂Cl₄N₅OS]⁺: 582.03, found: 582.00 [M+H]⁺

### 3-((5-(3,5-dichloro-4-((2,6-dichlorophenyl)diazenyl)phenyl)pentyl)oxy)-4-(1-methyl-1,2,5,6-tetrahydropyridin-3-yl)-1,2,5-thiadiazole (Example 36)

According to general methodL, 3-(4-((5-(3,5-dichloro-4-((2,6-dichlorophenyl)diazenyl)phenyl)pentyl)oxy)-1,2,5-thiadiazol-3-yl)-1-methylpyridin-1-ium iodide **(25;** 59 mg, 0.083 mmol, 1.0 equiv.) was reduced with NaBH₄ (3 mg, 0.25 mmol, 3.0 equiv.) in a mixture of dichloromethane/methanol (1:1) (~2 mL). Purification by silica column chromatography (dichloromethane/methanol 20:1) and subsequent purification by reverse phase (C18) flash chromatography (H₂O/MeOH) yielded 3-((5-(3,5-dichloro-4-((2,6-dichlorophenyl)diazenyl)phenyl)pentyl)oxy)-4-(1-methyl-1,2,5,6-tetrahydropyridin-3-yl)-1,2,5-thiadiazole (36; 21 mg, 0.04 mmol, 42%) as a red oil. ¹H NMR (400 MHz, Chloroform-*d*) ¹H NMR (400 MHz, Chloroform-d) δ 7.69-7.60 (m, 1H), 7.50 - 7.40 (m, 4H), 7.25 - 7.20 (m, 1H), 4.71 - 4.60 (m, 2H), 3.70 - 3.60 (m, 2H), 2.86 (t, *J =* 7.6 Hz, 1H), 2.80 - 2.74 (m, 2H), 2.69 - 2.60 (m, 5H), 2.14 - 2.04 (m, 2H), 1.98 - 1.84 (m, 2H), 1.79 - 1.64 (m, 2H). ¹³C NMR (101 MHz, Chloroform-d) ¹³C NMR (101 MHz, Chloroform-d) δ 162.56, 148.09, 146.97, 145.41, 145.37, 129.53, 129.48, 129.25, 129.10, 128.50, 127.74, 127.28, 70.75, 55.17, 51.38, 46.05, 35.21, 30.59, 28.84, 26.75, 25.75.MS (ESI, positive): *m*/*z* calcd. for [C₂₅H₂₂Cl₄N₅OS]⁺: 586.06 found: 586.05 [M+H]⁺. Purity (HPLC254nm) = 99%.

### 1.5. Preparation of a compound of formula (I) not encompassed by the invention, wherein R¹-R⁴ are F, R⁵ is O and n is 4 (Comparative example 33).

### 4-(4-amino-3,5-difluorophenyl)but-3-yn-1-ol

According to general method H, 4-bromo-2,6-difluoroaniline (1000 mg, 4.81 mmol, 1.0 equiv.) was coupled with but-3-yn-1-ol (1.09 mL, 1010 mg, 14.4 mmol, 3.0 equiv.) using triphenylphosphine, copper(I) iodide and bis(triphenylphosphine)-palladium(II) chloride, stirring the mixture in a solution of NEt₃/THF (20 mL) at 70°C for 16 hours. Purification by silica column chromatography (petroleum ether/ethyl acetate 5:2) afforded 4-(4-amino-3,5-difluorophenyl)but-3-yn-1-ol (806 mg, 4.09 mmol, 85%) as a brown solid. ¹H NMR (400 MHz, Chloroform-d) δ 6.97 - 6.81 (m, 2H), 3.78 (t, *J* = 6.3 Hz, 2H), 3.12 (s, 2H), 2.64 (t, *J* = 6.3 Hz, 2H). ¹³C NMR (101 MHz, Chloroform-d) δ 151.29 (dd, *J* = 240.5, 8.9 Hz), 124.79 (t, *J* = 16.4 Hz), 115.03 - 114.41 (m), 111.81 - 111.30 (m), 85.61, 81.10 (t, *J* = 3.5 Hz), 61.25, 23.87. MS (ESI, positive): m/z calcd. for [C₁₀H₁₀F₂NO]⁺: 198.07, found: 198.10 [M+H]⁺.

### 4-(4-amino-3,5-difluorophenyl)butan-1-ol

To a solution of 4-(4-amino-3,5-difluorophenyl)but-3-yn-1-ol (200 mg, 1.01 mmol) in MeOH (4 mL) was given palladium on activated charcoal (20 mg, 10 wt%). The reaction was flushed with H₂ and stirred under an atmosphere of H₂ at ambient temperature overnight. Afterwards, the mixture was ordinarily filtrated over a paper filter, concentrated and purified by silica column chromatography (petroleum ether/ ethyl acetate 5:2) to give 4-(4-amino-3,5-difluorophenyl)butan-1-ol (180 mg, 0.89 mmol, 89%) as a white solid. ¹H NMR (400 MHz, Chloroform-d) δ 6.74 - 6.56 (m, 2H), 3.70 - 3.59 (m, 2H), 2.80 (s, 0H), 2.52 (t, *J* = 7.2 Hz, 2H), 1.72 - 1.50 (m, 4H). ¹³C NMR (101 MHz, Chloroform-d) δ 152.17 (dd, *J* = 240.1, 8.3 Hz), 131.99 (t, *J=* 7.8 Hz), 121.75-121.42 (m), 111.03-110.59 (m), 62.84, 34.79 (t, *J* = 1.6 Hz), 32.20, 27.47. MS (ESI, positive): m/z calcd. for [C₁₁H₁₄F₂NO]⁺: 202.10, found: 202.05 [M+H]⁺.

### 2,6-difluoro-4-(4-((4-(pyridin-3-yl)-1,2,5-thiadiazol-3-yl)oxy)butyl)aniline

According to general procedure I, 3-chloro-4-(pyridin-3-yl)-1,2,5-thiadiazole (470 mg, 2.38 mmol, 1.5 equiv.) was reacted with 4-(4-amino-3,5-difluorophenyl)butan-1-ol (320 mg, 1.59 mmol, 1.0 equiv.) using NaH (253 mg, 6.33 mmol, 4.0 equiv.) in dry THF (10 mL) stirring the reaction for 13 h at 70°C. Purification by column chromatography (petroleum ether/ethyl acetate 5:1 to 4:1) yielded 2,6-difluoro-4-(4-((4-(pyridin-3-yl)-1,2,5-thiadiazol-3-yl)oxy)butyl)aniline (264 mg, 0.73 mmol, 46%) as a beige solid. ¹H NMR (400 MHz, Chloroform-*d*) δ 9.47 - 9.27 (m, 1H), 8.71 - 8.55 (m, 1H), 8.46 - 8.27 (m, 1H), 7.46 - 7.31 (m, 1H), 6.72 - 6.56 (m, 2H), 4.66 - 4.42 (m, 2H), 3.89 - 3.40 (m, 2H), 2.67 - 2.43 (m, 2H), 1.98 - 1.66 (m, 4H). ¹³C NMR (101 MHz, Chloroform-d) δ 162.75, 152.07 (dd, *J* = 240.2, 8.3 Hz), 150.20, 148.65, 145.00, 134.71, 131.20 (t, *J =* 7.8 Hz), 127.66, 123.47, 121.66 (t, *J =* 16.5 Hz), 111.11 - 110.52 (m), 71.07, 34.44, 28.25, 27.50. MS (ESI, positive): m/z calcd. for [C₁₇H₁₇F₂N₄OS]⁺: 363.11, found: 363.10 [M+H]⁺.

### 3-(4-(4-((2,6-difluorophenyl)diazenyl)-3,5-difluorophenyl)butoxy)-4-(pyridin-3-yl)-1,2,5-thiadiazole

A solution of 1,3-difluoro-2-nitrosobenzene (180 mg, 1.26 mmol, 2.0 equiv.) and 2,6-difluoro-4-(4-((4-(pyridin-3-yl)-1,2,5-thiadiazol-3-yl)oxy)butyl)aniline (230 mg, 0.63 mmol, 1 equiv.) in acetic acid/toluene/TFA (6:6:1; 2.5 mL) was stirred overnight at ambient temperature. The reaction mixture was concentrated under reduced pressure. The residue was dissolved in EtOAc and washed with sat. aq. NaHCO₃ and brine. After drying the organic layer over Na₂SO₄, it was concentrated and purified by silica column chromatography (petroleum ether/ethyl acetate 5:3) yielding 3-(4-(4-((2,6-difluorophenyl)diazenyl)-3,5-difluorophenyl)butoxy)-4-(pyridin-3-yl)-1,2,5-thiadiazole (255 mg, 0.52 mmol, 83%) as a red oil. ¹H NMR (400 MHz, Chloroform-d) δ 9.40 - 9.37 (m, 1H), 8.68 - 8.65 (m, 1H), 8.43 - 8.37 (m, 1H), 7.44 - 7.39 (m, 1H), 7.22 - 7.13 (m, 1H), 6.88 - 6.80 (m, 2H), 6.71 - 6.65 (m, 2H), 4.53 (t, *J* = 6.3 Hz, 2H), 2.65 (t, *J* = 7.6 Hz, 2H), 1.94 - 1.75 (m, 4H). ¹³C NMR (101 MHz, Chloroform-d) δ 162.74, 151.96 (dd, *J =* 253.7, 5.5 Hz), 151.84 (dd), 150.14, 148.42, 145.41, 145.00, 135.12, 129.80 (t, *J* = 9.2 Hz), 123.88 - 123.55 (m), 112.34 - 111.63 (m), 70.93, 35.30 - 34.89 (m), 28.43, 26.98. MS (ESI, positive): m/z calcd. for [C₂₃H₁₈F₄N₅OS]⁺:488.12, found: 488.15 [M+H]⁺.

### 3-(4-(4-(4-((2,6-difluorophenyl)diazenyl)-3,5-difluorophenyl)butoxy)-1,2,5-thiadiazol-3-yl)-1-methylpyridin-1-ium iodide

According to general method K, 3-(4-(4-((2,6-difluorophenyl)diazenyl)-3,5-difluorophenyl)butoxy)-4-(pyridin-3-yl)-1,2,5-thiadiazole (250 mg, 0.513 mmol, 1.0 equiv.) was methylated using iodomethane (319 µL, 5.13 mmol, 10 equiv.) in acetone, stirring the reaction for two overnights at ambient temperature. Purification by silica column chromatography (dichloromethane/methanol 20:1) yielded 3-(4-(4-(4-((2,6-difluorophenyl)diazenyl)-3,5-difluorophenyl)butoxy)-1,2,5-thiadiazol-3-yl)-1-methylpyridin-1-ium iodide (323 mg, 0.513 mmol, quant.) as a red solid. ¹H NMR (400 MHz, DMSO-*d*₆) δ 9.51 - 9.43 (m, 1H), 9.10 - 9.01 (m, 2H), 8.30 - 8.20 (m, 1H), 7.65 - 7.55 (m, 1H), 7.47 - 7.37 (m, 1H), 7.37 - 7.30 (m, 1H), 7.30 - 7.23 (m, 1H), 7.20 - 7.13 (m, 1H), 7.12 - 7.06 (m, 1H), 4.59 - 4.50 (m, 2H), 4.47 - 4.42 (m, 3H), 2.83 - 2.61 (m, 2H), 1.96 - 1.65 (m, 4H). ¹³C NMR (101 MHz, DMSO-*d*₆) δ 162.82, 155.96, 154.25, 152.74 (dd, *J* = 134.5, 4.5 Hz), 152.67 (dd), 149.77 - 149.52 (m), 147.32 (t, *J* = 9.0 Hz), 145.82 - 145.47 (m), 144.30 - 143.98 (m), 142.14 - 142.01 (m), 141.26 -141.20 (m), 131.48 (t, *J* = 10.1 Hz), 130.43-130.30 (m), 128.26 - 128.04 (m), 113.53 - 112.24 (m), 71.50, 48.71, 34.17, 27.68, 26.46. MS (ESI, positive): m/z calcd. for [C₂₄H₂₀F₄N₅OS]⁺:502.13, found: 502.10 [M]⁺.

### 3-(4-(4-((2,6-difluorophenyl)diazenyl)-3,5-difluorophenyl)butoxy)-4-(1-methyl-1,2,5,6-tetrahydropyridin-3-yl)-1,2,5-thiadiazole (Comparative Example 33)

According to general method L, 3-(4-(4-(4-((2,6-difluorophenyl)diazenyl)-3,5-difluorophenyl)butoxy)-1,2,5-thiadiazol-3-yl)-1-methylpyridin-1-ium iodide (254 mg, 0.40 mmol, 1.0 equiv.) was reduced with NaBH₄ (42.4 mg, 1.12 mmol, 2.8 equiv.) using methanol/dichloromethane (1:3; 2 mL) as solvent and stirring the reaction at ambient temperature for one overnight. Purification by silica column chromatography (dichloromethane/ methanol 20:1) and subsequent reverse phase (C18) flash column chromatography (water/MeOH) yielded 3-(4-(4-((2,6-difluorophenyl)diazenyl)-3,5-difluorophenyl)butoxy)-4-(1-methyl-1,2,5,6-tetrahydropyridin-3-yl)-1,2,5-thiadiazole (33; 43 mg, 85 µmol, 21%) as a red oil. ¹H NMR (400 MHz, Chloroform-d) δ 7.13 - 7.01 (m, 1H), 6.96 - 6.80 (m, 2H), 6.72 - 6.62 (m, 2H), 4.59 - 4.40 (m, 1H), 3.78 (s, 2H), 2.95 (s, 2H), 2.83 - 2.54 (m, 2H), 1.94 - 1.69 (m, 7H), 7.41 - 7.14 (m, 4H). ¹³C NMR (101 MHz, Chloroform-*d*) δ 162.39, 155.77 (dd, J = 261.7, 4.8 Hz), 155.60 (dd, J = 260.5, 4.2 Hz), 147.72 (t, J = 9.6 Hz), 146.84, 131.98 (t, J = 10.0 Hz), 131.20 (t, J = 10.4 Hz), 130.01 - 129.62 (m), 129.38, 128.43, 112.77, 112.85 - 112.27 (m), 70.34, 55.05, 51.26, 45.92, 35.36, 28.27, 27.01, 26.59.MS (ESI, positive): m/z calcd. for [C₂₄H₂₄F₄N₅OS]⁺: 506.16, found: 506.20 [M+H]⁺. Purity (HPLC₂₅₄ₙₘ) = 97%.

### 2. Results

### 2.1. Photophysical characterization and Gag - Phospholipase C Interaction Assay

The photophysical properties of the compounds was determined using UV/Vis spectroscopy according to the procedures described above. For Examples and Comparative Examples 27b-g, 28a, 28b, 29a and 29b, the most enriching wavelengths were conserved at 455 and 400 nm for maximal trans-enrichment and 365 nm for maximal cis-enrichment. Also, no decomposition upon irradiation and the typical thermal stabilities were observed. Difluorination, however, resulted in lower cis-enrichment upon irradiation with 365nm (PSS₃₆₅ₙₘ: *cis*/*trans =* 70:30). The tested compounds showed no photobleaching and the thermal stability of the cis-isomer was not altered. Comparative example 33 and Example 34 could be operated with blue (450 nm, cis→trans) and green light (530 nm, trans→cis), whereas tetra-ortho-chlorinated compound of Example 36 could be switched with blue (450 nm, cis→trans) and orange/red light (590 nm, trans→cis). Thermostability and photobleaching behaviour were in accordance with the prototypical photoswitchable moieties of each species, allowing for clean, reversible switching, highly enriched photostationary states and long thermal relaxation half-lives.

Subsequently, compounds were tested at their maximally *cis*/*trans*-enriched PSSs for their ability to light-dependently activate the human muscarinic receptor 1 (hM1R) *in vitro.* A split luciferase complementation assay (SLCA) which detects interactions between Gαq and its effector phospholipase C-β3 (PLC-β3) as a quantifiable parameter of receptor activation was employed, as previously described. The wavelength emitted by the modified luciferase from the click-beetle *Pyrophorus plagiophthalamus* (λmax = 613nm) shows no interference with the compounds photoisomerization. The results are shown in Figure 1. All compounds were shown to be "cis-on", that means, more active in their thermodynamically less stable *cis*-configuration. In photopharmacology this is a highly desired feature for the majority of applications. This way, the photoswitch can on its own only turn itself inactive instead of spontaneously forming the bioactive isomer by thermal relaxation in the dark. The compounds were highly potent agonists comparable in potency. However, while C2 (Comparative Example 27b) and C3 (Comparative Example 27c) linker lengths resulted in active molecules with an up to two-digit nanomolar potency these compounds do not show a strong potency- or efficacy-shift upon irradiation. Useful functional photoswitching is only observed employing linker lengths of C4 (Example 27d) and C5 (Example 27e), both result in an approximately 3 fold increase of potency upon irradiation with UV light (see Figure 1). Additionally, compound (Example 27e) shows a 25% reduced maximal efficacy in its *trans-*configuration. Further elongation of linker lengths leads to reduced overall maximal efficacy and a decrease in functional photoswitching (Example 27f and Comparative Example 27g: Eₘₐₓ = 80%; EC_{50*trans*}/EC_{50*cis*} ≈ 2). Biological evaluation of Comparative Example 33 showed complete elimination of any functional photoswitching. Cis and *trans* enriched states, even though being almost quantitatively switched, showed identical activity in the assay. Some functional photoswitching could be recovered by elongation of the linker to five methylene units (Example 34), which was also observed for the tetra-ortho-chlorinated compound (Example 36).

A summary of the thermal relaxation, *cis*/*trans* ratios and biological effects of the relevant photostationary states is gathered in the table below.

| Compound | *Thermal Relaxation* | *cis*/*trans* ratio | EC₅₀ [nM] | %Emax |
|---|---|---|---|---|
| Comparative Example 27b | 3.5% | PSS₃₆₅: 90/10 | 121 | 95.4 ± 1.1 |
| | | PSS₄₅₅: 20/80 | 143 | 88.2 ± 1.5 |
| | | | | |
| Comparative Example 27c | 3.5% | PSS₃₆₅: 70/30 | 60.3 | 115 ± 1.4 |
| | | PSS₄₅₅: 20/80 | 104 | 114 ± 1.4 |
| | | | | |
| Example 27d | 3.1% | PSS₃₆₅: 93/7 | 44.0 | 108 ± 1.2 |
| | | PSS₄₅₅: 20/80 | 130 | 96.0 ± 1.5 |
| | | | | |
| Example 27e | 5.6% | PSS₃₆₅: 92/8 | 63.6 | 94.3 ± 0.8 |
| | | PSS₄₅₅: 20/80 | 198 | 69.2 ± 1.1 |
| | | | | |
| Example 27f | 5.6% | PSS₃₆₅: 92/8 | 180 | 79.0 ± 0.6 |
| | | PSS₄₅₅: 20/80 | 371 | 64.7 ± 0.6 |
| | | | | |
| Comparative Example 27g | 5.1% | PSS₃₆₅: 85/15 | 194 | 79.2 ± 1.8 |
| | | PSS₄₅₅: 14/86 | 395 | 84.2 ± 4.4 |
| | | | | |
| Example 28a | 3.7% | PSS₃₆₅: 86/14 | 41.9 | 105 ± 1.0 |
| | | PSS₄₅₅: 19/81 | 127 | 97.4 ± 1.6 |
| | | | | |
| Comparative Example 28b | 3.4% | PSS₃₆₅: 73/27 | 116 | 99.4 ± 0.9 |
| | | PSS₄₅₅: 20/80 | 107 | 93.4 ± 0.7 |
| | | | | |
| Example 29a | 4.2% | PSS₃₆₅: 77/23 | 49.6 | 97.4 ± 0.2 |
| | | PSS₄₅₅: 18/82 | 164 | 84.5 ± 0.9 |
| | | | | |
| Example 29b | 5.6% | PSS₃₆₅: 70/30 | 78.7 | 106 ± 0.7 |
| | | PSS₄₅₅: 18/82 | 208 | 99.9 ± 0.8 |
| | | | | |
| Comparative Example 33 | 1.7% (14 h) | PSS₅₂₈: 91/9 | 186 | 99.4 ± 1.2 |
| | | PSS₄₀₅: 11/89 | 163 | 96.4 ± 1.5 |
| | | | | |
| Example 34 | 1.8% (16 h) | PSS₅₂₈: 90/10 | 93.3 | 98.6 ± 0.7 |
| | | PSS₄₀₅: 12/88 | 139 | 90.0 ± 0.5 |
| | | | | |
| Example 36 | 1.3 % (18 h) | PSS₅₉₀: 89/11 | 199 | 77.8 ± 1.7 |
| | | PSS₄₀₅: 10/90 | 205 | 62.0 ± 1.3 |

### 2.2. Fluorescence Imaging of Intracellular Ca²⁺

The photopharmacological properties of compound from Example 27e and Examples 29a,b were tested for their ability to induce cytosolic calcium oscillations *in vitro* under 1PE and 2PE conditions. Genetically encoded calcium indicator GCaMP6s (ex. 488 nm, em. 510nm) was used for fluorescence imaging (fluorescence responses were quantified by peak amplitude of acetylcholine (ACh)).

Calcium imaging was performed before and after 1PE of the compounds with UV light of 365 nm (Figure 2 A-F), which showed that the cytosolic calcium oscillations were induced by the cis-configuration of the compounds but not by the *trans*-configuration. Neither the application of the compounds in *trans*-enriched state (dark-adapted state) nor 1PE with 455 nm elicited fluorescence responses. Moreover, the photoresponses could be dynamically photomodulated. Converting the compounds to the trans-configuration live on the cells, using 455 nm for Example 27e and Example 29a and 400 nm for Example 29b (Figure 2 A-C), resulted in significantly reduced responses which could be reverted by irradiation with 365 nm light. Quantification by peak amplitude and analysis showed a complete on/off switching at several concentrations (Figure 2 D-F) and fluorescence photoresponses were only observed in cells expressing M₁ receptors.

Manipulation of M₁R activity using 2PE was achieved with an inverted laser-scanning microscope equipped with a pulsed NIR laser (Figure 2 G-L) and the described calcium imaging assay. The utilized wavelengths for 2PE experiments correspond to twice the wavelength used for 1PE. Dark adapted compounds from Examples 29a,b caused no fluorescence signal, but irradiation with 730 nm caused M₁R activation, indicating that significant *trans* to *cis* isomerization had occurred. Interestingly, the observed photoresponses could be completely inhibited under 2PE conditions at longer wavelengths, suggesting that *cis* to *trans* photoisomerization by 2PE is also viable. In particular, 910 nm, for Example 29a (Figure 2 G,H) and 800 nm, for Example 29b (Figure 2 J,K). Again, the trans-configurations did not produce calcium responses, and 2PE conditions alone did not produce any stimulation artifacts. Photoresponses required M₁R expression. These results are quantified in Figure 2 (I,L) and demonstrate that both compounds from Examples 29a and 29b can isomerize in both ways using 2PE, with Example 29a showing an improved bidirectional switching.

Compounds from Examples 34 and 36 were also tested for their ability to induce cytosolic calcium oscillations *in vitro* under 1PE conditions. The above described calcium imaging assay using HEK cells co-expressing human M₁R and the calcium indicator R-GECOL (ex. 562nm, em. 600nm) was employed. Here, carbachol (CCh) was used as a control (Figure 3). When the compound from Example 34 or 36 was applied to the cells in their dark adapted trans-configuration, it resulted in no significant responses.

A robust increase of intracellular calcium was observed under illumination with 530 nm (Figure 3 A,B) and 590 nm (Figure 3 D,E), which induced the *trans*-to-*cis* isomerization of Example 34 and 36, respectively. Calcium responses were abolished by illumination with 400 nm for Example 34 (Figure 3 A,B) and 455 nm for Example 36 (Figure 3 D,E), which photoisomerize back to their *trans*-enriched states. To further validate the cis-on activity of Example 36, we applied the 590 nm pre-illuminated cis-Example 36. This resulted in a more evident effect. The quantification of the peak amplitude confirmed that *trans*-Example 34 (Figure 3 C) and *trans*-Example 36 (Figure 3 D) did not evoke any effect, while both cis-Example 34 (Figure 3 C) and cis-Example 36 (Figure 3 D) stimulated the release of intracellular calcium (similar or above CCh) at all the concentrations used (0.3, 1 and 10 µM). No responses were observed in control experiments with HEK cells not expressing human M₁R, thus confirming that the calcium oscillations were evoked by this receptor specifically. Altogether, the *in vitro* data demonstrated that both compounds can be dynamically photomodulated with respect to their agonistic behavior and both are more active in their cis-configuration.

### 2.3. Neuronal photomodulation in vitro and in vivo

Compound of Examples 29a and 29b were tested for their ability to reversibly photo-regulate the firing activity in cultured hippocampal neurons under 1PE (Figure 4 A-D). Procedures were performed in accordance with the European guidelines for animal care and use in research and were approved by the Animal Experimentation Ethics Committee at the University of Barcelona. The membrane-permeable calcium fluorescence indicator OGB-1-AM (excitation 494 nm, emission 523 nm) was used for fluorescence imaging. Application of the vehicle, trans-Example 29a or trans-Example 29b did not produce any change in calcium fluorescence responses, whereas enriching the *cis* configuration of both compounds, by illumination of the preparation with UV light, triggered neuronal responses (Figure 4A,C). Quantification of the results (Figure 4 B,D) showed an significant increase in neuronal firing frequency, induced by the *cis-*configurations of both compounds, compared to their *trans*-configuration. In particular, Example 29b yielded significantly higher firing frequency at 365 than 400 nm illumination. These results indicate that it is possible to photoactivate neurons using the compounds of the present invention.

Control of spontaneous calcium activity in mice cortex under 2PE using Example 29a was tested. OGB-1-AM was pressure-injected through a glass pipette into the cortex of anesthetized mice (Figure 4 E). Typically, 1 h after injection, all cells within a radius of 300 µm were labeled. Spontaneous calcium transients were monitored (ex. 800 nm, em. 510 nm) at 100 µm below the cortical surface for several minutes before and after 730 nm illumination (single plane scan), with or without the compound. Application of the trans-compound did not alter cortical activity, which remains alike the control (Figure 4F), whereas photoconversion to the cis-configuration increased its excitability (Figure 4 F). These results were quantified by means of the response amplitude and the area under the curve (AUC) of the response time course. Both parameters showed that Example 29a significantly alters firing activity upon photoactivation (Figure 4 G,H).

Subsequently, the potential of Example 29a to enable reversible 2P modulation of cortical activity *in vivo* was evaluated. After photoconversion of Example 29a to the pharmacologically active cis-isomer, 800 nm light was applied (twice the power density used for imaging) to switch it back to its trans-configuration (Figure 4 I). A decrease back to control level in firing activity was observed suggesting that Example 29a is effectively back isomerized by 2PE *in vivo.* The analysis of the response amplitude and AUC shows a significant reduction in cortical excitability upon deactivation of cis-Example 29a by 2PE at 800 nm (Figure 4 J,K).

### 2.4. Photomodulation of zebrafish motility

In an animal model (zebrafish larvae) for photopharmacological applications, the effect of Example 36 was evaluated. Example 36 suggested to have the biggest potential for biological applications [Matera, C. et al. Int.J. Mol. Sci. 2022, 23, 10114; Matera, C. et al. J. Am. Chem. Soc. 2018, 140, 15764-15773]. It is known that MRs play key roles in a myriad of central processes, such as locomotion. To achieve our purpose, we designed a behavioral assay to record and quantify the locomotor activity. The advantages of zebrafish larvae (Danio rerio) is their transparency facilitating the delivery of light, their small size and the permeability to small molecules.[Basnet, R. M. et al. Biomedicines 2019, 7, 23]. Moreover, the zebrafish brain presents a structural organization and a cellular morphology like higher vertebrates, such as humans [Basnet, R. M. et al. Biomedicines 2019, 7, 23]. Interestingly, the genome of zebrafish comprises orthologs of 71% of human genes, including all five the mAChRs. The experiments were performed on blinded zebrafish larvae. This to be certain that the effect on the motility was triggered by the compound rather than through natural photoresponsive behaviors. Zebrafish larval movements were tracked using a Zebrabox device for automated behavioral recordings. These animals at 7 days post-fertilization (7 dpf) were randomly divided into control (i.e., vehicle) and treatment groups (i.e., Example 36 added to water) with each fish placed in a distinct well of a 96-well plate. After the relaxation period of 20 min, the blinded animals were exposed to two consecutive cycles of 590 nm and 455 nm light. To determine whether the molecule affected the behaviors, the swimming distances were measured over time for the treated and control groups (Figure 5 A). Intriguingly, the animal under 455 nm light displayed high swimming activity, which was reduced under 590 nm light. Furthermore, no significant differences in locomotor activity were observed between the control groups (Figure 5 A). The quantification and statistical analysis of the total distances swum by the control and the treated group during the light cycles is displayed in Figure 5 B. The swimming activity of treatment group blind larvae significantly increase under 455 nm (*trans*-Example 36), whereas it is reduced under 590 nm light (*cis*-Example 36). The activation of the M1Rs by the cis-Example 36 could be responsible for the partial inhibition of the locomotory activity. However, the mechanism underlying the outcome of the response (i.e., locomotion) to a cholinergic input is overly complex. Several evidence suggest the existence of a complex interplay between the cholinergic and dopaminergic systems in the basal ganglia that is responsible for locomotion [Crans, R. A. J. et al. Front. Pharmacol. 2020, 11, 194]. In fact, different muscarinic receptor subtypes at interneurons and medium spiny neurons could be activated or suppressed, thereby resulting in the diversification of the locomotor activity [Gerber, D. J.et al. Proc. Natl. Acad. Sci. U.S.A. 2001, 98, 15312-15317; Crans, R. A. J. et al. Neural Regen. Res. 2021, 16, 1406-1408]. Overall, the *in vivo* studies in zebrafish larvae demonstrated that Example 36 allows reversible modulation of the locomotory activity *in vivo.* In addition, these animals were still alive about 15 h after the experiments, presenting a clear absence of acute toxicity.

### 2.5. Conclusion

The compounds of the invention are "cis-on" switches, which means that only by irradiation with light the active conformation can be obtained. This is preferable to *trans-*on switches as spontaneous activation by non-irradiation dependent processes like thermal relaxation is not possible. The compounds of the invention enable reversible manipulation of muscarinic receptors that is unprecedented with other compounds at 1PE and 2PE. Example 29a stands out by its potency, efficacy, drug-likeness, low activity in the dark, and most importantly by its ability to bidirectionally modulate cortical activity *in vitro* and *in vivo* using wavelengths bearing deep tissue penetration and multiphoton focusing. Moreover, compound 36 is distinguished by its full agonism, micromolar potency, and orange-red light activation *in vitro* and *in vivo.* Activation of endogenous M₁Rs in spatiotemporal patterns will allow dissecting the pathophysiological roles of muscarinic subtypes in cholinergic neurotransmission. In combination with suitable illumination devices, compound 36 gathers several favorable properties to support photopharmacological therapies for the PNS and CNS.

Based on the ability of other reported muscarinic photoswitchable ligands like PAI134 or BAI135 to enable reversible modulation of slow brain-waves *in vivo,* the compounds of the invention allow the reactivation of brain regions displaying reduced activity as a result of stroke [Cassidy, J. M. et al. Stroke 2020, 51, 1442-1450] or traumatic brain injury [Hogan, S. E. et al. J. Clin. Sleep Med. 2020, 16, 1445-1454; Kaada, B. R. et al. **1961,** 13, 785-789]; the regulation of brain activity associated with sleep disorders; [Kaltiainen, H. et al. Brain Topogr. 2018, 31, 1037-1046] the induction of faster recovery from anesthesia [Modarres, M. H. et al. Neurobiol. Sleep Circadian Rhythms 2017, 2, 59-70] or the awakening from coma state [Torao-Angosto, M. et al. Front. Syst. Neurosci. 2021, 15, DOI: 10.3389/fnsys.2021.609645] In comparison to PAI or BAI the compounds of the invention exhibits the clear advantage of being more "drug-like" than the former two due to their size and molecular charge.

## Claims

1. A compound of formula (I): 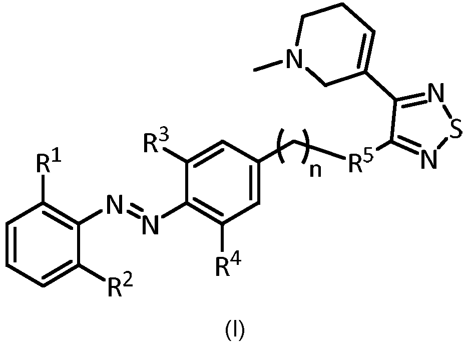 wherein
R¹, R², R³ and R⁴ are independently selected from the group consisting of H, halogen atom and C₁-C₃ alkoxy,
R⁵ is selected from O and S, and
n is an integer from 4 to 6,
with the proviso that when n is 4, R¹ and R² are not F simultaneously,
or a pharmaceutically acceptable salt or stereoisomer thereof.

2. Compound according to any one of the preceding claims, wherein R⁵ is O.

3. Compound according to claim 1 or 2, wherein R¹, R², R³ and R⁴ are independently selected from the group consisting of H, halogen atom and methoxy.

4. Compound according to any one of the preceding claims, wherein R¹, R², R³ and R⁴ are independently selected from the group consisting of H and halogen atom.

5. Compound according to any one of the preceding claims, wherein the halogen atom is independently selected from F and CI.

6. Compound according to any one of the preceding claims, wherein R¹, R², R³ and R⁴ are independently selected from the group consisting of H and F.

7. Compound according to any one of the preceding claims, wherein R³ and R⁴ are both H.

8. Compound according to any one of the preceding claims, wherein:
- R¹ and R² are both H,
- one of R¹ and R² is H and the other one is F, or
- R¹ and R² are both F.

9. Compound according to any one of claims 1 to 5, wherein R¹, R², R³ and R⁴ are halogen atom; preferably wherein R¹, R², R³ and R⁴ are simultaneously F or wherein R¹, R², R³ and R⁴ are simultaneously Cl.

10. Compound according to any one of the preceding claims, wherein n is 4 or 5.

11. Compound according to claim 1, which is selected from the group consisting of:
3-(1-methyl-1,2,5,6-tetrahydropyridindin-3-yl)-4-(4-(4-(phenyldiazenyl)phenyl)butoxy)-1,2,5-thiadiazole,
3-(1-methyl-1,2,5,6-tetrahydropyridin-3-yl)-4-((5-(4-(phenyldiazenyl)phenyl)-pentyl)oxy)-1,2,5-thiadiazole,
3-(1-methyl-1,2,5,6-tetrahydropyridin-3-yl)-4-((6-(4-(phenyldiazenyl)phenyl)hexyl)-oxy)-1,2,5-thiadiazole,
3-(4-(4-((2-fluorophenyl)diazenyl)phenyl)butoxy)-4-(1-methyl-1,2,5,6-tetrahydropyridin-3-yl)-1,2,5-thiadiazole,
3-((5-(4-((2-fluorophenyl)diazenyl)phenyl)pentyl)oxy)-4-(1-methyl-1,2,5,6-tetrahydropyridin- 3-yl)-1,2,5-thiadiazole,
3-((5-(4-((2,6-difluorophenyl)diazenyl)phenyl)pentyl)oxy)-4-(1-methyl-1,2,5,6-tetrahydropyridin-3-yl)-1,2,5-thiadiazole,
3-((5-(4-((2,6-difluorophenyl)diazenyl)-3,5-difluorophenyl)pentyl)oxy)-4-(1-methyl-1,2,5,6-tetrahydropyridin-3-yl)-1,2,5-thiadiazole, and
3-((5-(3,5-dichloro-4-((2,6-dichlorophenyl)diazenyl)phenyl)pentyl)oxy)-4-(1-methyl-1,2,5,6-tetrahydropyridin-3-yl)-1,2,5-thiadiazole,
or a pharmaceutically acceptable salt or stereoisomer thereof.

12. Pharmaceutical composition comprising a compound according to any one of claims 1 to 10 and a pharmaceutically acceptable excipient.

13. Compound according to any one of claims 1 to 11 or pharmaceutical composition according to claim 12, for use in medicine.

14. Compound according to any one of claims 1 to 11 or pharmaceutical composition according to claim 12, for use in the treatment and/or prevention of neurological disorders.

15. Compound or pharmaceutical composition for use according to claim 14, wherein the neurological disorder is selected from the group consisting of schizophrenia, Alzheimer's disease, Parkinson's disease, stroke, traumatic brain injury, sleep disorders, and coma state.
